# EUROPEAN PATENT APPLICATION

(11) **EP 4 782 547 A1**
(43) Date of publication of application: **29.07.2026**
(21) Application number: 25154567.9
(22) Date of filing: 28.01.2025
(51) Int. Cl.: C12P 21/00, C07B 59/00, C12N 5/00, C12P 7/6409, C12P 19/00, C12P 19/12

(54) **A METHOD OF PREPARATION OF 13C LABELED BIOMOLECULES**

(71) Applicant: Univerzita Pardubice, 532 10 Pardubice (CZ)
(72) Inventor: Holcapek, Michal, Stare Hradiste (CZ); Rousar, Tomas, Chocen (CZ); Bacova, Jana, Pardubice (CZ); Smid, Lenka, Pardubice (CZ); Jirasko, Robert, Pardubice (CZ); Peterka, Ondrej, Pteni (CZ); Lasko, Zuzana, Chocen (CZ)
(74) Representative: Harber IP s.r.o.

(57) **Abstract**

The present invention provides a method for preparation of fully ¹³C isotopically labeled biomolecules or biomolecules having a predetermined ¹³C isotopic labeling by biosynthesis in a mammalian cell line, which comprises the following steps:
a) providing a mammalian cell line capable of growing in serum-free media;
b) culturing the cell line obtained in step b) in a serum-free medium containing: fully ¹³C isotopically labeled amino acids or ¹³C isotopically labeled amino acids, fully ¹³C isotopically labeled glucose and/or fully ¹³C isotopically labeled galactose, or ¹³C isotopically labeled glucose and/or ¹³C isotopically labeled galactose, fully ¹³C isotopically labeled choline or ¹³C isotopically labeled choline, vitamins, distilled water, insulin, transferrin, sodium selenite, and inorganic salts wherein when the inorganic salt contains carbon, it is preferably fully ¹³C isotopically labeled;
c) obtaining ¹³C isotopically labeled biomolecules from the cells or medium after the cultivation of step b).

The resulting biomolecules and their mixtures are suitable for use as internal standards in metabolomics and fluxonics, as well as in biotechnological research as standards or markers.

## Description

### Field of Art

This invention describes a method for preparing ¹³C labeled biomolecules with high isotopic purity, unlocking novel applications in bioanalysis and biological research. These ¹³C fully or specifically labeled lipids, metabolites, or proteins can serve, for example, as internal standards for mass spectrometry-based quantitation and as tracers for investigating metabolic pathways in fluxomics and other biological experiments.

### Background Art

Lipidomic, metabolomic, and proteomic analyses, integral components of omics studies, rely on mass spectrometry (MS) coupled with separation techniques such as liquid chromatography (LC) or supercritical fluid chromatography (SFC). Accurate quantitation, essential for these analyses, requires the precise determination of molar concentrations. This process needs the use of exogenous internal standards (IS), which are introduced into biological samples prior to processing. Isotopically labeled IS, typically containing deuterium or ¹³C atoms, are considered the gold standard due to their structural similarity to analytes and absence from biological systems, thereby facilitating robust and accurate quantitation. However, several challenges persist.

Isotopically labeled IS, particularly for rare or complex biomolecules, are scarce and costly to produce. Deuterium-labeled IS often exhibit retention time shifts and isotopic migration during MS fragmentation, which compromises accuracy. Although ¹³C-labeled IS do not suffer from these issues, their high cost and limited supply remain significant barriers. The current standard practice in omics studies detecting hundreds or thousands of analytes involves the use of only one or a few IS per class of analytes, based on the assumption of similar ionization efficiencies and suppression effects. However, this approach has significant limitations, particularly in the analysis of isomeric forms or when measurements vary across different instruments or laboratories. To enable comprehensive multiomics quantitation, fully isotopically labeled IS (i.e., with ¹³C enrichment of more than 98% in ¹³C-labeled biomolecules) for all chromatographically resolved analytes are required. At present, however, no existing solution fulfills this requirement.

Various efforts have been undertaken to bridge this gap, with ¹³C-labeling strategies showing promise. The generation of fully ¹³C-labeled metabolites from yeast [WO 2018/007599] and bacteria [M.A. Jaber, B. de Falco, S. Abdelrazig, C.A. Ortori, D.A. Barrett, D.H. Kim. Anal. Methods 15 (2023) 2925] as internal standards for the comprehensive lipidomic and metabolomics analysis in human plasma has been explored. This approach is relatively simple, cost-effective, and produces high isotopic purity. However, the metabolite production capacity of yeast and bacteria does not align with human biological systems and shows insufficient coverage of the human lipidome for comprehensive lipidomic or metabolomic quantification.

In other studies, regarding metabolomic tracing, animal [P. Li, M. Lammerhofer. Anal. Chem. 94 (2022) 15332] or human [S.J. Yoon, J.A. Combs, A. Falzone, N. Prieto-Farigua, S. Caldwell, D.H. Ackerman, E.R. Flores, G.M. Denicola. Cancer Res. 83 (2023) 1426; L. Stuani, F. Riols, P. Millard, M. Sabatier, A. Batut, E. Saland, F. Viars, L. Tonini, S. Zaghdoudi, L.K. Linares. Int. J. Mol. Sci. 19 (2018) 3325] cell lines were used to generate a limited number of ¹³C-labeled metabolites by culturing in cell culture medium containing a limited amount of ¹³C-labeled amino acids or glucose. Thus, these studies did not produce sufficient number of ¹³C-labeled biomolecules for use as internal standards in omics experiments.

In a separate effort, culture of human cell lines in a medium containing fully ¹³C-labeled amino acids and glucose were used only in one study to produce ¹³C-labeled metabolites [N. Grankvist, J.D. Watrous, K.A. Lagerborg, Y. Lyutvinskiy, M. Jain, R. Nilsson. Cell Chem. Biol. 25 (2018) 1419]. However, generation of biomolecules with high isotopic purity was still not achieved, especially larger molecules contained a rather balanced mix of ¹²C and ¹³C. The authors state that about 70 % of obtained molecules contained some ¹³C, and only very small molecules (e.g. pyruvate) could be fully ¹³C-labeled. Addressing the challenges do develop the method to produce the spectrum of fully ¹³C-enriched internal standards for mass spectroscopy measurement requires the development of an economical, scalable, and reliable method for generating ¹³C-labeled compounds that properly reflect human biological profiles of biomolecules.

### Summary of the Invention

The present invention provides a method for preparation of fully ¹³C isotopically labeled biomolecules or biomolecules having a predetermined ¹³C isotopic labeling by biosynthesis in a mammalian cell line, which comprises the following steps:
a) providing a mammalian cell line capable of growing in serum-free media;
b) culturing the cell line obtained in step b) in a serum-free medium containing:
   - fully ¹³C isotopically labeled amino acids or ¹³C isotopically labeled amino acids,
   - fully ¹³C isotopically labeled glucose and/or fully ¹³C isotopically labeled galactose, or ¹³C isotopically labeled glucose and/or ¹³C isotopically labeled galactose,
   - fully ¹³C isotopically labeled choline or ¹³C isotopically labeled choline,
   - vitamins,
   - distilled water,
   - insulin,
   - transferrin,
   - sodium selenite, and
   - inorganic salts wherein when the inorganic salt contains carbon, it is preferably fully ¹³C isotopically labeled;
c) obtaining ¹³C isotopically labeled biomolecules from the cells or medium after the cultivation of step b).

The term "fully ¹³C isotopically labeled" molecule/compound refers to a molecule in which all carbons are ¹³C with isotopic purity of at least 97%, preferably at least 98%, and more preferably at least 99%.

The term "predetermined ¹³C isotopic labeling" refers to biomolecules containing ¹³C atoms at predetermined positions, with isotopic purity of at least 97%, preferably at least 98%, and more preferably at least 99%.

The high isotopic purity (i.e., isotopic purity of at least 97%, preferably at least 98%, and more preferably at least 99%) of ¹³C labeling of the obtained biomolecules is an important advantage of the method of the invention over the prior art.

In one embodiment, the present invention provides a method for preparation of fully ¹³C isotopically labeled biomolecules by biosynthesis in a mammalian cell line, which comprises the following steps:
a) providing a mammalian cell line capable of growing in serum-free media;
b) culturing the cell line obtained in step b) in a serum-free medium substantially composed of:
   - fully ¹³C isotopically labeled amino acids,
   - fully ¹³C isotopically labeled glucose and/or fully ¹³C isotopically labeled galactose,
   - fully ¹³C isotopically labeled choline or ¹³C isotopically labeled choline,
   - vitamins,
   - distilled water,
   - insulin,
   - transferrin,
   - sodium selenite, and
   - inorganic salts wherein when the inorganic salt contains carbon, it is fully ¹³C isotopically labeled;
c) obtaining ¹³C isotopically labeled biomolecules from the cells or medium after the cultivation of step b).

In one embodiment, the present invention provides a method for preparation of biomolecules having a predetermined ¹³C isotopic labeling by biosynthesis in a mammalian cell line, which comprises the following steps:
a) providing a mammalian cell line capable of growing in serum-free media;
b) culturing the cell line obtained in step b) in a serum-free medium containing:
   - ¹³C isotopically labeled amino acids,
   - ¹³C isotopically labeled glucose and/or ¹³C isotopically labeled galactose,
   - ¹³C isotopically labeled choline,
   - vitamins,
   - distilled water,
   - insulin,
   - transferrin,
   - sodium selenite, and
   - inorganic salts wherein when the inorganic salt contains carbon, it is optionally fully ¹³C isotopically labeled;
   wherein the amino acids, glucose and/or galactose, and/or choline are ¹³C isotopically labeled in positions corresponding to the predetermined ¹³C isotopic labeling of the produced biomolecule(s),
c) obtaining ¹³C isotopically labeled biomolecules from the cells or medium after the cultivation of step b).

The mammalian cell line is preferably a cancer cell line.

The mammalian cell line is preferably a human cell line, more preferably a human cancer cell line.

The mammalian cell line is preferably a non-adherent cell line (suspension cell line).

Most preferably, the human cell line is HL-60 cell line.

In a preferred embodiment of step a), a cell line is cultured in a cell growth medium supplemented with fetal bovine serum, wherein over several passages the concentration of fetal bovine serum is reduced until zero content of fetal bovine serum in the cell growth medium is reached. This preferred embodiment of step a) serves to acclimatize the human cell line to growth in a medium without fetal bovine serum (or, in a serum-free medium).

A cell growth medium for the preferred embodiment of step a) is a medium which is suitable for culturing the cell line used. Typical cell growth media are, for example, RPMI, MEM or DMEM media. The medium in the preferred embodiment of step a) is preferably supplemented by glutamine, antibiotic and/or antimycotic. More preferably, glutamine is supplemented in the amount of 0.2 to 3.5 mmol/L, even more preferably 2 mmol/L. More preferably, the antibiotic is a mixture of penicillin and streptomycin, even more preferably in an amount of about 50 U/mL penicillin, about 50 µg/mL streptomycin.

In a particularly preferred embodiment, the cell growth medium is initially supplemented with 10 % (v/v) fetal bovine serum (FBS), and the amount of the FBS is reduced to 0 % during 10 to 20 days, preferably during 13 to 15 days.

Preferably, the culture in the preferred embodiment of step a) is carried out at standard conditions, i.e. 5% CO₂ at 37 °C.

It is noted that any other known procedure for obtaining a cell line capable of growing in serum-free medium can be used.

Subsequently to step a) but prior to step b), the cell line capable of growing in serum-free medium may further be cultured in the cell growth medium wherein the content of CO₂ is gradually reduced, preferably the content of CO₂ is reduced down to 2.5 % CO₂.

It has been observed by the inventors that reducing the content of CO₂ below 2.5 % has a negative effect on viability of the cells during a subsequent long-term cultivation. For a subsequent short-term cultivation, the content of CO₂ can be reduced even below 2.5 % CO₂.

In step b), the culture medium contains exclusively ¹³C labeled nutrients for production of fully ¹³C labeled biomolecules, or nutrients ¹³C labeled in predetermined positions for production of biomolecules having a predetermined ¹³C isotopic labeling. These nutrients include naturally occurring amino acids (which may optionally be in the form of salts and/or hydrates), choline and glucose and/or galactose. The naturally occurring amino acids are fully ¹³C labeled for production of fully ¹³C labeled biomolecules, or only certain amino acids, or only certain positions of amino acids may be ¹³C labeled for production of biomolecules having a predetermined ¹³C isotopic labeling.

The set of amino acids preferably contains or consists of at least the following 13 amino acids: L-arginine (more preferably in the form of monohydrochloride), L-histidine (more preferably in the form of monohydrochloride), L-isoleucine, L-leucin, L-lysine (more preferably in the form of monohydrochloride), L-methionine, L-threonine, L-phenylalanine, L-tryptophan, L-tyrosine, L-valine, L-glutamine, L-cysteine. The set of amino acids may further contain one or more of the following amino acids: glycine, L-asparagine (more preferably in the form of monohydrate), L-proline, L-serine, L-alanine, L-glutamic acid, L-aspartic acid.

Preferably, the set of amino acids contains or consists of at least 15, more preferably at least 16, even more preferably at least 17, yet more preferably at least 18, yet more preferably at least 19 naturally occurring amino acids, most preferably 20 naturally occurring amino acids. Typically, cells grow and propagate more slowly when the amount of naturally occurring amino acids is below 18 or 19.

The culture medium in step b) may optionally contain also fully ¹³C labeled non-naturally occuring amino acids, if it is needed for the production of the desired biomolecule(s).

D-glucose and/or D-galactose, which is another important nutrient, shall be fully ¹³C labeled for production of fully ¹³C labeled biomolecules, or only certain positions of glucose/galactose may be ¹³C labeled for production of biomolecules having a predetermined ¹³C isotopic labeling.

Choline (more preferably in the form of chloride) preferably contains at least two ¹³C atoms, more preferably all atoms in choline are ¹³C atoms for production of fully ¹³C labeled biomolecules, or only certain positions of choline may be ¹³C labeled for production of biomolecules having a predetermined ¹³C isotopic labeling.

Furthermore, the culture medium in step b) contains vitamins needed for the growth and metabolic processes of the human cells. In most cases, atoms of these vitamins are not incorporated into the metabolic products of the cells, as the vitamins act as catalysts, and thus the vitamins do not need to be ¹³C labeled. They may be ¹³C labeled, though.

Some vitamins, such as niacin and myoinositol, are incorporated into the biomolecules produced by the cell. It is thus preferred that these vitamins are ¹³C labeled or fully ¹³C labeled, if the corresponding biomolecules are of interest as products of the present invention. These vitamins may be not need to be ¹³C labeled if the corresponding biomolecules are not of interest as products, or if the small portions of the corresponding biomolecules coming from these vitamins can contain ¹²C atoms while still complying with the requirements for products (e.g., as internal standards). In any case, major part of these biomolecules will contain ¹³C atoms.

The set of vitamins preferably contains or consists of at least the following vitamins: pantothenate (more preferably in the form of calcium salt), folic acid, niacinamide, pyridoxine (more preferably in the form of hydrochloride) and/or pyridoxal, riboflavin, thiamine (more preferably in the form of hydrochloride), and I-inositol and/or myo-inositol. The set of vitamins may optionally further contain one or more of the further vitamins: biotin, vitamin B12, p-aminobenzoic acid.

Yet furthermore, the culture medium in step b) contains inorganic salts to participate in metabolic processes in the human cell line, as well as to maintain ionic strength, osmolality and osmolarity of the medium. The inorganic salts are salts of metal cations and anions derived from inorganic acids. The metal cations preferably contain or consist of at least the following cations: sodium(I), calcium(II), magnesium(II), potassium(I). Furthermore, the metal cations may further contain one or more of: iron(III), zinc(II), copper(II). The anions preferably contain or consist of the following anions: bicarbonate (preferably fully ¹³C labeled), sulfate, chloride, phosphate monobasic. The metal anions may further contain one or more of: nitrate, phosphate dibasic.

The set of inorganic salts preferably contains or consists of at least the following salts: calcium chloride, magnesium sulfate, potassium chloride, sodium chloride, sodium phosphate monobasic (preferably in the form of monohydrate). The set of inorganic salts may further contain one or more of: sodium bicarbonate (fully ¹³C labeled), magnesium chloride, iron(III) nitrate (preferably in the form of a nonahydrate), ferric sulfate, zinc sulfate, copper sulfate, sodium phosphate dibasic.

Particularly preferably, the culture medium in step c) contains at least some of the following components in the following amounts:

| **Component** | **Summary formula** | **Preferred concentration interval [mg/L]** | **Most preferred concentration [mg/L]** |
|---|---|---|---|
| L-Arginine monohydrochloride (all ¹³C) | ¹³C₆H₁₄N₄O₂ · HCl | 5-250 | 147.5 |
| L-Histidine monohydrochloride monohydrate (all ¹³C) | ¹³C₆H₉N₃O₂ · HCl · H₂O | 5-50 | 31.48 |
| L-Isoleucine (all ¹³C) | ¹³C₆H₁₃NO₂ | 3-70 | 54.47 |
| L-Leucine (all ¹³C) | ¹³C₆H₁₃NO₂ | 3-70 | 59.05 |
| L-Lysine monohydrochloride (all ¹³C) | ¹³C₆H₁₄N₂O₂ · HCl | 10-150 | 91.25 |
| L-Methionine (all ¹³C) | ¹³C₅H₁₁NO₂S | 1-30 | 17.24 |
| L-Threonine (all ¹³C) | ¹³C₄H₉NO₃ | 5-70 | 53.45 |
| L-Phenylalanine (all ¹³C) | ¹³C₉H₁₁NO₂ | 3-50 | 35.48 |
| L-Tryptophan (all ¹³C) | ¹³C₁₁H₁₂N₂O₂ | 1-30 | 9.02 |
| L-Tyrosine (all ¹³C) | ¹³C₉H₁₁NO₃ | 3-70 | 55.79 |
| L-Valine (all ¹³C) | ¹³C₅H₁₁NO₂ | 7-70 | 52.85 |
| L-Glutamine (all ¹³C) | ¹³C₅H₁₀N₂O₃ | 20-500 | 365.0 |
| L-Cysteine (all ¹³C) | ¹³C₃H₇NO₂S | 10-150 | 100.0 |
| Glycine (all ¹³C) | ¹³C₂H₅NO₂ | 0-40 | 18.75 |
| L-Asparagine monohydrate (all ¹³C) | ¹³C₄H₈N₂O₃ · H₂O | 0-60 | 7.5 |
| L-Proline (all ¹³C) | ¹³C₅H₉NO₂ | 0-60 | 17.25 |
| L-Serine (all ¹³C) | ¹³C₃H₇NO₃ | 0-50 | 26.25 |
| L-Alanine (all ¹³C) | ¹³C₃H₇NO₂ | 0-60 | 4.5 |
| L-Glutamic acid (all ¹³C) | ¹³C₅H₉NO₄ | 0-30 | 7.35 |
| L-Aspartic acid (all ¹³C) | ¹³C₄H₇NO₄ | 0-30 | 6.65 |
| D-Glucose (all ¹³C) | ¹³C₆H₁₂O₆ | 180-6000 | 3150.0 |
| Choline chloride (fully ¹²C or ¹³C₂) | C₃H₁₄NOCl or ¹³C₂¹²C₃H₁₄NOCl | 0.2-15 | 3.0 |
| Biotin^{a} | C₁₀H₁₆N₂O₃S | 0.00001-0.3 | 0.2 |
| D-Calcium pantothenate^{a} | C₁₈H₃₂CaN₂O₁₀ | 0.01-2 | 0.25 |
| Folic acid^{a} | C₁₉H₁₉N₇O₆ | 0.002-4 | 1.0 |
| Niacinamide^{a} | C₆H₆N₂O | 0.005-3 | 1.0 |
| Pyridoxine hydrochloride^{a} | C₈H₁₁NO₃ · HCl | 0.003-3 | 1.0 |
| Riboflavin^{a} | C₁₇H₂₀N₄O₆ | 0.1-0.5 | 0.2 |
| Thiamine hydrochloride^{a} | C₁₂H₁₇N₄OS · HCl | 0.01-3 | 1.0 |
| Vitamin B12^{a} | C₆₃H₈₈CoN₁₄O₁₄P | 0.0001-0.9 | 0.005 |
| Myo-inositol^{a} | C₆H₁₂O₆ | 0.000005-50 | 35.0 |
| p-Aminobenzoic acid^{a} | C₇H₇NO₂ | 0.1-20 | 1.0 |
| Sodium bicarbonate (all ¹³C) | NaH¹³CO₃ | 0-5000 | 1200.0 |
| Iron(III) nitrate nonahydrate | Fe(NO₃)₃ · 9H₂O | 0-0.1 | 0.05 |
| Ferric sulfate | FeSO₄ · 7H₂O | 0-2 | 0.417 |
| Zinc sulfate | ZnSO₄ · 7H₂O | 0-2 | 0.432 |
| Cupric sulfate | CuSO₄ · 5H₂O | 0-0.01 | 0.0013 |
| Calcium chloride | CaCl₂ · 2H₂O | 20-200 | 154.5 |
| Magnesium sulfate | MgSO₄ | 0-130 | 48.84 |
| Potassium chloride | KCl | 150-500 | 311.8 |
| Sodium chloride | NaCl | 4000-10000 | 6996.0 |
| Sodium phosphate dibasic | Na₂HPO₄ | 0-300 | 71.02 |
| Sodium phosphate monobasic monohydrate | NaH₂PO₄ | 0-300 | 54.3 |
| Magnesium chloride | MgCl₂ · 6H₂O | 40-150 | 61.2 |
| Calcium nitrate | Ca(NO₃)₂ · 4H₂O | 0-200 | 100.0 |

The supplementation of culture medium with insulin, transferrin and sodium selenite is necessary to ensure sustainable cell growth. Insulin and transferrin may be ¹³C labeled/enriched or may contain natural distribution of ¹³C carbons and ¹²C carbons.

In a preferred embodiment, the concentrations of the supplements are: 5 to 20 µg/mL insulin (preferably about 10 µg/mL insulin), 3 to 10 µg/mL transferrin (preferably about 5.5 µg/mL transferrin), 3 to 10 ng/mL sodium selenite (preferably about 5 ng/mL sodium selenite).

In step c), the ¹³C labeled biomolecules can be obtained from culture supernatant or from lysed cells.

The ¹³C labeled biomolecules obtained by the method of the invention may be used, for example, as internal standards for analytical methods, in particular for metabolomic analyses, or as standards or markers in biological and biotechnological research.

Biomolecules include, in particular, lipids, lipoproteins, small molecules involved in metabolomics (i.e., amino acids and their derivates, nucleic bases, nucleosides, nucleotides, saccharides and their phosphates, small organic acids and alcohols), proteins, peptides and polysaccharides produced in mammalian, in particular human, cells as metabolites or in cellular processes as functional proteins, lipids, peptides, polysaccharides or lipoproteins.

The biomolecules may be obtained from the supernatant and/or from the cells. They may be used as a mixture - especially as internal standards, or desired biomolecules may be isolated from the mixture. The use of mammalian cell lines for producing the labeled biomolecules is particularly advantageous when the obtained mixture of biomolecules is used as a standard, as the composition of the mixture is close to the composition of biomolecules in human or more generally mammalian cells.

### Brief description of drawings

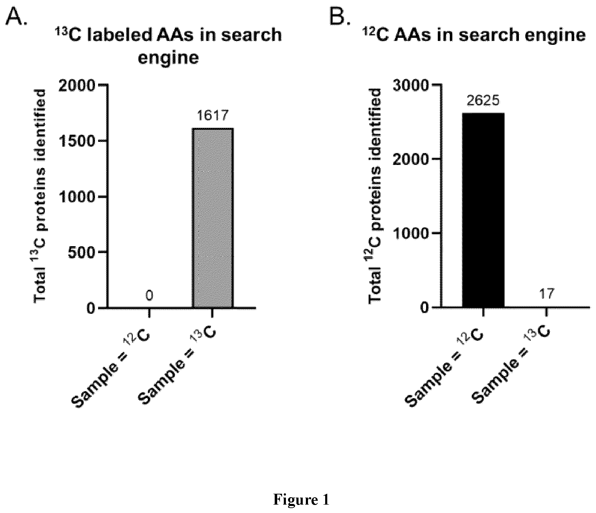

Figure 1: Total number of proteins identified in Proteome Discoverer (v3.0) using the Mascot search engine under different labeling conditions: (A) all amino acids (AAs) set as fully labeled with ¹³C; and (B) all AAs set as non-labeled (¹²C). The use of ¹³C labeling resulted in a decrease in the total number of identified proteins; however, no "light" false positives were observed. Despite the reduction, the identification of 1617 proteins remains sufficient for global comparative proteomic analyses. Under the standard setup without ¹³C labeling, the software identified 2625 proteins, but with 17 false-positive hits.

### Detailed description

The present invention provides a method that allows the preparation of stable isotopically labeled ¹³C isotopes of commonly present human biomolecules with the primary focus on lipids and metabolites and also illustrates the complete isotopic conversion of proteins. The following description shows some preferred embodiments of the invention, which should not be construed as limiting the scope of protection which is determined solely by the claims.

The method is based on the cultivation of specific human-derived cancer cell lines wherein almost all nutrients involved in the biosynthesis are fully labeled with ¹³C (choline is currently commercially available only partially labeled with ¹³C) (Table 1) or labeled with ¹³C in positions corresponding to the desired biomolecule, without the need for ¹²C fetal bovine serum. Several unlabeled compounds are present in culture medium with natural ¹²C isotopic distribution to maintain cell proliferation and viability. The key advantage of this strategy compared to prior art is the fact that we obtain fully (or in predetermined positions) ¹³C labeled biomolecules with high isotopic purity >97% with the same pattern and concentrations similar to those of common human biological samples, enabling the direct use for multiomics quantitation based on MS using ¹³C labeled IS for each quantified analyte. The nutrients shall be ¹³C labeled with high isotopic purity >97%.

**Table 1. Formulation of ¹³C culture medium.**

| **Component** | **Isotopic purity [%]** | **Summary formula** | **Molecular weight [g/mol]** | **Concentration [mg/L]** |
|---|---|---|---|---|
| L-Arginine monohydrochloride (all ¹³C) | 99 | ¹³C₆H₁₄N₄O₂ · HCl | 216.6 | 147.5 |
| L-Histidine monohydrochloride monohydrate (all ¹³C) | 97 - 99 | ¹³C₆H₉N₃O₂ · HCl · H₂O | 215.6 | 31.48 |
| L-Isoleucine (all ¹³C) | 99 | ¹³C₆H₁₃NO₂ | 137.1 | 54.47 |
| L-Leucine (all ¹³C) | 99 | ¹³C₆H₁₃NO₂ | 137.1 | 59.05 |
| L-Lysine monohydrochloride (all ¹³C) | 99 | ¹³C₆H₁₄N₂O₂ · HCl | 225.1 | 91.25 |
| L-Methionine (all ¹³C) | 99 | ¹³C₅H₁₁NO₂S | 154.2 | 17.24 |
| L-Threonine (all ¹³C) | 97 - 99 | ¹³C₄H₉NO₃ | 123.1 | 53.45 |
| L-Phenylalanine (all ¹³C) | 99 | ¹³C₉H₁₁NO₂ | 174.1 | 35.48 |
| L-Tryptophan (all ¹³C) | 99 | ¹³C₁₁H₁₂N₂O₂ | 215.1 | 9.02 |
| L-Tyrosine (all ¹³C) | 99 | ¹³C₉H₁₁NO₃ | 190.1 | 55.79 |
| L-Valine (all ¹³C) | 99 | ¹³C₅H₁₁NO₂ | 122.1 | 52.85 |
| L-Glutamine (all ¹³C) | 99 | ¹³C₅H₁₀N₂O₃ | 151.1 | 365.0 |
| L-Cysteine (all ¹³C) | 99 | ¹³C₃H₇NO₂S | 124.1 | 100.0 |
| Glycine (all ¹³C) | 97 - 99 | ¹³C₂H₅NO₂ | 77.1 | 18.75 |
| L-Asparagine monohydrate (all ¹³C) | 99 | ¹³C₄H₈N₂O₃ · H₂O | 154.1 | 7.5 |
| L-Proline (all ¹³C) | 99 | ¹³C₅H₉NO₂ | 120.1 | 17.25 |
| L-Serine (all ¹³C) | 99 | ¹³C₃H₇NO₃ | 108.1 | 26.25 |
| L-Alanine (all ¹³C) | 99 | ¹³C₃H₇NO₂ | 92.1 | 4.5 |
| L-Glutamic acid (all ¹³C) | 99 | ¹³C₅H₉NO₄ | 152.1 | 7.35 |
| D-Glucose (all ¹³C) | 99 | ¹³C₆H₁₂O₆ | 186.1 | 3150.0 |
| Choline chloride (fully ¹²C or ¹³C₂) | 99 | C₃H₁₄NOCl or ¹³C₂¹²C₃H₁₄NOCl | 139.6 or 141.6 | 3.0 |
| Biotin^{a} | - | C₁₀H₁₆N₂O₃S | 244.3 | 0.2 |
| D-Calcium pantothenate ^{a} | - | C₁₈H₃₂CaN₂O₁₀ | 477.5 | 0.25 |
| Folic acid^{a} | - | C₁₉H₁₉N₇O₆ | 441.4 | 1.0 |
| Niacinamide^{a} | - | C₆H₆N₂O | 122.1 | 1.0 |
| Pyridoxine hydrochloride^{a} | - | C₈H₁₁NO₃ · HCl | 205.6 | 1.0 |
| Riboflavin^{a} | - | C₁₇H₂₀N₄O₆ | 376.4 | 0.2 |
| Thiamine hydrochloride^{a} | - | C₁₂H₁₇N₄OS · HCl | 337.3 | 1.0 |
| Vitamin B12^{a} | - | C₆₃H₈₈CoN₁₄O₁₄P | 1355.0 | 0.005 |
| Myo-inositol^{a} | - | C₆H₁₂O₆ | 180.2 | 35.0 |
| p-Aminobenzoic acid^{a} | - | C₇H₇NO₂ | 137.1 | 1.0 |
| Sodium bicarbonate (all ¹³C) | 99 | NaH¹³CO₃ | 85.0 | 1200.0 |
| Iron(III) nitrate nonahydrate | - | Fe(NO₃)₃ · 9H₂O | 404.0 | 0.05 |
| Ferric sulfate | - | FeSO₄ · 7H₂O | 278.0 | 0.417 |
| Zinc sulfate | - | ZnSO₄ · 7H₂O | 288.0 | 0.432 |
| Cupric sulfate | - | CuSO₄ · 5H₂O | 250.0 | 0.0013 |
| Calcium chloride | - | CaCl₂ · 2H₂O | 111.0 | 154.5 |
| Magnesium sulfate | - | MgSO₄ | 120.4 | 48.84 |
| Potassium chloride | - | KCI | 75.0 | 311.8 |
| Sodium chloride | - | NaCl | 58.4 | 6996.0 |
| Sodium phosphate dibasic | - | Na₂HPO₄ | 142.0 | 71.02 |
| Sodium phosphate monobasic monohydrate | - | NaH₂PO₄ | 138.0 | 54.3 |
| Magnesium chloride | - | MgCl₂ · 6H₂O | 288.0 | 61.2 |

| | | | | |
|---|---|---|---|---|
| ^{'.a} Unlabeled compounds presented in culture medium with natural ¹²C isotopic distribution to maintain cell proliferation and viability. | | | | |

### Biosynthesis of ¹³C labeled biomolecules

The HL-60 cell line (ATCC No. CCL-240) containing natural composition of ¹²C/¹³C were used and gradually accustomed to the change of environment necessary for the synthesis of ¹³C labeled biomolecules. The HL-60 cell line was cultured in commercial Dulbecco's modified Eagle's medium (DMEM/F12 = 1:1, Merck) with 10% (v/v) fetal bovine serum, 2 mmol/L glutamine, 50 µg/mL penicillin/streptomycin solution (all purchased from Invitrogen-Gibco) in an atmosphere of 5% CO₂ at 37 °C for 1 week. Then HL-60 cells were cultured in medium with a reduction in the concentration of fetal bovine serum medium from 10% to 0% for 14 days. When the concentration of fetal bovine serum was 0%, the medium was supplemented with 10 µg/mL insulin, 5.5 µg/mL transferrin, and 5 ng/mL sodium selenite to ensure sustainable cell growth. Finally, the atmosphere of growing cells was changed to 2.5% CO₂.

Then HL-60 cells began to grow in the medium consisting of ¹³C nutrients shown in Table 1, distilled water, 50 U/mL penicillin, 50 µg/mLstreptomycin and supplemented with 10 µg/mL insulin, 5.5 µg/mL transferrin, and 5 ng/mL sodium selenite. All compounds were dissolved in the distilled water to form ¹³C culture medium. Cells were cultivated in medium with ¹³C-substrates in 6-well plates and 75 cm² culture flasks. The initial density of the cells was 4×10⁵ cells per well in a 6-well plate and the culture medium was changed every 4 days. The cells were harvested after 18 days of cultivation (see Table 2). Cell aliquots were prepared and centrifuged at 1500g for 5 min and the supernatant was removed. The cell pellets containing 2 million cells were then put in liquid nitrogen to ensure cell lysis and stored at - 80 °C until analyzed.

**Table 2. Doubling time and population doublings of HL-60 cells cultured in ¹³C culture medium.**

| **Day of cultivation** | **Passage number** | **Doubling time (h)** | **Population doublings** |
|---|---|---|---|
| 3 | 10 | 31.9 | 2.3x |
| 7 | 11 | 30.8 | 5.4x |
| 10 | 12 | 25.0 | 8.3x |
| 14 | 13 | 26.2 | 11.9x |
| 18 | 14 | 31.2 | 15x |

### Verification of isotopic purity based on LC/MS

The isotopic purity of the input chemicals and the final ¹³C biosynthetic products is verified based on the isotopic ratio that can be measured by the method allowing the determination of elemental composition and the abundance of individual isotopes within the compound studied, namely using chromatographic technique combined with high resolution (HR) mass spectrometry resulting in separation of complex matrix and high mass accuracy by MS. In the final product of human cancer cell line cultured in ¹³C medium, the mass of ¹³C labeled compounds is shifted by mass units corresponding to the number of ¹³C carbons. The elemental compositions and percent abundances of individual isotopes are calculated using LC/MS based on the typical isotopic pattern of observed ions, including exact masses and ratio of individual isotopic peaks (see below). The composition of all biomolecules in the initial cell line corresponds to the normal natural isotopic distribution. The percentage of ¹²C isotopes in natural molecules is 98.9%, while the remaining 1.1% corresponds to the ¹³C isotope. The isotopic purity of the biosynthesized ¹³C labeled compounds (products of cell line cultivated in a ¹³C environment) depends on the isotopic purity of the input chemicals used in the nutrient for cell cultivation including nature isotopes. This means that if the isotopic purity of input chemicals for biosynthesis is 98%, the isotopic purity of the final ¹³C labeled biosynthesized products cannot be higher than 98%. For the biosynthesis of ¹³C labeled standards, the isotopic purity of input fully ¹³C labeled chemicals used in the nutrient medium must be of high purity, minimally 97%, preferably higher than 98%, and more preferably higher than 98.5%.

### Isolation and analysis of biomolecules present in HL-60 cell lines

Lipids and metabolites were isolated from cells using modified Folch extraction described elsewhere [D. Wolrab, M. Chocholoušková, R. Jirásko, O. Peterka, M. Holčapek, Anal. Bioanal. Chem. 412 (2020) 2375]. Individual samples were measured using methods based on the coupling of LC/MS with high resolution and the ion currents for a given value of m/z (ion mass of interest) in a narrow isolation window ± 0.003 Da were reconstructed. Subsequently, we verified whether a chromatographic peak was present for this mass and displayed the averaged mass spectrum of this peak. Each ¹³C and ¹²C molecules have a specific retention time and are ionized to form specific ions (see **Tables 3, 4, and 5),** which consist of the respective isotopic peaks with specific relative abundances (their intensities). Based on the characteristic isotopic pattern and accurate masses of ions observed in the mass spectrum, the elemental composition and isotopic purity of the identified biomolecules were calculated. Graphical dependences of retention times on the carbon number or the number of double bonds in fatty acyl chains were constructed to support the identification of lipid species in homologous lipid series found in the studied cell lines. All lipid species within the individual lipid classes listed in Table 4 showed characteristic retention behavior and fit well with a second-degree polynomial regression. Generally, the chromatographic retention time increases with a higher number of carbons and decreases with an increasing number of double bonds for lipid species within each lipid class.

At least 63 small metabolites were found in the extract of ¹³C cell line and 764 lipid species within 26 lipid classes were found in the extract of ¹³C cell line, namely, lysophosphatidylcholines (LPC), ether-lysophosphatidylcholines (LPC O-), lysophosphatidylethanolamines (LPE), ether-lysophosphatidylethanolamines (LPE O-), lysophosphatidylserines (LPS), ether-lysophosphatidylserines (LPS O-), lysophosphatidylglycerols (LPG), lysophosphatidylinositols (LPI), phosphatidylcholines (PC), ether-phosphatidylcholines (PC O-), phosphatidylethanolamines (PE), ether-phosphatidylethanolamines (PE O-), phosphatidylinositols (PI), phosphatidylglycerols (PG), phosphatidylserines (PS), phosphatidic acids (PA), cardiolipins (CL), sphingomyelins (SM), ceramides (Cer), hexosylceramides (HexCer), dihexosylceramides (Hex2Cer), tetrahexosylceramides (Hex4Cer), gangliosides (GM3), sterols, diacylglycerols (DG), triacylglycerols (TG). **Tables 3, 4, and 5** show the list of representative lipids, metabolites, and proteins found in initial ¹²C cell line and ¹³C cell line together with their elemental composition, isotopic purity and their relative abundances found in cell line. Importantly, the composition of biomolecules in human cell line is similar to that of human plasma, as shown by the comparison of relative abundances in individual samples. The extract of ¹³C cell line is added to the biological sample resulting in one labeled internal standard per each analyte, which is a new concept of comprehensive multiomics quantitation that has not been used so far.

**Table 3. Examples of particular ¹³C labeled small biomolecules found in the cell line cultivated with ¹³C labeled substrates and their ¹²C analogue found in the cell line cultivated under normal ¹²C conditions (nd abbreviation means not determined).**

| **Name of small molecule** | **t_{R} [min]** | **Elemental Composition (neutral form)** | | **Experimental ¹³C isotopic purity [%]** | **Relative abundance in samples** | |
|---|---|---|---|---|---|---|
| | | **¹²C cell lines** | **¹³C cell lines** | | **¹³C cell lines** | **¹²C cell lines** |
| **amino acids** | | | | | | |
| N-Acetyl-L-alanine | 4.10 | C₅H₉NO₃ | ¹³C₅H₉NO₃ | 98.1 | 0.1 | 0.1 |
| Phenylalanine | 4.48 | C₉H₁₁NO₂ | ¹³C₉H₁₁NO₂ | 99.5 | 4.6 | 4.3 |
| leucine | 4.58 | C₆H₁₃NO₂ | ¹³C₆H₁₃NO₂ | 99.3 | 9.7 | 8.5 |
| Tryptophan | 4.62 | C₁₁H₁₂N₂O₂ | ¹³C₁₁H₁₂N₂O₂ | 99.2 | 1.0 | 1.4 |
| isoleucine | 4.78 | C₆H₁₃NO₂ | ¹³C₆H₁₃NO₂ | 99.2 | 7.9 | 7.1 |
| Methionine | 5.10 | C₅H₁₁NO₂S | ¹³C₅H₁₁NO₂S | 99.1 | 0.4 | 0.5 |
| valine | 5.30 | C₅H₁₁NO₂ | ¹³C₅H₁₁NO₂ | 99.3 | 2.8 | 2.7 |
| Tyrosine | 5.43 | C₉H₁₁NO₃ | ¹³C₉H₁₁NO₃ | 99.3 | 6.9 | 4.3 |
| Proline | 5.52 | C₅H₉NO₂ | ¹³C₅H₉NO₂ | 98.7 | 8.6 | 6.5 |
| alanine | 6.35 | C₃H₇NO₂ | ¹³C₃H₇NO₂ | 98.4 | 0.3 | 0.3 |
| Threonine | 6.40 | C₄H₉NO₃ | ¹³C₄H₉NO₃ | 99.3 | 1.5 | 1.5 |
| glycine | 6.78 | C₂H₅NO₂ | ¹³C₂H₅NO₂ | 98.9 | 0.8 | 1.0 |
| Glutamine | 7.00 | C₅H₁₀N₂O₃ | ¹³C₅H₁₀N₂O₃ | 99.2 | 44.9 | 50.1 |
| serine | 7.05 | C₃H₇NO₃ | ¹³C₃H₇NO₃ | 99.3 | 0.8 | 1.0 |
| Asparagine | 7.08 | C₄H₈N₂O₃ | ¹³C₄H₈N₂O₃ | 98.6 | 0.6 | 0.9 |
| Histidine | 7.20 | C₆H₉N₃O₂ | ¹³C₆H₉N₃O₂ | 99.2 | 1.0 | 0.4 |
| Glutamic acid | 7.36 | C₅H₉NO₄ | ¹³C₅H₉NO₄ | 98.7 | 2.6 | 2.6 |
| Aspartic acid | 7.58 | C₄H₇NO₄ | ¹³C₄H₇NO₄ | 98.2 | 0.2 | 0.4 |
| Cysteine | 8.50 | C₃H₇NO₂S | ¹³C₃H₇NO₂S | 99.8 | 0.05 | 0.01 |
| Cystine | 9.95 | C₆H₁₂N₂O₄S₂ | ¹³C₆H₁₂N₂O₄S₂ | 98.8 | 0.7 | 0.8 |
| Ornithine | 11.12 | C₅H₁₂N₂O₂ | ¹³C₅H₁₂N₂O₂ | 99.3 | 3.4 | 4.2 |
| Arginine | 11.12 | C₆H₁₄N₄O₂ | ¹³C₆H₁₄N₄O₂ | 99.3 | 1.4 | 1.5 |
| Lysine | 11.45 | C₆H₁₄N₂O₂ | ¹³C₆H₁₄N₂O₂ | 99.3 | 1.8 | 0.4 |

| **nucleic bases and nucleosides** | | | | | | |
|---|---|---|---|---|---|---|
| Methylthioadenosine | 2.23 | C₁₁H₁₅N₅O₃S | ¹³C₁₀C₁H₁₅N₅O₃S | 98.5 | 8.3 | 6.2 |
| hypoxantine | 3.05 | C₅H₄N₄O | ¹³C₄C₁H₄N₄O | 97.4 | 29.0 | 30.6 |
| Uridine | 3.10 | C₉H₁₂N₂O₆ | ¹³C₈C₁H₁₂N₂O₆ | 98.0 | 3.3 | 1.1 |
| Adenosine | 3.16 | C₁₀H₁₃N₅O₄ | ¹³C₉C₁H₁₃N₅O₄ | 97.6 | 7.9 | 5.4 |
| inosine | 3.73 | C₁₀H₁₂N₄O₅ | ¹³C₉C₁H₁₂N₄O₅ | 97.8 | 30.2 | 19.1 |
| Cytosine | 3.97 | C₄H₅N₃O | ¹³C₃C₁H₅N₃O | 97.5 | 10.5 | 7.5 |
| Guanine | 4.05 | C₅H₅N₅O | ¹³C₄C₁H₅N₅O | 97.4 | 5.7 | 20.5 |
| Cytidine | 4.34 | C₉H₁₃N₃O₅ | ¹³C₈C₁H₁₃N₃O₅ | 98.6 | 5.1 | 9.7 |

| **nucleotides** | | | | | | |
|---|---|---|---|---|---|---|
| AMP | 7.91 | C₁₀H₁₄N₅O₇P | ¹³C₉C₁H₁₄N₅O₇P | 97.6 | 11.3 | 11.3 |
| UMP | 8.16 | C₉H₁₃N₂O₉P | ¹³C₈C₁H₁₃N₂O₉P | 97.8 | 3.6 | 5.3 |
| IMP | 8.37 | C₁₀H₁₃N₄O₈P | ¹³C₉C₁H₁₃N₄O₈P | 97.6 | 8.6 | 23.5 |
| NAD | 8.38 | C₂₁H₂₇N₇O₁₄P₂ | ¹³C₁₄C₇H₂₇N₇O₁₄P₂ | 98.0 | 1.1 | 0.7 |
| CMP | 8.69 | C₉H₁₄N₃O₈P | ¹³C₈C₁H₁₄N₃O₈P | 98.0 | 1.4 | 1.9 |
| GMP | 8.86 | C₁₀H₁₄N₅O₈P | ¹³C₉C₁H₁₄N₅O₈P | 97.7 | 2.5 | 4.5 |
| ADP | 8.88 | C₁₀H₁₅N₅O₁₀P₂ | ¹³C₉C₁H₁₅N₅O₁₀P₂ | 97.5 | 22.3 | 15.8 |
| UDP | 9.16 | C₉H₁₄N₂O₁₂P₂ | ¹³C₈C₁H₁₄N₂O₁₂P₂ | 97.9 | 6.2 | 5.8 |
| CDP | 9.52 | C₉H₁₅N₃O₁₁P₂ | C₈C₁H₁₅N₃O₁₁P₂ | 97.6 | 1.7 | 1.6 |
| GDP | 9.74 | C₁₀H₁₅N₅O₁₁P₂ | ¹³C₉C₁H₁₅N₅O₁₁P₂ | 97.7 | 5.0 | 4.2 |
| ATP | 9.80 | C₁₀H₁₆N₅O₁₃P₃ | ¹³C₉C₁H₁₆N₅O₁₃P₃ | 97.5 | 22.5 | 14.0 |
| UTP | 10.13 | C₉H₁₅N₂O₁₅P₃ | ¹³C₈C₁H₁₅N₂O₁₅P₃ | 98.1 | 7.1 | 6.0 |
| CTP | 10.42 | C₉H₁₆N₃O₁₄P₃ | ¹³C₈C₁H₁₆N₃O₁₄P₃ | 97.7 | 3.0 | 2.6 |
| GTP | 10.63 | C₁₀H₁₆N₅O₁₄P₃ | ¹³C₉C₁H₁₆N₅O₁₄P₃ | 97.5 | 3.8 | 2.6 |

| **saccharides and their phosphates** | | | | | | |
|---|---|---|---|---|---|---|
| D-glukose/D-galactose | 5.25 | C₆H₁₂O₆ | ¹³C₆H₁₂O₆ | 98.8 | 35.9 | 30.6 |
| Myoinositol | 7.18 | C₆H₁₂O₆ | ¹³C₆H₁₂O₆ | nd | 50.3 | 51.2 |
| α-Lactose | 6.65 | C₁₂H₂₂O₁₁ | ¹³C₁₂H₂₂O₁₁ | 99.3 | 0.8 | 0.9 |
| Hexose phosphate A | 8.70 | C₆H₁₃O₉P | ¹³C₆H₁₃O₉P | 98.9 | 1.3 | 0.5 |
| Hexose phosphate B | 8.80 | C₆H₁₃O₉P | ¹³C₆H₁₃O₉P | 99.6 | 0.9 | 1.3 |
| Hexose phosphate C | 8.95 | C₆H₁₃O₉P | ¹³C₆H₁₃O₉P | 99.0 | 0.5 | 0.5 |
| Hexose phosphate D | 9.15 | C₆H₁₃O₉P | ¹³C₆H₁₃O₉P | 98.8 | 0.3 | 0.5 |
| D-glucose 6-phosphate | 9.28 | C₆H₁₃O₉P | ¹³C₆H₁₃O₉P | 98.7 | 1.7 | 1.9 |
| Pentose phosphate | 8.2 | C₅H₁₁O₈P | ¹³C₅H₁₁O₈P | 99.6 | 0.4 | 0.4 |
| Gluconic acid | 6.0 | C₄H₁₂O₇ | ¹³C₆H₁₂O₇ | 98.8 | 6.6 | 11.0 |
| Glucuronic acid / Galacturonic acid | 7.02 | C₆H₁₀O₇ | ¹³C₆H₁₀O₇ | 98.7 | 1.2 | 1.1 |

| **Organic acids and other metabolites** | | | | | | |
|---|---|---|---|---|---|---|
| Niacinamide | 1.70 | C₆H₆N₂O | ¹³C₅C₁H₆N₂O | nd | 4.2 | 6.5 |
| Choline | 3.40 | C₅H₁₄NO | ¹³C₅H₁₄NO | nd | 73.7 | 79.4 |
| Pyruvic acid | 5.20 | C₃H₄O₃ | ¹³C₃H₄O₃ | 98.1 | 0.1 | <0.1 |
| Lactic acid | 5.20 | C₃H₆O₃ | ¹³C₃H₆O₃ | 99.0 | 1.0 | 0.5 |
| Sarcosine | 6.34 | C₃H₇NO₂ | ¹³C₃H₇NO₂ | 98.7 | 0.6 | 0.5 |
| Phosphocholine | 7.20 | C₅H₁₅NO₄P | ¹³C₅H₁₅NO₄P | nd | 18.7 | 11.9 |
| Glycerolphosphoetha nolamine | 7.40 | C₅H₁₄NO₆P | ¹³C₅H₁₄NO₆P | 98.8 | 1.6 | 1.1 |

**Table 4. List of lipid classes with the example of particular ¹³C labeled lipids found in cell line cultivated with ¹³C substrates and their ¹²C analogues found in cell line cultivated under normal ¹²C conditions (nd abbreviation means not determined). If the superscript of the nucleon number is not given, then this atom has a natural isotopic abundances.**

| **Lipid species** | **t_{R} [min]** | **Elemental Composition (neutral form)** | | **Experimental ¹³C isotopic purity [%]** | **Relative abundance in samples** | |
|---|---|---|---|---|---|---|
| | | **¹²C cell lines** | **¹³C cell lines** | | **¹³C cell lines** | **¹²C cell lines** |
| **LPC** | | | | | | |
| LPC 14:0 | 1.79 | C₂₂H₄₆O₇N₁P₁ | C₅H₄₆O₇N₁P₁¹³C₁₇ | 98.3 | 2.4 | 2.1 |
| LPC 16:0 A | 2.48 | C₂₄H₅₀O₇N₁P₁ | C₅H₅₀O₇N₁P₁¹³C₁₉ | 98.3 | 18.5 | 11.1 |
| LPC 16:0 B | 2.67 | C₂₄H₅₀O₇N₁P₁ | C₅H₅₀O₇N₁P₁¹³C₁₉ | 98.3 | 9.8 | 11.5 |
| LPC 16:1 A | 1.90 | C₂₄H₄₈O₇N₁P₁ | C₅H₄₈O₇N₁P₁¹³C₁₉ | 98.7 | 9.3 | 10.3 |
| LPC 16:1 B | 1.96 | C₂₄H₄₈O₇N₁P₁ | C₅H₄₈O₇N₁P₁¹³C₁₉ | 98.4 | 10.0 | 9.6 |
| LPC 17:0 | 2.80 | C₂₅H₅₂O₇N₁P₁ | C₅H₅₂O₇N₁P₁¹³C₂₀ | 99.6 | 0.3 | 0.2 |
| LPC 18:2 | 2.00 | C₂₆H₅₀O₇N₁P₁ | C₅H₅₀O₇N₁P₁¹³C₂₁ | 98.6 | 1.1 | 1.6 |
| LPC 18:1 A | 2.60 | C₂₆H₅₂O₇N₁P₁ | C₅H₅₂O₇N₁P₁¹³C₂₁ | 98.6 | 27.2 | 27.5 |
| LPC 18:1 B | 2.80 | C₂₆H₅₂O₇N₁P₁ | C₅H₅₂O₇N₁P₁¹³C₂₁ | 98.4 | 7.3 | 9.9 |
| LPC 18:0 A | 3.50 | C₂₆H₅₄O₇N₁P₁ | C₅H₅₄O₇N₁P₁¹³C₂₁ | 98.9 | 0.7 | 0.4 |
| LPC 18:0 B | 3.85 | C₂₆H₅₄O₇N₁P₁ | C₅H₅₄O₇N₁P₁¹³C₂₁ | 98.6 | 4.2 | 7.6 |
| LPC 20:3 | 2.47 | C₂₈H₅₂O₇N₁P₁ | C₅H₅₂O₇N₁P₁¹³C₂₃ | 98.8 | 6.0 | 4.1 |
| LPC 20:2 | 2.80 | C₂₈H₅₄O₇N₁P₁ | C₅H₅₄O₇N₁P₁¹³C₂₃ | 99.2 | 1.0 | 1.0 |
| LPC 20:1 A | 3.65 | C₂₈H₅₆O₇N₁P₁ | C₅H₅₆O₇N₁P₁¹³C₂₃ | 98.7 | 0.2 | 0.3 |
| LPC 20:1 B | 3.95 | C₂₈H₅₆O₇N₁P₁ | C₅H₅₆O₇N₁P₁¹³C₂₃ | 99.2 | 0.4 | 0.4 |
| LPC 24:1 | 7.60 | C₃₂H₆₄O₇N₁P₁ | C₅H₆₄O₇N₁P₁¹³C₂₇ | 99.9 | 0.3 | 0.2 |
| LPC 24:0 | 9.82 | C₃₂H₆₆O₇N₁P₁ | C₅H₆₆O₇N₁P₁¹³C₂₇ | 99.1 | 0.9 | 1.1 |
| LPC 26:1 | 9.75 | C₃₄H₆₈O₇N₁P₁ | C₅H₆₈O₇N₁P₁¹³C₂₉ | 99.3 | 0.6 | 1.0 |

| **LPC O-** | | | | | | |
|---|---|---|---|---|---|---|
| LPC O-16:0 | 3.15 | C₂₄H₅₂O₆N₁P₁ | C₅H₅₂O₆N₁P₁¹³C₁₉ | 98.0 | 57.2 | 59.6 |
| LPC O-18:1 | 3.28 | C₂₆H₅₄O₆N₁P₁ | C₅H₅₄O₆N₁P₁¹³C₂₁ | 98.9 | 15.2 | 8.8 |
| LPC O-18:0 | 4.55 | C₂₆H₅₆O₆N₁P₁ | C₅H₅₆O₆N₁P₁¹³C₂₁ | 98.5 | 26.3 | 30.1 |
| LPC O-24:1 | 8.57 | C₃₂H₆₆O₆N₁P₁ | C₅H₆₆O₆N₁P₁¹³C₂₇ | 98.7 | 1.4 | 1.5 |

| LPE | | | | | | |
|---|---|---|---|---|---|---|
| LPE 16:1 A | 1.98 | C₂₁H₄₂O₇N₁P₁ | ¹³C₂₁H₄₂O₇N₁P₁ | 98.7 | 6.4 | 6.6 |
| LPE 16:1 B | 2.05 | C₂₁H₄₂O₇N₁P₁ | ¹³C₂₁H₄₂O₇N₁P₁ | 98.5 | 6.6 | 5.3 |
| LPE 16:0 A | 2.60 | C₂₁H₄₄O₇N₁P₁ | ¹³C₂₁H₄₄O₇N₁P₁ | 99.0 | 2.0 | 1.3 |
| LPE 16:0 B | 2.80 | C₂₁H₄₄O₇N₁P₁ | ¹³C₂₁H₄₄O₇N₁P₁ | 99.6 | 1.5 | 2.1 |
| LPE 17:1 | 2.30 | C₂₂H₄₄O₇N₁P₁ | ¹³C₂₂H₄₄O₇N₁P₁ | 99.1 | 0.3 | 0.2 |
| LPE 17:0 | 2.88 | C₂₂H₄₆O₇N₁P₁ | ¹³C₂₂H₄₆O₇N₁P₁ | 99.3 | 0.3 | 0.1 |
| LPE 18:3 | 1.88 | C₂₃H₄₂O₇N₁P₁ | ¹³C₂₃H₄₂O₇N₁P₁ | 98.9 | 1.4 | 1.5 |
| LPE 18:2 | 2.17 | C₂₃H₄₄O₇N₁P₁ | ¹³C₂₃H₄₄O₇N₁P₁ | 98.5 | 1.4 | 2.2 |
| LPE 18:1 A | 2.75 | C₂₃H₄₆O₇N₁P₁ | ¹³C₂₃H₄₆O₇N₁P₁ | 98.7 | 24.4 | 23.7 |
| LPE 18:1 B | 2.95 | C₂₃H₄₆O₇N₁P₁ | ¹³C₂₃H₄₆O₇N₁P₁ | 99.2 | 3.3 | 3.1 |
| LPE 18:0 | 4.05 | C₂₃H₄₈O₇N₁P₁ | ¹³C₂₃H₄₈O₇N₁P₁ | 98.5 | 8.3 | 8.4 |
| LPE 20:4 | 2.15 | C₂₅H₄₄O₇N₁P₁ | ¹³C₂₅H₄₄O₇N₁P₁ | 99.5 | 0.5 | 0.5 |
| LPE 20:3 A | 2.42 | C₂₅H₄₆O₇N₁P₁ | ¹³C₂₅H₄₆O₇N₁P₁ | 99.1 | 1.6 | 1.6 |
| LPE 20:3 B | 2.60 | C₂₅H₄₆O₇N₁P₁ | ¹³C₂₅H₄₆O₇N₁P₁ | 98.6 | 34.8 | 38.5 |
| LPE 20:2 | 3.01 | C₂₅H₄₈O₇N₁P₁ | ¹³C₂₅H₄₈O₇N₁P₁ | 99.0 | 1.0 | 0.7 |
| LPE 20:1 A | 3.89 | C₂₅H₅₀O₇N₁P₁ | ¹³C₂₅H₅₀O₇N₁P₁ | 99.2 | 0.4 | 0.4 |
| LPE 20:1 B | 4.05 | C₂₅H₅₀O₇N₁P₁ | ¹³C₂₅H₅₀O₇N₁P₁ | 99.7 | 0.4 | 0.2 |
| LPE 20:0 | 5.75 | C₂₅H₅₂O₇N₁P₁ | ¹³C₂₅H₅₂O₇N₁P₁ | 99.2 | 0.1 | 0.2 |
| LPE 22:4 A | 2.60 | C₂₇H₄₈O₇N₁P₁ | ¹³C₂₇H₄₈O₇N₁P₁ | 99.4 | 0.4 | 0.3 |
| LPE 22:4 B | 3.05 | C₂₇H₄₈O₇N₁P₁ | ¹³C₂₇H₄₈O₇N₁P₁ | 98.5 | 3.5 | 2.1 |
| LPE 22:1 | 5.65 | C₂₇H₅₄O₇N₁P₁ | ¹³C₂₇H₅₄O₇N₁P₁ | 99.0 | 0.2 | 0.2 |
| LPE 24:0 | 10.19 | C₂₉H₆₀O₇N₁P₁ | ¹³ C₂₉H₆₀O₇N₁P₁ | 99.3 | 0.9 | 0.9 |

| **LPE O-** | | | | | | |
|---|---|---|---|---|---|---|
| LPE O-16:0 | 3.35 | C₂₁H₄₄O₆N₁P₁ | ¹³C₂₁H₄₄O₆N₁P₁ | 99.6 | 7.5 | 9.8 |
| LPE O-16:1 | 3.21 | C₂₁H₄₄O₆N₁P₁ | ¹³C₂₁H₄₄O₆N₁P₁ | 99.0 | 30.9 | 19.5 |
| LPE O-18:1 | 4.65 | C₂₃H₄₈O₆N₁P₁ | ¹³C₂₃H₄₈O₆N₁P₁ | 98.9 | 35.5 | 29.6 |
| LPE O-18:0 | 4.84 | C₂₃H₅₀O₆N₁P₁ | ¹³C₂₃H₅₀O₆N₁P₁ | 98.8 | 26.1 | 41.1 |

| **LPS** | | | | | | |
|---|---|---|---|---|---|---|
| LPS 18:1 | 2.18 | C₂₄H₄₆O₉P₁N₁ | ¹³C₂₄H₄₆O₉P₁N₁ | 99.8 | 21.1 | 21.2 |
| LPS 18:0 | 3.15 | C₂₄H₄₈O₉P₁N₁ | ¹³C₂₄H₄₈O₉P₁N₁ | 99.0 | 34.0 | 32.4 |

| **LPS O-** | | | | | | |
|---|---|---|---|---|---|---|
| LPS O-16:1 | 2.80 | C₂₂H₄₄O₈P₁N₁ | ¹³C₂₂H₄₄O₈P₁N₁ | 98.5 | 41.0 | 43.9 |
| LPS O-18:0 | 3.70 | C₂₄H₅₀O₈P₁N₁ | ¹³C₂₄H₅₀O₈P₁N₁ | 99.1 | 3.9 | 2.4 |

| **LPG** | | | | | | |
|---|---|---|---|---|---|---|
| LPG 18:1 A | 2.30 | C₂₄H₄₇O₉P₁ | ¹³C₂₄H₄₇O₉P₁ | 98.6 | 60.5 | 70.4 |
| LPG 18:1 B | 2.45 | C₂₄H₄₇O₉P₁ | ¹³C₂₄H₄₇O₉P₁ | 98.3 | 16.1 | 11.8 |
| LPG 18:0 A | 3.09 | C₂₄H₄₉O₉P₁ | ¹³C₂₄H₄₉O₉P₁ | 99.3 | 14.3 | 8.0 |
| LPG 18:0 B | 3.33 | C₂₄H₄₉O₉P₁ | ¹³C₂₄H₄₉O₉P₁ | 99.0 | 9.1 | 9.8 |

| **LPI** | | | | | | |
|---|---|---|---|---|---|---|
| LPI 16:1 | 1.59 | C₂₅H₄₇O₁₂P₁ | C₆H₄₇O₁₂N₀P₁¹³C₁₉ | 99.5 | 1.6 | 1.4 |
| LPI 16:0 | 2.17 | C₂₅H₄₉O₁₂P₁ | C₆H₄₉O₁₂N₀P₁¹³C₁₉ | 98.5 | 0.8 | 1.1 |
| LPI 18:2 | 1.80 | C₂₇H₄₉O₁₂P₁ | C₆H₄₉O₁₂N₀P₁¹³C₂₁ | 99.4 | 1.7 | 1.9 |
| LPI 18:1 A | 2.15 | C₂₇H₅₁O₁₂P₁ | C₆H₅₁O₁₂N₀P₁¹³C₂₁ | 98.2 | 20.1 | 18.1 |
| LPI 18:1 B | 2.30 | C₂₇H₅₁O₁₂P₁ | C₆H₅₁O₁₂N₀P₁¹³C₂₁ | 99.5 | 8.8 | 9.2 |
| LPI 18:0 | 3.10 | C₂₇H₅₁O₁₂P₁ | C₆H₅₃O₁₂N₀P₁¹³C₂₁ | 98.5 | 38.2 | 33.7 |
| LPI 20:3 | 2.05 | C₂₉H₅₁O₁₂P₁ | C₆H₅₁O₁₂N₀P₁¹³C₂₃ | 98.3 | 25.2 | 28.4 |
| LPI 20:2 | 2.32 | C₂₉H₅₃O₁₂P₁ | C₆H₅₃O₁₂N₀P₁¹³C₂₃ | 99.7 | 3.6 | 6.2 |

| PC | | | | | | |
|---|---|---|---|---|---|---|
| PC 28:0 | 10.63 | C₃₆H₇₂O₈N₁P₁ | C₅H₇₂O₈N₁P₁¹³C₃₁ | 98.6 | 0.3 | 0.3 |
| PC 29:0 | 11.17 | C₃₇H₇₄O₈N₁P₁ | C₅H₇₄O₈N₁P₁¹³C₃₂ | 98.6 | 0.1 | <0.1 |
| PC 30:1 | 11.07 | C₃₈H₇₄O₈N₁P₁ | C₅H₇₄O₈N₁P₁¹³C₃₃ | 98.5 | 1.0 | 1.1 |
| PC 30:0 | 12.33 | C₃₈H₇₆O₈N₁P₁ | C₅H₇₆O₈N₁P₁¹³C₃₃ | 98.3 | 2.9 | 2.6 |
| PC 31:1 | 11.90 | C₃₉H₇₆O₈N₁P₁ | C₅H₇₆O₈N₁P₁¹³C₃₄ | 98.5 | 0.2 | 0.1 |
| PC 32:2 A | 10.95 | C₄₀H₇₆O₈N₁P₁ | C₅H₇₆O₈N₁P₁¹³C₃₅ | 98.4 | 0.5 | 0.4 |
| PC 32:2 B | 11.23 | C₄₀H₇₆O₈N₁P₁ | C₅H₇₆O₈N₁P₁¹³C₃₅ | 98.3 | 1.5 | 1.4 |
| PC 32:2 C | 11.47 | C₄₀H₇₆O₈N₁P₁ | C₅H₇₆O₈N₁P₁¹³C₃₅ | 98.4 | 1.3 | 1.1 |
| PC 32:1 A | 12.45 | C₄₀H₇₈O₈N₁P₁ | C₅H₇₈O₈N₁P₁¹³C₃₅ | 98.3 | 6.0 | 6.2 |
| PC 32:1 B | 12.68 | C₄₀H₇₈O₈N₁P₁ | C₅H₇₈O₈N₁P₁¹³C₃₅ | 98.3 | 9.4 | 10.3 |
| PC 32:1 C | 12.93 | C₄₀H₇₈O₈N₁P₁ | C₅H₇₈O₈N₁P₁¹³C₃₅ | 98.3 | 1.1 | 0.4 |
| PC 32:0 | 13.70 | C₄₀H₈₀O₈N₁P₁ | C₅H₈₀O₈N₁P₁¹³C₃₅ | 98.4 | 5.1 | 3.0 |
| PC 33:2 A | 11.76 | C₄₁H₇₈O₈N₁P₁ | C₅H₇₈O₈N₁P₁¹³C₃₆ | 98.5 | 0.1 | <0.1 |
| PC 33:2 B | 12.02 | C₄₁H₇₈O₈N₁P₁ | C₅H₇₈O₈N₁P₁¹³C₃₆ | 98.4 | 0.1 | 0.1 |
| PC 33:2 C | 12.27 | C₄₁H₇₈O₈N₁P₁ | C₅H₇₈O₈N₁P₁¹³C₃₆ | 98.2 | 0.1 | 0.1 |
| PC 33:1 A | 13.12 | C₄₁H₈₀O₈N₁P₁ | C₅H₈₀O₈N₁P₁¹³C₃₆ | 98.5 | 0.7 | 0.6 |
| PC 33:1 B | 13.33 | C₄₁H₈₀O₈N₁P₁ | C₅H₈₀O₈N₁P₁¹³C₃₆ | 99.1 | 0.3 | 0.3 |
| PC 34:3 A | 11.20 | C₄₂H₇₈O₈N₁P₁ | C₅H₇₈O₈N₁P₁¹³C₃₇ | 98.5 | 0.1 | 0.1 |
| PC 34:3 B | 11.35 | C₄₂H₇₈O₈N₁P₁ | C₅H₇₈O₈N₁P₁¹³C₃₇ | 98.5 | 0.3 | 0.3 |
| PC 34:3 C | 11.60 | C₄₂H₇₈O₈N₁P₁ | C₅H₇₈O₈N₁P₁¹³C₃₇ | 98.4 | 0.6 | 0.5 |
| PC 34:3 D | 11.82 | C₄₂H₇₈O₈N₁P₁ | C₅H₇₈O₈N₁P₁¹³C₃₇ | 98.5 | 0.3 | 0.2 |
| PC 34:3 E | 12.05 | C₄₂H₇₈O₈N₁P₁ | C₅H₇₈O₈N₁P₁¹³C₃₇ | 98.5 | 0.4 | 0.3 |
| PC 34:2 A | 12.52 | C₄₂H₈₀O₈N₁P₁ | C₅H₈₀O₈N₁P₁¹³C₃₇ | 98.3 | 4.8 | 5.3 |
| PC 34:2 B | 12.75 | C₄₂H₈₀O₈N₁P₁ | C₅H₈₀O₈N₁P₁¹³C₃₇ | 98.3 | 6.7 | 6.6 |
| PC 34:2 C | 12.96 | C₄₂H₈₀O₈N₁P₁ | C₅H₈₀O₈N₁P₁¹³C₃₇ | 98.4 | 1.7 | 1.0 |
| PC 34:1 A | 13.75 | C₄₂H₈₂O₈N₁P₁ | C₅H₈₂O₈N₁P₁¹³C₃₇ | 98.3 | 14.6 | 15.9 |
| PC 34:1 B | 13.95 | C₄₂H₈₂O₈N₁P₁ | C₅H₈₂O₈N₁P₁¹³C₃₇ | 98.4 | 2.9 | 2.5 |
| PC 34:0 | 14.81 | C₄₂H₈₄O₈N₁P₁ | C₅H₈₄O₈N₁P₁¹³C₃₇ | 98.4 | 1.7 | 1.3 |
| PC 35:2 | 13.18 | C₄₃H₈₂O₈N₁P₁ | C₅H₈₂O₈N₁P₁¹³C₃₈ | 98.1 | 0.3 | 0.3 |
| PC 35:1 A | 14.06 | C₄₃H₈₄O₈N₁P₁ | C₅H₈₄O₈N₁P₁¹³C₃₈ | 98.2 | 0.4 | 0.3 |
| PC 35:1 B | 14.32 | C₄₃H₈₄O₈N₁P₁ | C₅H₈₄O₈N₁P₁¹³C₃₈ | 98.2 | 0.3 | 0.3 |
| PC 35:0 | 15.28 | C₄₃H₈₆O₈N₁P₁ | C₅H₈₆O₈N₁P₁¹³C₃₈ | 98.3 | <0.1 | <0.1 |
| PC 36:4 A | 11.60 | C₄₄H₈₀O₈N₁P₁ | C₅H₈₀O₈N₁P₁¹³C₃₉ | 98.4 | <0.1 | <0.1 |
| PC 36:4 B | 11.75 | C₄₄H₈₀O₈N₁P₁ | C₅H₈₀O₈N₁P₁¹³C₃₉ | 98.4 | 0.1 | 0.1 |
| PC 36:4 C | 12.12 | C₄₄H₈₀O₈N₁P₁ | C₅H₈₀O₈N₁P₁¹³C₃₉ | 98.5 | 0.2 | 0.2 |
| PC 36:4 D | 12.35 | C₄₄H₈₀O₈N₁P₁ | C₅H₈₀O₈N₁P₁¹³C₃₉ | 98.4 | 0.3 | 0.2 |
| PC 36:3 A | 12.70 | C₄₄H₈₂O₈N₁P₁ | C₅H₈₂O₈N₁P₁¹³C₃₉ | 98.3 | 0.9 | 0.9 |
| PC 36:3 B | 12.82 | C₄₄H₈₂O₈N₁P₁ | C₅H₈₂O₈N₁P₁¹³C₃₉ | 98.3 | 1.7 | 1.7 |
| PC 36:3 C | 13.00 | C₄₄H₈₂O₈N₁P₁ | C₅H₈₂O₈N₁P₁¹³C₃₉ | 98.3 | 0.9 | 0.9 |
| PC 36:3 D | 13.17 | C₄₄H₈₂O₈N₁P₁ | C₅H₈₂O₈N₁P₁¹³C₃₉ | 98.4 | 0.7 | 0.7 |
| PC 36:3 E | 13.43 | C₄₄H₈₂O₈N₁P₁ | C₅H₈₂O₈N₁P₁¹³C₃₉ | 98.4 | 1.3 | 1.1 |
| PC 36:2 A | 13.80 | C₄₄H₈₄O₈N₁P₁ | C₅H₈₄O₈N₁P₁¹³C₃₉ | 98.3 | 11.7 | 12.4 |
| PC 36:2 B | 14.00 | C₄₄H₈₄O₈N₁P₁ | C₅H₈₄O₈N₁P₁¹³C₃₉ | 98.3 | 2.7 | 2.8 |
| PC 36:1 | 14.85 | C₄₄H₈₆O₈N₁P₁ | C₅H₈₆O₈N₁P₁¹³C₃₉ | 98.4 | 5.5 | 7.3 |
| PC 36:0 | 15.76 | C₄₄H₈₈O₈N₁P₁ | C₅H₈₈O₈N₁P₁¹³C₃₉ | 98.6 | 0.1 | 0.1 |
| PC 37:1 | 15.10 | C₄₅H₈₈O₈N₁P₁ | C₅H₈₈O₈N₁P₁¹³C₄₀ | 98.3 | 0.1 | <0.1 |
| PC 38:5 A | 12.15 | C₄₆H₈₂O₈N₁P₁ | C₅H₈₂O₈N₁P₁¹³C₄₁ | 98.4 | <0.1 | <0.1 |
| PC 38:5 B | 12.45 | C₄₆H₈₂O₈N₁P₁ | C₅H₈₂O₈N₁P₁¹³C₄₁ | 98.3 | 0.1 | 0.1 |
| PC 38:5 C | 12.65 | C₄₆H₈₂O₈N₁P₁ | C₅H₈₂O₈N₁P₁¹³C₄₁ | 98.3 | <0.1 | <0.1 |
| PC 38:5 D | 12.84 | C₄₆H₈₂O₈N₁P₁ | C₅H₈₂O₈N₁P₁¹³C₄₁ | 98.5 | 0.1 | <0.1 |
| PC 38:4 A | 12.97 | C₄₆H₈₄O₈N₁P₁ | C₅H₈₄O₈N₁P₁¹³C₄₁ | 98.2 | 0.1 | 0.1 |
| PC 38:4 B | 13.16 | C₄₆H₈₄O₈N₁P₁ | C₅H₈₄O₈N₁P₁¹³C₄₁ | 98.4 | 0.3 | 0.3 |
| PC 38:4 C | 13.45 | C₄₆H₈₄O₈N₁P₁ | C₅H₈₄O₈N₁P₁¹³C₄₁ | 98.3 | 1.4 | 1.3 |
| PC 38:4 D | 13.65 | C₄₆H₈₄O₈N₁P₁ | C₅H₈₄O₈N₁P₁¹³C₄₁ | 98.4 | 0.3 | 0.2 |
| PC 38:3 A | 13.98 | C₄₆H₈₆O₈N₁P₁ | C₅H₈₆O₈N₁P₁¹³C₄₁ | 98.4 | 0.7 | 0.8 |
| PC 38:3 B | 14.25 | C₄₆H₈₆O₈N₁P₁ | C₅H₈₆O₈N₁P₁¹³C₄₁ | 98.5 | 0.7 | 0.8 |
| PC 38:3 C | 14.58 | C₄₆H₈₆O₈N₁P₁ | C₅H₈₆O₈N₁P₁¹³C₄₁ | 98.4 | 0.5 | 0.4 |
| PC 38:3 D | 14.80 | C₄₆H₈₈O₈N₁P₁ | C₅H₈₈O₈N₁P₁¹³C₄₁ | 98.4 | 0.6 | 0.6 |
| PC 38:2 A | 14.94 | C₄₆H₈₈O₈N₁P₁ | C₅H₈₈O₈N₁P₁¹³C₄₁ | 98.4 | 0.8 | 1.0 |
| PC 38:2 B | 15.77 | C₄₆H₉₀O₈N₁P₁ | C₅H₉₀O₈N₁P₁¹³C₄₁ | 98.2 | 0.6 | 0.7 |
| PC 38:1 | 16.56 | C₄₆H₉₂O₈N₁P₁ | C₅H₉₂O₈N₁P₁¹³C₄₁ | 98.3 | 0.1 | 0.2 |
| PC 38:0 | 13.10 | C₄₈H₈₄O₈N₁P₁ | C₅H₈₄O₈N₁P₁¹³C₄₃ | 98.5 | <0.1 | <0.1 |
| PC 40:6 | 13.45 | C₄₈H₈₆O₈N₁P₁ | C₅H₈₆O₈N₁P₁¹³C₄₃ | 98.4 | 0.1 | 0.1 |
| PC 40:5 A | 13.65 | C₄₈H₈₆O₈N₁P₁ | C₅H₈₆O₈N₁P₁¹³C₄₃ | 98.3 | <0.1 | <0.1 |
| PC 40:5 B | 13.90 | C₄₈H₈₆O₈N₁P₁ | C₅H₈₆O₈NP¹³C₄₃ | 98.3 | <0.1 | 0.1 |
| PC 40:5 C | 14.30 | C₄₈H₈₈O₈N₁P₁ | C₅H₈₈O₈N₁P₁¹³C₄₃ | 98.5 | 0.1 | <0.1 |
| PC 40:4 A | 14.54 | C₄₈H₈₈O₈N₁P₁ | C₅H₈₈O₈N₁P₁¹³C₄₃ | 98.5 | 0.4 | 0.4 |
| PC 40:4 B | 14.98 | C₄₈H₉₀O₈N₁P₁ | C₅H₉₀O₈N₁P₁¹³C₄₃ | 98.7 | 0.1 | 0.1 |
| PC 40:3 C | 15.28 | C₄₈H₉₀O₈N₁P₁ | C₅H₉₀O₈N₁P₁¹³C₄₃ | 98.7 | 0.1 | 0.1 |
| PC 40:3 D | 15.75 | C₄₈H₉₂O₈N₁P₁ | C₅H₉₂O₈N₁P₁¹³C₄₃ | 98.5 | 0.1 | 0.1 |
| PC 40:2 | 16.50 | C₄₈H₉₄O₈N₁P₁ | C₅H₉₄O₈N₁P₁¹³C₄₃ | 98.4 | 0.1 | 0.1 |
| PC 40:1 | 14.55 | C₅₀H₉₀O₈N₁P₁ | C₅H₉₀O₈N₁P₁¹³C₄₅ | 98.4 | 0.1 | 0.1 |
| PC 42:5 | 15.25 | C₅₀H₉₂O₈N₁P₁ | C₅H₉₂O₈N₁P₁¹³C₄₅ | 98.6 | <0.1 | <0.1 |
| PC 42:4 A | 15.25 | C₅₀H₉₂O₈N₁P₁ | C₅H₉₂O₈N₁P₁¹³C₄₅ | 98.7 | <0.1 | <0.1 |
| PC 42:4 B | 15.80 | C₅₀H₉₄O₈N₁P₁ | C₅H₉₄O₈N₁P₁¹³C₄₅ | 98.6 | 0.1 | <0.1 |
| PC 42:3 | 16.48 | C₅₀H₉₆O₈N₁P₁ | C₅H₉₆O₈N₁P₁¹³C₄₅ | 98.4 | 0.1 | 0.1 |
| PC 42:2 | 17.27 | C₅₀H₉₈O₈N₁P₁ | C₅H₉₈O₈N₁P₁¹³C₄₅ | 98.3 | 0.2 | 0.2 |
| PC 42:1 | 17.90 | C₅₀H₁₀₀O₈N₁P₁ | C₅H₁₀₀O₈N₁P₁¹³C₄₅ | 98.2 | 0.1 | 0.2 |
| PC 42:0 | 17.19 | C₅₂H₁₀₀O₈N₁P₁ | C₅H₁₀₀O₈N₁P₁¹³C₄₇ | 98.4 | <0.1 | <0.1 |
| PC 44:2 | 17.90 | C₅₂H₁₀₂O₈N₁P₁ | C₅H₁₀₂O₈N₁P₁¹³C₄₇ | 98.4 | 0.1 | 0.1 |

| **PC O-** | | | | | | |
|---|---|---|---|---|---|---|
| PC O-30:1 A | 11.80 | C₃₈H₇₆O₇N₁P₁ | C₅H₇₆O₇N₁P₁¹³C₃₃ | 98.5 | 0.1 | 0.1 |
| PC O-30:1 B | 12.07 | C₃₈H₇₆O₇N₁P₁ | C₅H₇₆O₇N₁P₁¹³C₃₃ | 98.3 | 0.2 | 0.2 |
| PC O-30:1 C | 12.96 | C₃₈H₇₆O₇N₁P₁ | C₅H₇₆O₇N₁P₁¹³C₃₃ | 98.3 | 0.3 | 0.3 |
| PC O-30:0 | 13.18 | C₃₈H₇₈O₇N₁P₁ | C₅H₇₈O₇N₁P₁¹³C₃₃ | 98.4 | 3.4 | 2.8 |
| PC O-32:2 | 12.15 | C₄₀H₇₈O₇N₁P₁ | C₅H₇₈O₇N₁P₁¹³C₃₅ | 98.3 | 0.4 | 0.4 |
| PC O-32:1 A | 13.27 | C₄₀H₈₀O₇N₁P₁ | C₅H₈₀O₇N₁P₁¹³C₃₅ | 98.3 | 7.5 | 8.2 |
| PC O-32:1 B | 13.49 | C₄₀H₈₀O₇N₁P₁ | C₅H₈₀O₇N₁P₁¹³C₃₅ | 98.3 | 16.7 | 17.1 |
| PC O-32:0 | 14.42 | C₄₀H₈₂O₇N₁P₁ | C₅H₈₂O₇N₁P₁¹³C₃₅ | 98.4 | 14.0 | 10.5 |
| PC O-33:0 A | 14.72 | C₄₁H₈₄O₇N₁P₁ | C₅H₈₄O₇N₁P₁¹³C₃₆ | 98.4 | 0.5 | 0.5 |
| PC O-33:0 B | 14.95 | C₄₁H₈₄O₇N₁P₁ | C₅H₈₄O₇N₁P₁¹³C₃₆ | 98.3 | 0.3 | 0.3 |
| PC O-34:3 | 12.30 | C₄₂H₈₀O₇N₁P₁ | C₅H₈₀O₇N₁P₁¹³C₃₇ | 97.8 | 0.2 | 0.1 |
| PC O-34:2 A | 13.30 | C₄₂H₈₂O₇N₁P₁ | C₅H₈₂O₇N₁P₁¹³C₃₇ | 98.4 | 2.7 | 1.9 |
| PC O-34:2 B | 13.48 | C₄₂H₈₂O₇N₁P₁ | C₅H₈₂O₇N₁P₁¹³C₃₇ | 98.3 | 1.8 | 2.7 |
| PC O-34:1 A | 14.45 | C₄₂H₈₄O₇N₁P₁ | C₅H₈₄O₇N₁P₁¹³C₃₇ | 98.3 | 22.3 | 23.7 |
| PC O-34:1 B | 14.65 | C₄₂H₈₄O₇N₁P₁ | C₅H₈₄O₇N₁P₁¹³C₃₇ | 98.3 | 4.8 | 4.4 |
| PC O-34:0 | 15.43 | C₄₂H₈₆O₇N₁P₁ | C₅H₈₆O₇N₁P₁¹³C₃₇ | 98.4 | 7.0 | 5.5 |
| PC O-35:1 A | 14.70 | C₄₃H₈₆O₇N₁P₁ | C₅H₈₆O₇N₁P₁¹³C₃₈ | 98.2 | 0.1 | 0.1 |
| PC O-35:1 B | 15.00 | C₄₃H₈₆O₇N₁P₁ | C₅H₈₆O₇N₁P₁¹³C₃₈ | 99.5 | 0.3 | 0.3 |
| PC O-35:0 | 15.67 | C₄₃H₈₈O₇N₁P₁ | C₅H₈₈O₇N₁P₁¹³C₃₈ | 98.5 | 0.1 | 0.2 |
| PC O-36:2 | 14.45 | C₄₄H₈₆O₇N₁P₁ | C₅H₈₆O₇N₁P₁¹³C₃₉ | 98.4 | 3.7 | 4.2 |
| PC O-36:1 | 15.47 | C₄₄H₈₈O₇N₁P₁ | C₅H₈₈O₇N₁P₁¹³C₃₉ | 98.4 | 6.3 | 7.8 |
| PC O-36:0 | 16.30 | C₄₄H₉₀O₇N₁P₁ | C₅H₉₀O₇N₁P₁¹³C₃₉ | 98.7 | 0.2 | 0.2 |
| PC O-38:4 A | 14.15 | C₄₆H₈₆O₇N₁P₁ | C₅H₈₆O₇N₁P₁¹³C₄₁ | 98.5 | 1.2 | 1.5 |
| PC O-38:4 B | 14.60 | C₄₆H₈₆O₇N₁P₁ | C₅H₈₆O₇N₁P₁¹³C₄₁ | 98.6 | 0.5 | 0.2 |
| PC O-38:3 A | 14.94 | C₄₆H₈₈O₇N₁P₁ | C₅H₈₈O₇N₁P₁¹³C₄₁ | 98.4 | 1.0 | 1.4 |
| PC O-38:3 A | 15.20 | C₄₆H₈₈O₇N₁P₁ | C₅H₈₈O₇N₁P₁¹³C₄₁ | 98.3 | 2.0 | 2.5 |
| PC O-38:2 | 15.55 | C₄₆H₉₀O₇N₁P₁ | C₅H₉₀O₇N₁P₁¹³C₄₁ | 98.5 | 0.4 | 0.6 |
| PC O-38:1 | 16.30 | C₄₆H₉₂O₇N₁P₁ | C₅H₉₂O₇N₁P₁¹³C₄₁ | 98.7 | 0.2 | 0.3 |
| PC O-38:0 | 17.10 | C₄₆H₉₄O₇N₁P₁ | C₅H₉₄O₇N₁P₁¹³C₄₁ | 98.2 | 0.1 | 0.1 |
| PC O-40:5 | 14.68 | C₄₈H₈₈O₇N₁P₁ | C₅H₈₈O₇N₁P₁¹³C₄₃ | 98.5 | 0.2 | 0.4 |
| PC O-40:4 A | 14.92 | C₄₈H₉₀O₇N₁P₁ | C₅H₉₀O₇N₁P₁¹³C₄₃ | 98.4 | 0.2 | 0.3 |
| PC O-40:4 B | 15.20 | C₄₈H₉₀O₇N₁P₁ | C₅H₉₀O₇N₁P₁¹³C₄₃ | 98.3 | 0.1 | 0.1 |
| PC O-40:4 C | 15.54 | C₄₈H₉₀O₇N₁P₁ | C₅H₉₀O₇N₁P₁¹³C₄₃ | 97.9 | 0.2 | 0.1 |
| PC O-40:4 D | 15.70 | C₄₈H₉₀O₇N₁P₁ | C₅H₉₀O₇N₁P₁¹³C₄₃ | 98.7 | 0.1 | 0.1 |
| PC O-40:3 | 15.85 | C₄₈H₉₂O₇N₁P₁ | C₅H₉₂O₇N₁P₁¹³C₄₃ | 98.5 | 0.3 | 0.3 |
| PC O-40:2 | 16.25 | C₄₈H₉₄O₇N₁P₁ | C₅H₉₄O₇N₁P₁¹³C₄₃ | 98.7 | 0.1 | 0.2 |
| PC O-40:1 | 17.00 | C₄₈H₉₆O₇N₁P₁ | C₅H₉₆O₇N₁P₁¹³C₄₃ | 98.2 | 0.1 | 0.2 |
| PC O-42:2 | 16.95 | C₅₀H₉₈O₇N₁P₁ | C₅H₉₈O₇N₁P₁¹³C₄₅ | 98.5 | 0.2 | 0.3 |

| PE | | | | | | |
|---|---|---|---|---|---|---|
| PE 30:1 A | 11.25 | C₃₅H₆₈O₈N₁P₁ | ¹³C₃₅H₆₈O₈N₁P₁ | 98.8 | <0.1 | <0.1 |
| PE 30:1 B | 11.55 | C₃₅H₆₈O₈N₁P₁ | ¹³C₃₅H₆₈O₈N₁P₁ | 98.8 | <0.1 | <0.1 |
| PE 30:0 | 12.70 | C₃₅H₇₀O₈N₁P₁ | ¹³C₃₅H₇₀O₈N₁P₁ | 98.7 | 0.1 | 0.1 |
| PE 32:2 A | 11.35 | C₃₇H₇₀O₈N₁P₁ | ¹³C₃₇H₇₀O₈N₁P₁ | 98.4 | 0.1 | 0.1 |
| PE 32:2 B | 11.64 | C₃₇H₇₀O₈N₁P₁ | ¹³C₃₇H₇₀O₈N₁P₁ | 98.5 | 0.3 | 0.2 |
| PE 32:2 C | 11.87 | C₃₇H₇₀O₈N₁P₁ | ¹³C₃₇H₇₀O₈N₁P₁ | 98.5 | 0.2 | 0.2 |
| PE 32:1 A | 12.80 | C₃₇H₇₂O₈N₁P₁ | ¹³C₃₇H₇₂O₈N₁P₁ | 98.5 | 1.5 | 1.6 |
| PE 32:1 B | 13.05 | C₃₇H₇₂O₈N₁P₁ | ¹³C₃₇H₇₂O₈N₁P₁ | 98.4 | 2.2 | 2.6 |
| PE 32:0 | 14.04 | C₃₇H₇₄O₈N₁P₁ | ¹³C₃₇H₇₄O₈N₁P₁ | 98.5 | 0.4 | 0.3 |
| PE 33:1 | 13.50 | C₃₈H₇₄O₈N₁P₁ | ¹³C₃₈H₇₄O₈N₁P₁ | 98.3 | 0.1 | 0.1 |
| PE 33:0 | 14.35 | C₃₈H₇₆O₈N₁P₁ | ¹³C₃₈H₇₆O₈N₁P₁ | 98.7 | 0.1 | 0.1 |
| PE 34:3 A | 11.82 | C₃₉H₇₂O₈N₁P₁ | ¹³C₃₉H₇₂O₈N₁P₁ | 98.6 | 0.2 | 0.1 |
| PE 34:3 B | 12.00 | C₃₉H₇₂O₈N₁P₁ | ¹³C₃₉H₇₂O₈N₁P₁ | 98.5 | 0.2 | 0.2 |
| PE 34:3 C | 12.20 | C₃₉H₇₂O₈N₁P₁ | ¹³C₃₉H₇₂O₈N₁P₁ | 98.5 | 0.1 | 0.1 |
| PE 34:2 A | 12.88 | C₃₉H₇₄O₈N₁P₁ | ¹³C₃₉H₇₄O₈N₁P₁ | 98.5 | 2.8 | 2.7 |
| PE 34:2 B | 13.10 | C₃₉H₇₄O₈N₁P₁ | ¹³C₃₉H₇₄O₈N₁P₁ | 98.5 | 4.1 | 3.6 |
| PE 34:2 C | 13.32 | C₃₉H₇₄O₈N₁P₁ | ¹³C₃₉H₇₄O₈N₁P₁ | 98.4 | 0.8 | 0.7 |
| PE 34:1 A | 14.08 | C₃₉H₇₆O₈N₁P₁ | ¹³C₃₉H₇₆O₈N₁P₁ | 98.4 | 11.8 | 9.9 |
| PE 34:1 B | 14.27 | C₃₉H₇₆O₈N₁P₁ | ¹³C₃₉H₇₆O₈N₁P₁ | 98.5 | 3.9 | 3.1 |
| PE 34:0 | 15.10 | C₃₉H₇₈O₈N₁P₁ | ¹³C₃₉H₇₈O₈N₁P₁ | 98.4 | 0.5 | 0.4 |
| PE 35:1 A | 14.62 | C₄₀H₇₈O₈N₁P₁ | ¹³C₄₀H₇₈O₈N₁P₁ | 98.1 | 0.3 | 0.2 |
| PE 35:1 B | 14.82 | C₄₀H₇₈O₈N₁P₁ | ¹³C₄₀H₇₈O₈N₁P₁ | 97.9 | 0.1 | <0.1 |
| PE 35:0 | 15.35 | C₄₀H₈₀O₈N₁P₁ | ¹³C₄₀H₈₀O₈N₁P₁ | 98.6 | <0.1 | <0.1 |
| PE 36:3 A | 13.17 | C₄₁H₇₆O₈N₁P₁ | ¹³C₄₁H₇₆O₈N₁P₁ | 98.4 | 1.1 | 1.2 |
| PE 36:3 B | 13.38 | C₄₁H₇₆O₈N₁P₁ | ¹³C₄₁H₇₆O₈N₁P₁ | 98.5 | 0.8 | 0.7 |
| PE 36:3 C | 13.58 | C₄₁H₇₆O₈N₁P₁ | ¹³C₄₁H₇₆O₈N₁P₁ | 98.4 | 0.5 | 0.3 |
| PE 36:3 D | 13.78 | C₄₁H₇₆O₈N₁P₁ | ¹³C₄₁H₇₆O₈N₁P₁ | 98.5 | 2.6 | 2.3 |
| PE 36:2 A | 14.12 | C₄₁H₇₈O₈N₁P₁ | ¹³C₄₁H₇₈O₈N₁P₁ | 98.4 | 15.6 | 16.2 |
| PE 36:2 B | 14.30 | C₄₁H₇₈O₈N₁P₁ | ¹³C₄₁H₇₈O₈N₁P₁ | 98.5 | 4.1 | 4.4 |
| PE 36:2 C | 14.49 | C₄₁H₇₈O₈N₁P₁ | ¹³C₄₁H₇₈O₈N₁P₁ | 98.5 | 0.7 | 0.8 |
| PE 36:2 D | 14.65 | C₄₁H₇₈O₈N₁P₁ | ¹³C₄₁H₇₈O₈N₁P₁ | 98.3 | 0.5 | 0.7 |
| PE 36:1 A | 15.13 | C₄₁H₈₀O₈N₁P₁ | ¹³C₄₁H₈₀O₈N₁P₁ | 98.4 | 19.7 | 17.9 |
| PE 36:1 B | 15.32 | C₄₁H₈₀O₈N₁P₁ | ¹³C₄₁H₈₀O₈N₁P₁ | 98.6 | 0.8 | 0.9 |
| PE 36:0 | 16.02 | C₄₁H₈₂O₈N₁P₁ | ¹³C₄₁H₈₂O₈N₁P₁ | 99.2 | <0.1 | <0.1 |
| PE 38:4 A | 13.50 | C₄₃H₇₈O₈N₁P₁ | ¹³C₄₃H₇₈O₈N₁P₁ | 98.5 | 0.4 | 0.4 |
| PE 38:4 B | 13.80 | C₄₃H₇₈O₈N₁P₁ | ¹³C₄₃H₇₈O₈N₁P₁ | 98.5 | 3.8 | 4.1 |
| PE 38:4 C | 14.23 | C₄₃H₇₈O₈N₁P₁ | ¹³C₄₃H₇₈O₈N₁P₁ | 98.6 | 0.3 | 0.2 |
| PE 38:3 A | 14.32 | C₄₃H₈₀O₈N₁P₁ | ¹³C₄₃H₈₀O₈N₁P₁ | 98.5 | 0.7 | 0.8 |
| PE 38:3 B | 14.59 | C₄₃H₈₀O₈N₁P₁ | ¹³C₄₃H₈₀O₈N₁P₁ | 98.4 | 1.5 | 1.5 |
| PE 38:3 C | 14.86 | C₄₃H₈₀O₈N₁P₁ | ¹³C₄₃H₈₀O₈N₁P₁ | 98.4 | 12.0 | 15.6 |
| PE 38:2 A | 15.08 | C₄₃H₈₂O₈N₁P₁ | ¹³C₄₃H₈₂O₈N₁P₁ | 98.4 | 0.5 | 0.6 |
| PE 38:2 B | 15.20 | C₄₃H₈₂O₈N₁P₁ | ¹³C₄₃H₈₂O₈N₁P₁ | 98.4 | 0.9 | 1.1 |
| PE 38:2 C | 15.32 | C₄₃H₈₂O₈N₁P₁ | ¹³C₄₃H₈₂O₈N₁P₁ | 98.4 | 0.4 | 0.5 |
| PE 38:1 | 16.01 | C₄₃H₈₄O₈N₁P₁ | ¹³C₄₃H₈₄O₈N₁P₁ | 98.4 | 0.2 | 0.2 |
| PE 40:4 A | 14.60 | C₄₅H₈₂O₈N₁P₁ | ¹³C₄₅H₈₂O₈N₁P₁ | 98.6 | 0.5 | 0.6 |
| PE 40:4 B | 14.95 | C₄₅H₈₂O₈N₁P₁ | ¹³C₄₅H₈₂O₈N₁P₁ | 98.5 | 0.4 | 0.4 |
| PE 40:4 C | 15.24 | C₄₅H₈₂O₈N₁P₁ | ¹³C₄₅H₈₂O₈N₁P₁ | 98.6 | 0.4 | 0.2 |
| PE 40:3 | 15.54 | C₄₅H₈₄O₈N₁P₁ | ¹³C₄₅H₈₄O₈N₁P₁ | 98.5 | 1.2 | 1.3 |
| PE 40:2 | 16.00 | C₄₅H₈₆O₈N₁P₁ | ¹³C₄₅H₈₆O₈N₁P₁ | 98.7 | 0.1 | 0.1 |
| PE 40:1 A | 16.80 | C₄₅H₈₈O₈N₁P₁ | ¹³C₄₅H₈₈O₈N₁P₁ | 98.5 | 0.1 | 0.1 |
| PE 40:1 B | 16.95 | C₄₅H₈₈O₈N₁P₁ | ¹³C₄₅H₈₈O₈N₁P₁ | 98.4 | 0.1 | 0.1 |
| PE 42:2 | 16.70 | C₄₇H₉₀O₈N₁P₁ | ¹³C₄₇H₉₀O₈N₁P₁ | 98.4 | 0.2 | 0.1 |
| PE 42:1 | 17.49 | C₄₇H₉₂O₈N₁P₁ | ¹³C₄₇H₉₂O₈N₁P₁ | 98.6 | 0.2 | 0.2 |

| **PE O-** | | | | | | |
|---|---|---|---|---|---|---|
| PE O-32:2 A | 13.46 | C₃₇H₇₂O₇N₁P₁ | ¹³C₃₇H₇₂O₇N₁P₁ | 98.5 | 1.8 | 1.9 |
| PE O-32:2 B | 13.67 | C₃₇H₇₂O₇N₁P₁ | ¹³C₃₇H₇₂O₇N₁P₁ | 98.5 | 2.1 | 2.0 |
| PE O-32:1 A | 13.86 | C₃₇H₇₄O₇N₁P₁ | ¹³C₃₇H₇₄O₇N₁P₁ | 98.4 | 0.1 | 0.1 |
| PE O-32:1 B | 14.64 | C₃₇H₇₄O₇N₁P₁ | ¹³C₃₇H₇₄O₇N₁P₁ | 98.7 | 0.4 | 0.3 |
| PE O-32:0 | 14.75 | C₃₇H₇₆O₇N₁P₁ | ¹³C₃₇H₇₆O₇N₁P₁ | 98.5 | 0.3 | 0.4 |
| PE O-33:1 A | 14.25 | C₃₈H₇₆O₇N₁P₁ | ¹³C₃₈H₇₆O₇N₁P₁ | 99.0 | <0.1 | <0.1 |
| PE O-33:1 B | 14.89 | C₃₈H₇₆O₇N₁P₁ | ¹³C₃₈H₇₆O₇N₁P₁ | 98.9 | 0.1 | 0.1 |
| PE O-33:0 A | 15.05 | C₃₈H₇₈O₇N₁P₁ | ¹³C₃₈H₇₈O₇N₁P₁ | 99.0 | <0.1 | <0.1 |
| PE O-33:0 B | 15.25 | C₃₈H₇₈O₇N₁P₁ | ¹³C₃₈H₇₈O₇N₁P₁ | 99.0 | <0.1 | <0.1 |
| PE O-34:3 A | 13.50 | C₃₉H₇₄O₇N₁P₁ | ¹³C₃₉H₇₄O₇N₁P₁ | 98.4 | 0.6 | 0.5 |
| PE O-34:3 B | 13.70 | C₃₉H₇₄O₇N₁P₁ | ¹³C₃₉H₇₄O₇N₁P₁ | 98.6 | 0.9 | 1.2 |
| PE O-34:3 C | 13.90 | C₃₉H₇₄O₇N₁P₁ | ¹³C₃₉H₇₄O₇N₁P₁ | 98.4 | 0.3 | 0.3 |
| PE O-34:3 D | 14.08 | C₃₉H₇₄O₇N₁P₁ | ¹³C₃₉H₇₄O₇N₁P₁ | 98.4 | 0.2 | 0.3 |
| PE O-34:2 A | 14.63 | C₃₉H₇₆O₇N₁P₁ | ¹³C₃₉H₇₆O₇N₁P₁ | 98.5 | 9.9 | 10.4 |
| PE O-34:2 B | 14.80 | C₃₉H₇₆O₇N₁P₁ | ¹³C₃₉H₇₆O₇N₁P₁ | 98.5 | 3.0 | 2.0 |
| PE O-34:1 A | 14.75 | C₃₉H₇₈O₇N₁P₁ | ¹³C₃₉H₇₈O₇N₁P₁ | 98.5 | 1.0 | 1.4 |
| PE O-34:1 B | 14.95 | C₃₉H₇₈O₇N₁P₁ | ¹³C₃₉H₇₈O₇N₁P₁ | 98.2 | 0.2 | 0.2 |
| PE O-34:1 C | 15.60 | C₃₉H₇₈O₇N₁P₁ | ¹³C₃₉H₇₈O₇N₁P₁ | 98.6 | 0.8 | 0.8 |
| PE O-34:0 | 15.75 | C₃₉H₈₀O₇N₁P₁ | ¹³C₃₉H₈₀O₇N₁P₁ | 98.5 | 0.8 | 0.9 |
| PE O-36:5 | 13.60 | C₄₁H₇₄O₇N₁P₁ | ¹³C₄₁H₇₄O₇N₁P₁ | 98.3 | 0.2 | 0.2 |
| PE O-36:4 A | 14.05 | C₄₁H₇₆O₇N₁P₁ | ¹³C₄₁H₇₆O₇N₁P₁ | 98.3 | 0.7 | 0.7 |
| PE O-36:4 B | 14.32 | C₄₁H₇₆O₇N₁P₁ | ¹³C₄₁H₇₆O₇N₁P₁ | 98.4 | 15.0 | 14.2 |
| PE O-36:3 A | 14.65 | C₄₁H₇₈O₇N₁P₁ | ¹³C₄₁H₇₈O₇N₁P₁ | 98.5 | 2.7 | 2.6 |
| PE O-36:3 B | 14.85 | C₄₁H₇₈O₇N₁P₁ | ¹³C₄₁H₇₈O₇N₁P₁ | 98.5 | 1.2 | 1.3 |
| PE O-36:3 C | 14.97 | C₄₁H₇₈O₇N₁P₁ | ¹³C₄₁H₇₈O₇N₁P₁ | 98.5 | 0.4 | 0.4 |
| PE O-36:2 | 15.62 | C₄₁H₈₀O₇N₁P₁ | ¹³C₄₁H₈₀O₇N₁P₁ | 98.4 | 8.3 | 6.8 |
| PE O-36:1 | 15.75 | C₄₁H₈₂O₇N₁P₁ | ¹³C₄₁H₈₂O₇N₁P₁ | 98.5 | 0.6 | 1.2 |
| PE O-36:0 | 16.59 | C₄₁H₈₄O₇N₁P₁ | ¹³C₄₁H₈₄O₇N₁P₁ | 98.8 | <0.1 | <0.1 |
| PE O-38:5 A | 14.35 | C₄₃H₇₈O₇N₁P₁ | ¹³ C₄₃H₇₈O₇N₁P₁ | 98.6 | 3.8 | 4.3 |
| PE O-38:5 B | 14.70 | C₄₃H₇₈O₇N₁P₁ | ¹³ C₄₃H₇₈O₇N₁P₁ | 98.5 | 3.1 | 2.0 |
| PE O-38:4 A | 15.05 | C₄₃H₈₀O₇N₁P₁ | ¹³ C₄₃H₈₀O₇N₁P₁ | 98.4 | 13.1 | 11.4 |
| PE O-38:4 B | 15.35 | C₄₃H₈₀O₇N₁P₁ | ¹³ C₄₃H₈₀O₇N₁P₁ | 98.4 | 17.1 | 20.9 |
| PE O-38:3 A | 15.24 | C₄₃H₈₂O₇N₁P₁ | ¹³C₄₃H₈₂O₇N₁P₁ | 98.6 | 0.6 | 0.8 |
| PE O-38:3 B | 15.50 | C₄₃H₈₂O₇N₁P₁ | ¹³C₄₃H₈₂O₇N₁P₁ | 98.5 | 0.6 | 1.6 |
| PE O-38:3 C | 15.70 | C₄₃H₈₂O₇N₁P₁ | ¹³C₄₃H₈₂O₇N₁P₁ | 98.4 | 0.7 | 0.8 |
| PE O-38:2 | 16.45 | C₄₃H₈₄O₇N₁P₁ | ¹³C₄₃H₈₄O₇N₁P₁ | 98.5 | 0.3 | 0.4 |
| PE O-40:5 | 15.67 | C₄₅H₈₂O₇N₁P₁ | ¹³ C₄₅H₈₂O₇N₁P₁ | 98.5 | 1.4 | 0.7 |
| PE O-40:4 A | 15.98 | C₄₅H₈₄O₇N₁P₁ | ¹³C₄₅H₈₄O₇N₁P₁ | 98.5 | 6.2 | 5.1 |
| PE O-40:4 B | 16.12 | C₄₅H₈₄O₇N₁P₁ | ¹³C₄₅H₈₄O₇N₁P₁ | 98.8 | 0.3 | 0.2 |
| PE O-40:3 A | 16.13 | C₄₅H₈₆O₇N₁P₁ | ¹³C₄₅H₈₆O₇N₁P₁ | 98.7 | 0.3 | 0.4 |
| PE O-40:3 B | 16.53 | C₄₅H₈₆O₇N₁P₁ | ¹³C₄₅H₈₆O₇N₁P₁ | 98.6 | 0.3 | 0.4 |
| PE O-40:2 | 17.20 | C₄₅H₈₈O₇N₁P₁ | ¹³C₄₅H₈₈O₇N₁P₁ | 98.2 | 0.1 | 0.1 |
| PE O-42:4 | 16.62 | C₄₇H₈₈O₇N₁P₁ | ¹³C₄₇H₈₈O₇N₁P₁ | 98.3 | <0.1 | 0.1 |
| PE O-42:3 | 17.10 | C₄₇H₉₀O₇N₁P₁ | ¹³C₄₇H₉₀O₇N₁P₁ | 98.8 | 0.1 | 0.2 |

| **PI** | | | | | | |
|---|---|---|---|---|---|---|
| PI 32:2 A | 9.43 | C₄₁H₇₅O₁₃P₁ | C₆H₇₅O₁₃P₁¹³C₃₅ | 98.6 | 0.1 | <0.1 |
| PI 32:2 B | 9.73 | C₄₁H₇₅O₁₃P₁ | C₆H₇₅O₁₃P₁¹³C₃₅ | 98.5 | <0.1 | <0.1 |
| PI 32:1 A | 11.15 | C₄₁H₇₇O₁₃P₁ | C₆H₇₇O₁₃P₁¹³C₃₅ | 98.3 | 0.3 | 0.2 |
| PI 32:1 B | 11.35 | C₄₁H₇₇O₁₃P₁ | C₆H₇₇O₁₃P₁¹³C₃₅ | 98.6 | 0.1 | 0.1 |
| PI 34:3 A | 9.72 | C₄₃H₇₇O₁₃P₁ | C₆H₇₇O₁₃P₁¹³C₃₇ | 98.4 | 0.1 | <0.1 |
| PI 34:3 B | 9.88 | C₄₃H₇₇O₁₃P₁ | C₆H₇₇O₁₃P₁¹³C₃₇ | 98.3 | 0.1 | <0.1 |
| PI 34:3 C | 10.00 | C₄₃H₇₇O₁₃P₁ | C₆H₇₇O₁₃P₁¹³C₃₇ | 98.2 | 0.1 | <0.1 |
| PI 34:3 D | 10.15 | C₄₃H₇₇O₁₃P₁ | C₆H₇₇O₁₃P₁¹³C₃₇ | 98.8 | <0.1 | <0.1 |
| PI 34:3 E | 10.40 | C₄₃H₇₇O₁₃P₁ | C₆H₇₇O₁₃P₁¹³C₃₇ | 98.8 | <0.1 | <0.1 |
| PI 34:3 F | 10.75 | C₄₃H₇₇O₁₃P₁ | C₆H₇₇O₁₃P₁¹³C₃₇ | 98.8 | <0.1 | <0.1 |
| PI 34:2 A | 11.20 | C₄₃H₇₉O₁₃P₁ | C₆H₇₉O₁₃P₁¹³C₃₇ | 98.5 | 1.5 | 1.1 |
| PI 34:2 B | 11.50 | C₄₃H₇₉O₁₃P₁ | C₆H₇₉O₁₃P₁¹³C₃₇ | 98.6 | 0.3 | 0.2 |
| PI 34:2 C | 11.74 | C₄₃H₇₉O₁₃P₁ | C₆H₇₉O₁₃P₁¹³C₃₇ | 98.3 | 0.1 | 0.1 |
| PI 34:2 D | 11.95 | C₄₃H₇₉O₁₃P₁ | C₆H₇₉O₁₃P₁¹³C₃₇ | 98.1 | 0.1 | 0.1 |
| PI 34:1 A | 12.59 | C₄₃H₈₁O₁₃P₁ | C₆H₈₁O₁₃P₁¹³C₃₇ | 98.5 | 2.3 | 2.3 |
| PI 34:1 B | 12.82 | C₄₃H₈₁O₁₃P₁ | C₆H₈₁O₁₃P₁¹³C₃₇ | 98.4 | 0.3 | 0.4 |
| PI 35:2 A | 11.90 | C₄₄H₈₁O₁₃P₁ | C₆H₈₁O₁₃P₁¹³C₃₈ | 98.3 | 0.1 | <0.1 |
| PI 35:2 B | 12.20 | C₄₄H₈₁O₁₃P₁ | C₆H₈₁O₁₃P₁¹³C₃₈ | 98.3 | <0.1 | <0.1 |
| PI 36:4 | 10.85 | C₄₅H₇₉O₁₃P₁ | C₆H₇₉O₁₃P₁¹³C₃₉ | 98.4 | 0.2 | 0.2 |
| PI 36:3 A | 11.43 | C₄₅H₈₁O₁₃P₁ | C₆H₈₁O₁₃P₁¹³C₃₉ | 98.4 | 0.6 | 0.4 |
| PI 36:3 B | 11.60 | C₄₅H₈₁O₁₃P₁ | C₆H₈₁O₁₃P₁¹³C₃₉ | 98.3 | 0.7 | 0.5 |
| PI 36:3 C | 12.03 | C₄₅H₈₁O₁₃P₁ | C₆H₈₁O₁₃P₁¹³C₃₉ | 98.5 | 0.6 | 0.5 |
| PI 36:3 D | 12.30 | C₄₅H₈₁O₁₃P₁ | C₆H₈₁O₁₃P₁¹³C₃₉ | 98.5 | 1.7 | 1.6 |
| PI 36:2 A | 12.65 | C₄₅H₈₃O₁₃P₁ | C₆H₈₃O₁₃P₁¹³C₃₉ | 98.3 | 8.5 | 7.3 |
| PI 36:2 B | 12.90 | C₄₅H₈₃O₁₃P₁ | C₆H₈₃O₁₃P₁¹³C₃₉ | 98.4 | 2.0 | 2.5 |
| PI 36:2 C | 13.08 | C₄₅H₈₃O₁₃P₁ | C₆H₈₃O₁₃P₁¹³C₃₉ | 98.3 | 0.8 | 0.6 |
| PI 36:2 D | 13.28 | C₄₅H₈₃O₁₃P₁ | C₆H₈₃O₁₃P₁¹³C₃₉ | 98.3 | 2.5 | 2.6 |
| PI 36:1 A | 13.80 | C₄₅H₈₅O₁₃P₁ | C₆H₈₅O₁₃P₁¹³C₃₉ | 98.4 | 10.6 | 11.7 |
| PI 36:1 B | 14.08 | C₄₅H₈₅O₁₃P₁ | C₆H₈₅O₁₃P₁¹³C₃₉ | 98.3 | 0.6 | 0.8 |
| PI 37:4 | 11.65 | C₄₆H₈₁O₁₃P₁ | C₆H₈₁O₁₃P₁¹³C₄₀ | 98.2 | 0.1 | <0.1 |
| PI 38:5 A | 11.10 | C₄₇H₈₁O₁₃P₁ | C₆H₈₁O₁₃P₁¹³C₄₁ | 98.5 | 0.5 | 0.3 |
| PI 38:5 B | 11.25 | C₄₇H₈₁O₁₃P₁ | C₆H₈₁O₁₃P₁¹³C₄₁ | 98.6 | 0.2 | 0.1 |
| PI 38:5 C | 11.64 | C₄₇H₈₁O₁₃P₁ | C₆H₈₁O₁₃P₁¹³C₄₁ | 98.7 | 0.2 | 0.1 |
| PI 38:4 A | 11.63 | C₄₇H₈₃O₁₃P₁ | C₆H₈₃O₁₃P₁¹³C₄₁ | 98.8 | 0.1 | <0.1 |
| PI 38:4 B | 11.80 | C₄₇H₈₃O₁₃P₁ | C₆H₈₃O₁₃P₁¹³C₄₁ | 98.2 | 0.1 | 0.1 |
| PI 38:4 C | 12.00 | C₄₇H₈₃O₁₃P₁ | C₆H₈₃O₁₃P₁¹³C₄₁ | 98.5 | 0.3 | 0.2 |
| PI 38:4 D | 12.35 | C₄₇H₈₃O₁₃P₁ | C₆H₈₃O₁₃P₁¹³C₄₁ | 98.3 | 5.9 | 4.6 |
| PI 38:4 E | 12.52 | C₄₇H₈₃O₁₃P₁ | C₆H₈₃O₁₃P₁¹³C₄₁ | 98.5 | 1.5 | 1.3 |
| PI 38:3 A | 12.82 | C₄₇H₈₅O₁₃P₁ | C₆H₈₅O₁₃P₁¹³C₄₁ | 98.4 | 0.9 | 1.0 |
| PI 38:3 B | 12.93 | C₄₇H₈₅O₁₃P₁ | C₆H₈₅O₁₃P₁¹³C₄₁ | 98.4 | 0.9 | 1.0 |
| PI 38:3 C | 13.25 | C₄₇H₈₅O₁₃P₁ | C₆H₈₅O₁₃P₁¹³C₄₁ | 98.4 | 2.4 | 2.4 |
| PI 38:3 D | 13.57 | C₄₇H₈₅O₁₃P₁ | C₆H₈₅O₁₃P₁¹³C₄₁ | 98.4 | 36.8 | 35.5 |
| PI 38:2 A | 13.92 | C₄₇H₈₇O₁₃P₁ | C₆H₈₇O₁₃P₁¹³C₄₁ | 98.4 | 4.1 | 5.6 |
| PI 38:2 B | 14.04 | C₄₇H₈₇O₁₃P₁ | C₆H₈₇O₁₃P₁¹³C₄₁ | 98.4 | 2.4 | 3.6 |
| PI 38:2 C | 14.14 | C₄₇H₈₇O₁₃P₁ | C₆H₈₇O₁₃P₁¹³C₄₁ | 98.4 | 1.1 | 1.4 |
| PI 38:2 D | 14.37 | C₄₇H₈₇O₁₃P₁ | C₆H₈₇O₁₃P₁¹³C₄₁ | 98.4 | 0.5 | 0.4 |
| PI 38:1 A | 14.78 | C₄₇H₈₉O₁₃P₁ | C₆H₈₉O₁₃P₁¹³C₄₁ | 98.3 | 0.1 | 0.1 |
| PI 38:1 B | 14.90 | C₄₇H₈₉O₁₃P₁ | C₆H₈₉O₁₃P₁¹³C₄₁ | 98.3 | 0.1 | 0.1 |
| PI 39:4 | 12.65 | C₄₈H₈₅O₁₃P₁ | C₆H₈₅O₁₃P₁¹³C₄₂ | 98.4 | 0.1 | <0.1 |
| PI 39:3 | 13.80 | C₄₈H₈₇O₁₃P₁ | C₆H₈₇O₁₃P₁¹³C₄₂ | 98.2 | 0.3 | 0.4 |
| PI 40:5 A | 12.18 | C₄₉H₈₅O₁₃P₁ | C₆H₈₅O₁₃P₁¹³C₄₃ | 98.5 | 0.1 | 0.1 |
| PI 40:5 B | 12.49 | C₄₉H₈₅O₁₃P₁ | C₆H₈₅O₁₃P₁¹³C₄₃ | 98.4 | 0.1 | 0.1 |
| PI 40:5 C | 12.64 | C₄₉H₈₅O₁₃P₁ | C₆H₈₅O₁₃P₁¹³C₄₃ | 98.2 | 0.2 | 0.2 |
| PI 40:5 D | 12.93 | C₄₉H₈₅O₁₃P₁ | C₆H₈₅O₁₃P₁¹³C₄₃ | 98.4 | 0.1 | <0.1 |
| PI 40:4 A | 13.26 | C₄₉H₈₇O₁₃P₁ | C₆H₈₇O₁₃P₁¹³C₄₃ | 98.4 | 0.9 | 1.0 |
| PI 40:4 B | 13.60 | C₄₉H₈₇O₁₃P₁ | C₆H₈₇O₁₃P₁¹³C₄₃ | 98.4 | 0.4 | 0.5 |
| PI 40:4 C | 13.96 | C₄₉H₈₇O₁₃P₁ | C₆H₈₇O₁₃P₁¹³C₄₃ | 98.2 | 0.2 | 0.1 |
| PI 40:3 A | 14.30 | C₄₉H₈₉O₁₃P₁ | C₆H₈₉O₁₃P₁¹³C₄₃ | 98.4 | 4.6 | 4.9 |
| PI 40:3 B | 14.57 | C₄₉H₈₉O₁₃P₁ | C₆H₈₉O₁₃P₁¹³C₄₃ | 98.3 | 0.2 | 0.2 |
| PI 40:2 | 14.90 | C₄₉H₉₁O₁₃P₁ | C₆H₉₁O₁₃P₁¹³C₄₃ | 98.3 | 0.2 | 0.3 |
| P142:5 | 13.60 | C₅₁H₈₉O₁₃P₁ | C₆H₈₉O₁₃P₁¹³C₄₅ | 98.7 | <0.1 | <0.1 |
| PI 42:4 A | 14.35 | C₅₁H₉₁O₁₃P₁ | C₆H₉₁O₁₃P₁¹³C₄₅ | 98.5 | <0.1 | <0.1 |
| PI 42:4 B | 14.60 | C₅₁H₉₁O₁₃P₁ | C₆H₉₁O₁₃P₁¹³C₄₅ | 98.5 | 0.1 | 0.1 |
| PI 42:3 | 15.05 | C₅₁H₉₃O₁₃P₁ | C₆H₉₃O₁₃P₁¹³C₄₅ | 98.3 | 0.1 | 0.1 |

| **PG** | | | | | | |
|---|---|---|---|---|---|---|
| PG 30:1 A | 9.65 | C₃₆H₆₉O₁₀P₁ | ¹³C₃₆H₆₉O₁₀P₁ | 99.7 | 0.1 | 0.1 |
| PG 30:1 B | 9.95 | C₃₆H₆₉O₁₀P₁ | ¹³C₃₆H₆₉O₁₀P₁ | 98.6 | 0.2 | 0.1 |
| PG 30:0 | 11.33 | C₃₆H₇₁O₁₀P₁ | ¹³C₃₆H₇₁O₁₀P₁ | 99.3 | 0.2 | 0.1 |
| PG 32:2 A | 9.86 | C₃₈H₇₁O₁₀P₁ | ¹³C₃₈H₇₁O₁₀P₁ | 98.2 | 0.5 | 0.4 |
| PG 32:2 B | 10.16 | C₃₈H₇₁O₁₀P₁ | ¹³C₃₈H₇₁O₁₀P₁ | 98.3 | 0.3 | 0.2 |
| PG 32:2 C | 10.43 | C₃₈H₇₁O₁₀P₁ | ¹³C₃₈H₇₁O₁₀P₁ | 99.0 | 0.3 | 0.1 |
| PG 32:1 A | 11.36 | C₃₈H₇₃O₁₀P₁ | ¹³C₃₈H₇₃O₁₀P₁ | 98.6 | 0.9 | 0.8 |
| PG 32:1 B | 11.45 | C₃₈H₇₃O₁₀P₁ | ¹³C₃₈H₇₃O₁₀P₁ | 98.6 | 0.7 | 0.7 |
| PG 32:1 C | 11.7 | C₃₈H₇₃O₁₀P₁ | ¹³C₃₈H_{?3}O₁₀P₁ | 98.4 | 0.9 | 0.6 |
| PG 32:0 | 12.78 | C₃₈H₇₅O₁₀P₁ | ¹³C₃₈H₇₅O₁₀P₁ | 98.8 | 0.9 | 0.4 |
| PG 34:2 A | 10.43 | C₄₀H₇₅O₁₀P₁ | ¹³C₄₀H₇₅O₁₀P₁ | 98.5 | 0.7 | 0.7 |
| PG 34:2 B | 10.69 | C₄₀H₇₅O₁₀P₁ | ¹³C₄₀H₇₅O₁₀P₁ | 98.8 | 0.9 | 0.8 |
| PG 34:2 C | 11.51 | C₄₀H₇₅O₁₀P₁ | ¹³C₄₀H₇₅O₁₀P₁ | 98.5 | 2.7 | 2.6 |
| PG 34:2 D | 11.75 | C₄₀H₇₅O₁₀P₁ | ¹³C₄₀H₇₅O₁₀P₁ | 98.3 | 2.9 | 2.3 |
| PG 34:1 | 12.8 | C₄₀H₇₇O₁₀P₁ | ¹³C₄₀H₇₇O₁₀P₁ | 98.6 | 39.1 | 41.6 |
| PG 34:0 | 13.98 | C₄₀H₇₉O₁₀P₁ | ¹³C₄₀H₇₉O₁₀P₁ | 98.6 | 1.2 | 1.1 |
| PG 36:2 | 12.9 | C₄₂H₇₉O₁₀P₁ | ¹³C₄₂H₇₉O₁₀P₁ | 98.5 | 5.4 | 5.4 |
| PG 36:1 | 13.98 | C₄₂H₈₁O₁₀P₁ | ¹³C₄₂H₈₁O₁₀P₁ | 98.5 | 42.1 | 41.9 |

| **PS** | | | | | | |
|---|---|---|---|---|---|---|
| PS 32:1 A | 11.30 | C₃₈H₇₂O₁₀P₁N₁ | ¹³C₃₈H₇₂O₁₀P₁N₁ | 98.7 | 0.6 | 0.6 |
| PS 32:1 B | 11.60 | C₃₈H₇₂O₁₀P₁N₁ | ¹³C₃₈H₇₂O₁₀P₁N₁ | 98.5 | 0.8 | 1.1 |
| PS 34:2 A | 11.40 | C₄₀H₇₄O₁₀P₁N₁ | ¹³C₄₀H₇₄O₁₀P₁N₁ | 98.4 | 0.6 | 0.6 |
| PS 34:2 B | 11.68 | C₄₀H₇₄O₁₀P₁N₁ | ¹³C₄₀H₇₄O₁₀P₁N₁ | 98.3 | 1.4 | 1.6 |
| PS 34:2 C | 11.95 | C₄₀H₇₄O₁₀P₁N₁ | ¹³C₄₀H₇₄O₁₀P₁N₁ | 98.2 | 0.5 | 0.4 |
| PS 34:1 A | 12.78 | C₄₀H₇₆O₁₀P₁N₁ | ¹³C₄₀H₇₆O₁₀P₁N₁ | 98.5 | 13.2 | 14.4 |
| PS 34:1 B | 13.00 | C₄₀H₇₆O₁₀P₁N₁ | ¹³C₄₀H₇₆O₁₀P₁N₁ | 98.5 | 11.6 | 10.9 |
| PS 36:3 A | 11.80 | C₄₂H₇₆O₁₀P₁N₁ | ¹³C₄₂H₇₆O₁₀P₁N₁ | 98.6 | 0.4 | 0.4 |
| PS 36:3 B | 12.05 | C₄₂H₇₆O₁₀P₁N₁ | ¹³C₄₂H₇₆O₁₀P₁N₁ | 98.0 | 0.2 | 0.3 |
| PS 36:2 A | 12.85 | C₄₂H₇₈O₁₀P₁N₁ | ¹³C₄₂H₇₈O₁₀P₁N₁ | 98.5 | 7.8 | 9.5 |
| PS 36:2 B | 13.07 | C₄₂H₇₈O₁₀P₁N₁ | ¹³C₄₂H₇₈O₁₀P₁N₁ | 98.3 | 3.9 | 6.2 |
| PS 36:2 C | 13.30 | C₄₂H₇₈O₁₀P₁N₁ | ¹³C₄₂H₇₈O₁₀P₁N₁ | 98.1 | 1.3 | 1.6 |
| PS 36:1 A | 13.98 | C₄₂H₈₀O₁₀P₁N₁ | ¹³C₄₂H₈₀O₁₀P₁N₁ | 98.4 | 44.8 | 39.1 |
| PS 36:1 B | 14.18 | C₄₂H₈₀O₁₀P₁N₁ | ¹³C₄₂H₈₀O₁₀P₁N₁ | 98.5 | 4.7 | 4.7 |
| PS 38:3 | 13.40 | C₄₄H₈₀O₁₀P₁N₁ | ¹³C₄₄H₈₀O₁₀P₁N₁ | 98.3 | 1.8 | 2.1 |
| PS 38:2 A | 14.08 | C₄₄H₈₂O₁₀P₁N₁ | ¹³C₄₄H₈₂O₁₀P₁N₁ | 98.2 | 1.1 | 0.7 |
| PS 38:2 B | 14.22 | C₄₄H₈₂O₁₀P₁N₁ | ¹³C₄₄H₈₂O₁₀P₁N₁ | 98.3 | 1.2 | 1.1 |
| PS 38:1 | 15.04 | C₄₄H₈₄O₁₀P₁N₁ | ¹³C₄₄H₈₄O₁₀P₁N₁ | 98.6 | 0.7 | 0.9 |
| PS 40:3 | 14.49 | C₄₆H₈₄O₁₀P₁N₁ | ¹³C₄₆H₈₄O₁₀P₁N₁ | 98.5 | 2.8 | 3.0 |
| PS 40:2 | 15.00 | C₄₆H₈₆O₁₀P₁N₁ | ¹³C₄₆H₈₆O₁₀P₁N₁ | 98.5 | 0.4 | 0.4 |
| PS 40:2 | 15.15 | C₄₆H₈₆O₁₀P₁N₁ | ¹³C₄₆H₈₆O₁₀P₁N₁ | 98.1 | 0.3 | 0.3 |

| **PA** | | | | | | |
|---|---|---|---|---|---|---|
| PA 30:1 A | 10.35 | C₃₃H₆₃O₈P₁ | ¹³C₃₃H₆₃O₈P₁ | 99.7 | 0.6 | 0.8 |
| PA 30:1 B | 10.64 | C₃₃H₆₃O₈P₁ | ¹³C₃₃H₆₃O₈P₁ | 99.9 | 0.7 | 0.6 |
| PA 30:0 | 11.92 | C₃₃H₆₅O₈P₁ | ¹³C₃₃H₆₅O₈P₁ | 99.9 | 1.4 | 2.7 |
| PA 32:2 A | 10.53 | C₃₅H₆₅O₈P₁ | ¹³C₃₅H₆₅O₈P₁ | 99.8 | 0.5 | 0.9 |
| PA 32:2 B | 10.79 | C₃₅H₆₅O₈P₁ | ¹³C₃₅H₆₅O₈P₁ | 99.2 | 1.3 | 1.0 |
| PA 32:2 C | 11.05 | C₃₅H₆₅O₈P₁ | ¹³C₃₅H₆₅O₈P₁ | 99.1 | 0.9 | 0.8 |
| PA 32:1 A | 12.03 | C₃₅H₆₇O₈P₁ | ¹³C₃₅H₆₇O₈P₁ | 98.6 | 3.4 | 5.5 |
| PA 32:1 B | 12.28 | C₃₅H₆₇O₈P₁ | ¹³C₃₅H₆₇O₈P₁ | 98.8 | 3.7 | 4.8 |
| PA 32:0 | 13.35 | C₃₅H₆₉O₈P₁ | ¹³C₃₅H₆₉O₈P₁ | 98.8 | 4.2 | 7.6 |
| PA 34:2 A | 12.14 | C₃₇H₆₉O₈P₁ | ¹³C₃₇H₆₉O₈P₁ | 98.5 | 4.3 | 3.5 |
| PA 34:2 B | 12.35 | C₃₇H₆₉O₈P₁ | ¹³C₃₇H₆₉O₈P₁ | 98.2 | 5.9 | 4.2 |
| PA 34:1 A | 13.12 | C₃₇H₇₁O₈P₁ | ¹³C₃₇H₇₁O₈P₁ | 98.4 | 3.5 | 1.4 |
| PA 34:1 B | 13.38 | C₃₇H₇₁O₈P₁ | ¹³C₃₇H₇₁O₈P₁ | 99.4 | 16.9 | 17.6 |
| PA 34:1 C | 13.6 | C₃₇H₇₁O₈P₁ | ¹³C₃₇H₇₁O₈P₁ | 98.2 | 6.4 | 6.8 |
| PA 34:0 | 14.5 | C₃₇H₇₃O₈P₁ | ¹³C₃₇H₇₃O₈P₁ | 98.6 | 4.6 | 7.2 |
| PA 36:2 | 13.45 | C₃₉H₇₃O₈P₁ | ¹³C₃₉H₇₃O₈P₁ | nd | 20.2 | 13.6 |
| PA 36:1 | 14.55 | C₃₉H₇₅O₈P₁ | ¹³C₃₉H₇₅O₈P₁ | nd | 19.2 | 19.6 |
| PA 36:0 | 15.5 | C₃₉H₇₇O₈P₁ | ¹³C₃₉H₇₇O₈P₁ | nd | 2.3 | 1.3 |

| **CL** | | | | | | |
|---|---|---|---|---|---|---|
| CL 64:3 | 18.22 | C₇₃H₁₃₄O₁₇P₂ | ¹³C₇₃H₁₃₄O₁₇P₂ | 98.2 | 2.6 | 3.3 |
| CL 64:4 | 17.8 | C₇₃H₁₃₂O₁₇P₂ | ¹³C₇₃H₁₃₂O₁₇P₂ | 99.0 | 2.7 | 2.7 |
| CL 66:3 | 18.7 | C₇₅H₁₄₀O₁₇P₂ | ¹³C₇₅H₁₄₀O₁₇P₂ | 99.2 | 3.6 | 4.7 |
| CL 66:4 | 18.24 | C₇₅H₁₃₈O₁₇P₂ | ¹³C₇₅H₁₃₈O₁₇P₂ | 98.5 | 12.2 | 11.9 |
| CL 68:3 | 19.1 | C₇₇H₁₄₄O₁₇P₂ | ¹³C₇₇H₁₄₄O₁₇P₂ | 98.5 | 5.0 | 4.1 |
| CL 68:4 | 18.68 | C₇₇H₁₄₂O₁₇P₂ | ¹³C₇₇H₁₄₂O₁₇P₂ | 98.8 | 25.3 | 30.3 |
| CL 68:5 | 18.32 | C₇₇H₁₄₀O₁₇P₂ | ¹³C₇₇H₁₄₀O₁₇P₂ | 98.8 | 8.8 | 4.4 |
| CL 70:3 | 19.5 | C₇₉H₁₄₈O₁₇P₂ | ¹³C₇₉H₁₄₈O₁₇P₂ | 98.9 | 3.6 | 3.1 |
| CL 70:4 | 19.08 | C₇₉H₁₄₆O₁₇P₂ | ¹³C₇₉H₁₄₆O₁₇P₂ | 97.8 | 20.9 | 21.0 |
| CL 70:5 | 18.76 | C₇₉H₁₄₄O₁₇P₂ | ¹³C₇₉H₁₄₄O₁₇P₂ | 98.3 | 4.6 | 5.9 |
| CL 72:4 | 19.45 | C₈₁H₁₅₀O₁₇P₂ | ¹³C₈₁H₁₅₀O₁₇P₂ | 98.3 | 6.7 | 5.7 |
| CL 72:5 | 19.15 | C₈₁H₁₄₈O₁₇P₂ | ¹³C₈₁H₁₄₈O₁₇P₂ | 99.2 | 4.1 | 2.7 |

| **SM** | | | | | | |
|---|---|---|---|---|---|---|
| SM 30:1;O2 | 8.3 | C₃₅H₇₁O₆N₂P₁ | C₅H₇₁O₆N₂P₁¹³C₃₀ | 99.6 | <0.1 | <0.1 |
| SM 32:2;O2 | 9.14 | C₃₇H₇₃O₆N₂P₁ | C₅H₇₃O₆N₂P₁¹³C₃₂ | 98.5 | 0.1 | <0.1 |
| SM 32:1;O2 | 10.55 | C₃₇H₇₅O₆N₂P₁ | C₅H₇₅O₆N₂P₁¹³C₃₂ | 98.3 | 1.6 | 1.2 |
| SM 32:0;O2 | 11.27 | C₃₇H₇₇O₆N₂P₁ | C₅H₇₇O₆N₂P₁¹³C₃₂ | 98.3 | 0.2 | <0.1 |
| SM 33:1;O2 | 11.52 | C₃₈H₇₇O₆N₂P₁ | C₅H₇₇O₆N₂P₁¹³C₃₃ | 98.1 | 0.1 | <0.1 |
| SM 34:2;O2 A | 10.88 | C₃₉H₇₇O₆N₂P₁ | C₅H₇₇O₆N₂P₁¹³C₃₄ | 98.5 | 0.1 | 0.1 |
| SM 34:2;O2 B | 11.25 | C₃₉H₇₇O₆N₂P₁ | C₅H₇₇O₆N₂P₁¹³C₃₄ | 98.4 | 1.9 | 3.3 |
| SM 34:1;O2 A | 11.8 | C₃₉H₇₉O₆N₂P₁ | C₅H₇₉O₆N₂P₁¹³C₃₄ | 98.2 | 0.8 | 0.2 |
| SM 34:1;O2 B | 12.1 | C₃₉H₇₉O₆N₂P₁ | C₅H₇₉O₆N₂P₁¹³C₃₄ | 98.1 | 0.1 | 0.1 |
| SM 34:1;O2 C | 12.36 | C₃₉H₇₉O₆N₂P₁ | C₅H₇₉O₆N₂P₁¹³C₃₄ | 98.3 | 43.2 | 50.8 |
| SM 34:0;O2 | 12.93 | C₃₉H₈₁O₆N₂P₁ | C₅H₈₁O₆N₂P₁¹³C₃₄ | 98.3 | 13.0 | 2.0 |
| SM 36:2;O2 | 12.9 | C₄₁H₈₁O₆N₂P₁ | C₅H₈₁O₆N₂P₁¹³C₃₆ | 98.0 | 0.6 | 1.1 |
| SM 36:1;O2 | 13.78 | C₄₁H₈₃O₆N₂P₁ | C₅H₈₃O₆N₂P₁¹³C₃₆ | 98.0 | 1.3 | 2.6 |
| SM 36:0;O2 | 14.25 | C₄₁H₈₅O₆N₂P₁ | C₅H₈₅O₆N₂P₁¹³C₃₆ | 98.2 | 0.3 | 0.1 |
| SM 40:2;O2 | 15.24 | C₄₅H₈₉O₆N₂P₁ | C₅H₈₉O₆N₂P₁¹³C₄₀ | 98.2 | 0.4 | 0.4 |
| SM 40:1;O2 | 15.92 | C₄₅H₉₁O₆N₂P₁ | C₅H₉₁O₆N₂P₁¹³C₄₀ | 98.4 | 1.9 | 2.3 |
| SM 40:0;O2 | 16.2 | C₄₅H₉₃O₆N₂P₁ | C₅H₉₃O₆N₂P₁¹³C₄₀ | 98.2 | 0.2 | <0.1 |
| SM 41:1;O2 | 16.35 | C₄₆H₉₃O₆N₂P₁ | C₅H₉₃O₆N₂P₁¹³C₄₁ | 99.6 | <0.1 | 0.1 |
| SM 42:3;O2 | 15.17 | C₄₇H₉₁O₆N₂P₁ | C₅H₉₁O₆N₂P₁¹³C₄₂ | 98.5 | 2.3 | 2.6 |
| SM 42:2;O2 A | 15.81 | C₄₇H₉₃O₆N₂P₁ | C₅H₉₃O₆N₂P₁¹³C₄₂ | 98.3 | 14.4 | 15.8 |
| SM 42:2;O2 B | 16.18 | C₄₇H₉₃O₆N₂P₁ | C₅H₉₃O₆N₂P₁¹³C₄₂ | 98.7 | 4.3 | 2.7 |
| SM 42:1;O2 | 16.75 | C₄₇H₉₅O₆N₂P₁ | C₅H₉₅O₆N₂P₁¹³C₄₂ | 98.3 | 12.1 | 13.0 |
| SM 42:0;O2 | 17.05 | C₄₇H₉₇O₆N₂P₁ | C₅H₉₇O₆N₂P₁¹³C₄₂ | 98.3 | 0.4 | 0.1 |
| SM 44:2;O2 | 16.65 | C₄₉H₉₇O₆N₂P₁ | C₅H₉₇O₆N₂P₁¹³C₄₄ | 99.1 | 0.6 | 0.8 |
| SM 44:1;O2 | 17.48 | C₄₉H₉₉O₆N₂P₁ | C₅H₉₉O₆N₂P₁¹³C₄₄ | 98.2 | 0.4 | 0.4 |

| **Cer** | | | | | | |
|---|---|---|---|---|---|---|
| Cer 32:1;O2 | 12.3 | C₃₂H₆₃O₃N₁ | ¹³C₃₂H₆₃O₃N₁ | 98.9 | 0.8 | 0.6 |
| Cer 32:0;O2 | 12.83 | C₃₂H₆₅O₃N₁ | ¹³C₃₂H₆₅O₃N₁ | 98.8 | 0.2 | 0.1 |
| Cer 33:1;O2 | 13.09 | C₃₃H₆₅O₃N₁ | ¹³C₃₃H₆₅O₃N₁ | 99.3 | <0.1 | 0.1 |
| Cer 33:0;O2 | 13.52 | C₃₃H₆₇O₃N₁ | ¹³C₃₃H₆₇O₃N₁ | 98.9 | <0.1 | 0.1 |
| Cer 34:2;O2 | 12.81 | C₃₄H₆₅O₃N₁ | ¹³C₃₄H₆₅O₃N₁ | 98.7 | 1.3 | 1.2 |
| Cer 34:1;O2 A | 13.19 | C₃₄H₆₇O₃N, | ¹³C₃₄H₆₇O₃N₁ | 98.4 | 0.7 | 0.2 |
| Cer 34:1;O2 B | 13.75 | C₃₄H₆₇O₃N₁ | ¹³C₃₄H₆₇O₃N₁ | 98.4 | 17.8 | 12.2 |
| Cer 34:0;O2 A | 14.18 | C₃₄H₆₉O₃N₁ | ¹³C₃₄H₆₉O₃N₁ | 98.5 | 4.3 | 1.1 |
| Cer 34:0;O2 B | 14.38 | C₃₄H₆₉O₃N₁ | ¹³C₃₄H₆₉O₃N₁ | 98.6 | 0.6 | 0.6 |
| Cer 35:1;O2 | 14.36 | C₃₅H₆₉O₃N₁ | ¹³C₃₅H₆₉O₃N₁ | 98.5 | 0.1 | 0.3 |
| Cer 35:0;O2 | 14.78 | C₃₅H₇₁O₃N₁ | ¹³C₃₅H₇₁O₃N₁ | 98.8 | <0.1 | 0.2 |
| Cer 36:2;O2 | 14.15 | C₃₆H₆₉O₃N₁ | ¹³C₃₆H₆₉O₃N₁ | 98.4 | 1.0 | 1.2 |
| Cer 36:1;O2 A | 14.48 | C₃₆H₇₁O₃N₁ | ¹³C₃₆H₇₁O₃N₁ | 98.8 | 0.3 | 0.2 |
| Cer 36: 1;O2 B | 14.94 | C₃₆H₇₁O₃N₁ | ¹³C₃₆H₇₁O₃N₁ | 98.7 | 1.8 | 2.6 |
| Cer 36:0;O2 | 15.3 | C₃₆H₇₃O₃N₁ | ¹³C₃₆H₇₃O₃N₁ | 98.6 | 0.3 | 0.4 |
| Cer 38:2;O2 | 15.25 | C₃₈H₇₃O₃N₁ | ¹³C₃₈H₇₃O₃N₁ | 98.6 | 0.1 | 0.2 |
| Cer 38:1;O2 A | 15.54 | C₃₈H₇₅O₃N₁ | ¹³C₃₈H₇₃O₃N₁ | 99.1 | 0.1 | 0.1 |
| Cer 38:1;O2 B | 15.93 | C₃₈H₇₅O₃N₁ | ¹³C₃₈H₇₃O₃N₁ | 98.8 | 0.2 | 1.1 |
| Cer 38:0;O2 | 16.21 | C₃₈H₇₇O₃N₁ | ¹³C₃₈H₇₇O₃N₁ | 98.9 | 0.1 | 1.0 |
| Cer 40:2;O2 A | 15.82 | C₄₀H₇₇O₃N₁ | ¹³C₄₀H₇₇O₃N₁ | 98.8 | 0.1 | 0.1 |
| Cer 40:2;O2 B | 16 | C₄₀H₇₇O₃N₁ | ¹³C₄₀H₇₇O₃N₁ | 98.9 | <0.1 | 0.1 |
| Cer 40:2;O2 | 16.18 | C₄₀H₇₇O₃N₁ | ¹³C₄₀H₇₇O₃N₁ | 98.5 | 0.6 | 0.8 |
| Cer 40:1;O2 A | 16.42 | C₄₀H₇₉O₃N₁ | ¹³C₄₀H₇₉O₃N₁ | 98.6 | 0.3 | 0.2 |
| Cer 40:1;O2 B | 16.75 | C₄₀H₇₉O₃N₁ | ¹³C₄₀H₇₉O₃N₁ | 98.4 | 2.3 | 5.2 |
| Cer 40:0;O2 | 17.04 | C₄₀H₈₁O₃N₁ | ¹³C₄₀H₈₁O₃N₁ | 98.3 | 0.7 | 2.6 |
| Cer 41:1;O2 | 17.15 | C₄₁H₈₁O₃N₁ | ¹³C₄₁H₈₁O₃N₁ | 98.5 | 0.2 | 3.0 |
| Cer 41:0;O2 | 17.41 | C₄₁H₈₃O₃N₁ | ¹³C₄₁H₈₃O₃N₁ | 98.9 | 0.1 | 1.8 |
| Cer 42:3;O2 A | 15.9 | C₄₂H₇₉O₃N₁ | ¹³C₄₂H₇₉O₃N₁ | 98.6 | 0.2 | 0.3 |
| Cer 42:3;O2 B | 16.1 | C₄₂H₇₉O₃N₁ | ¹³C₄₂H₇₉O₃N₁ | 98.5 | 5.0 | 3.7 |
| Cer 42:2;O2 A | 16.33 | C₄₂H₈₁O₃N₁ | ¹³C₄₂H₈₁O₃N₁ | 98.6 | 2.1 | 0.5 |
| Cer 42:2;O2 B | 16.65 | C₄₂H₈₁O₃N₁ | ¹³C₄₂H₈₁O₃N₁ | 98.5 | 15.7 | 11.7 |
| Cer 42:2;O2 C | 16.98 | C₄₂H₈₁O₃N₁ | ¹³C₄₂H₈₁O₃N₁ | 98.5 | 6.7 | 3.9 |
| Cer 42:1;O2 A | 16.94 | C₄₂H₈₃O₃N₁ | ¹³C₄₂H₈₃O₃N₁ | 98.4 | 2.0 | 0.9 |
| Cer 42:1;O2 B | 17.2 | C₄₂H₈₃O₃N₁ | ¹³C₄₂H₈₃O₃N₁ | 98.5 | 1.9 | 0.6 |
| Cer 42:1;O2 C | 17.5 | C₄₂H₈₃O₃N₁ | ¹³C₄₂H₈₃O₃N₁ | 98.5 | 24.5 | 23.9 |
| Cer 42:0;O2 | 17.74 | C₄₂H₈₅O₃N₁ | ¹³C₄₂H₈₅O₃N₁ | 98.4 | 2.9 | 5.4 |
| Cer 43:1;O2 | 17.85 | C₄₃H₈₅O₃N₁ | ¹³C₄₃H₈₅O₃N₁ | 98.1 | 0.3 | 0.4 |
| Cer 44:3;O2 A | 16.7 | C₄₄H₈₃O₃N₁ | ¹³C₄₄H₈₃O₃N₁ | 98.4 | 0.4 | 0.3 |
| Cer 44:3;O2 B | 16.9 | C₄₄H₈₃O₃N₁ | ¹³C₄₄H₈₃O₃N₁ | 98.5 | 0.7 | 0.6 |
| Cer 44:2;O2 A | 17.4 | C₄₄H₈₅O₃N₁ | ¹³C₄₄H₈₅O₃N₁ | 98.5 | 1.5 | 1.3 |
| Cer 44:2;O2 B | 17.58 | C₄₄H₈₅O₃N₁ | ¹³C₄₄H₈₅O₃N₁ | 98.6 | 0.4 | 0.5 |
| Cer 44:1;O2 A | 17.65 | C₄₄H₈₇O₃N₁ | ¹³C₄₄H₈₇O₃N₁ | 98.4 | 0.3 | 0.3 |
| Cer 44:1;O2 B | 17.8 | C₄₄H₈₇O₃N₁ | ¹³C₄₄H₈₇O₃N₁ | 98.3 | 0.3 | 0.3 |
| Cer 44:1;O2 C | 18.15 | C₄₄H₈₇O₃N₁ | ¹³C₄₄H₈₇O₃N₁ | 98.3 | 1.0 | 4.4 |
| Cer 44:0;O2 | 18.38 | C₄₄H₈₉O₃N₁ | ¹³C₄₄H₈₉O₃N₁ | 98.5 | 0.1 | 3.6 |

| **HexCer** | | | | | | |
|---|---|---|---|---|---|---|
| HexCer 34:1;O2 | 12.79 | C₄₀H₇₇O₈N₁ | ¹³C₄₀H₇₇O₈N₁ | 98.6 | 23.1 | 26.0 |
| HexCer 34:0;O2 | 13.3 | C₄₀H₇₉O₈N₁ | ¹³C₄₀H₇₉O₈N₁ | 98.5 | 3.1 | 0.6 |
| HexCer 36:2;O2 | 13.3 | C₄₂H₇₉O₈N₁ | ¹³C₄₂H₇₉O₈N₁ | nd | 0.2 | 0.1 |
| HexCer 36:1;O2 | 14.23 | C₄₂H₈₁O₈N₁ | ¹³C₄₂H₈₁O₈N₁ | 98.4 | 1.5 | 3.1 |
| HexCer 36:0;O2 | 14.53 | C₄₂H₈₃O₈N₁ | ¹³C₄₂H₈₃O₈N₁ | 98.5 | 0.3 | 0.1 |
| HexCer 38:1;O2 | 15.19 | C₄₄H₈₅O₈N₁ | ¹³C₄₄H₈₅O₈N₁ | 99.0 | 0.5 | 0.4 |
| HexCer 40:2;O2 A | 15.12 | C₄₆H₈₇O₈N₁ | ¹³C₄₆H₈₇O₈N₁ | 99.5 | 0.1 | 0.1 |
| HexCer 40:2;O2 B | 15.48 | C₄₆H₈₇O₈N₁ | ¹³C₄₆H₈₇O₈N₁ | nd | 0.1 | 0.1 |
| HexCer 40:1;O2 | 16.1 | C₄₆H₈₉O₈N₁ | ¹³C₄₆H₈₉O₈N₁ | 98.6 | 6.5 | 7.0 |
| HexCer 40:0;O2 | 16.4 | C₄₆H₉₁O₈N₁ | ¹³C₄₆H₉₁O₈N₁ | 98.5 | 0.8 | 0.2 |
| HexCer 41:1;O2 | 16.5 | C₄₇H₉₁O₈N₁ | ¹³C₄₇H₉₁O₈N₁ | 98.8 | 0.3 | 0.2 |
| HexCer 42:3;O2 | 15.4 | C₄₈H₈₉O₈N₁ | ¹³C₄₈H₈₉O₈N₁ | nd | 0.4 | 0.5 |
| HexCer 42:2;O2 | 16 | C₄₈H₉₁O₈N₁ | ¹³C₄₈H₉₁O₈N₁ | 98.5 | 17.7 | 17.8 |
| HexCer 42:1;O2 | 16.87 | C₄₈H₉₃O₈N₁ | ¹³C₄₈H₉₃O₈N₁ | 98.5 | 39.4 | 39.0 |
| HexCer 42:0;O2 | 17.15 | C₄₈H₉₅O₈N₁ | ¹³C₄₈H₉₅O₈N₁ | 98.4 | 1.8 | 0.4 |
| HexCer 44:3;O2 | 16.1 | C₅₀H₉₃O₈N₁ | ¹³C₅₀H₉₃O₈N₁ | 98.4 | 0.3 | 0.5 |
| HexCer 44:2;O2 | 16.78 | C₅₀H₉₅O₈N₁ | ¹³C₅₀H₉₅O₈N₁ | 98.4 | 1.5 | 1.7 |
| HexCer 44:1;O2 | 17.57 | C₅₀H₉₇O₈N₁ | ¹³C₅₀H₉₇O₈N₁ | 98.4 | 1.0 | 1.3 |
| HexCer 34:0;O3 | 12.05 | C₄₀H₇₉O₉N₁ | ¹³C₄₀H₇₉O₉N₁ | 98.5 | 0.3 | 0.3 |
| HexCer 42:1;O3 | 15.55 | C₄₈H₉₃O₉N₁ | ¹³C₄₈H₉₃O₉N₁ | 98.6 | 0.3 | nd |
| HexCer 42:0;O3 | 16.48 | C₄₈H₉₅O₉N₁ | ¹³C₄₈H₉₅O₉N₁ | nd | 0.7 | 0.7 |

| **Hex2Cer** | | | | | | |
|---|---|---|---|---|---|---|
| Hex2Cer 32:1;O2 | 10.64 | C₄₄H₈₃O₁₃N₁ | ¹³C₄₄H₈₃O₁₃N₁ | 98.4 | 0.9 | 0.8 |
| Hex2Cer 32:0;O2 | 11.32 | C₄₄H₈₅O₁₃N₁ | ¹³C₄₄H₈₅O₁₃N₁ | 98.7 | 0.1 | <0.1 |
| Hex2Cer 34:2;O2 | 11.25 | C₄₆H₈₅O₁₃N₁ | ¹³C₄₆H₈₅O₁₃N₁ | 98.5 | 0.3 | 0.4 |
| Hex2Cer 34:1;O2 A | 11.78 | C₄₆H₈₇O₁₃N₁ | ¹³C₄₆H₈₇O₁₃N₁ | 98.7 | 0.2 | 0.1 |
| Hex2Cer 34:1;O2 B | 12.38 | C₄₆H₈₇O₁₃N₁ | ¹³C₄₆H₈₇O₁₃N₁ | 98.5 | 35.6 | 36.2 |
| Hex2Cer 34:0;O2 | 12.9 | C₄₆H₈₉O₁₃N₁ | ¹³C₄₆H₈₉O₁₃N₁ | 98.5 | 4.1 | 0.9 |
| Hex2Cer 35:1;O2 | 13.09 | C₄₇H₈₉O₁₃N₁ | ¹³C₄₇H₈₉O₁₃N₁ | 99.2 | 0.2 | 0.1 |
| Hex2Cer 36:2;O2 | 12.85 | C₄₈H₈₉O₁₃N₁ | ¹³C₄₈H₈₉O₁₃N₁ | 98.2 | 0.2 | 0.3 |
| Hex2Cer 3 6:1;O2 | 13.75 | C₄₈H₉₁O₁₃N₁ | ¹³C₄₈H₉₁O₁₃N₁ | 98.5 | 1.5 | 1.9 |
| Hex2Cer 38:1;O2 | 14.85 | C₅₀H₉₅O₁₃N₁ | ¹³C₅₀H₉₅O₁₃N₁ | 98.3 | 0.3 | 0.3 |
| Hex2Cer 38:0;O2 | 15.21 | C₅₀H₉₇O₁₃N₁ | ¹³C₅₀H₉₇O₁₃N₁ | 98.5 | 0.1 | <0.1 |
| Hex2Cer 40:2;O2 A | 14.8 | C₅₂H₉₇O₁₃N₁ | ¹³C₅₂H₉₇O₁₃N₁ | 99.3 | 0.2 | 0.2 |
| Hex2Cer 40:2;O2 B | 15.15 | C₅₂H₉₇O₁₃N₁ | ¹³C₅₂H₉₇O₁₃N₁ | 99.5 | 0.2 | 0.2 |
| Hex2Cer 40:1;O2 A | 15.4 | C₅₂H₉₉O₁₃N₁ | ¹³C₅₂H₉₉O₁₃N₁ | 98.8 | 0.1 | <0.1 |
| Hex2Cer 40:1;O2 B | 15.79 | C₅₂H₉₉O₁₃N₁ | ¹³C₅₂H₉₉O₁₃N₁ | 98.5 | 5.4 | 6.0 |
| Hex2Cer 40:0;O2 | 16.1 | C₅₂H₁₀₁O₁₃N₁ | ¹³C₅₂H₁₀₁O₁₃N₁ | 98.5 | 0.8 | 0.2 |
| Hex2Cer 41:1;O2 | 16.2 | C₅₃H₁₀₁O₁₃N₁ | ¹³C₅₃H₁₀₁O₁₃N₁ | 98.4 | 0.1 | 0.1 |
| Hex2Cer 42:3;O2 A | 14.86 | C₅₄H₉₉O₁₃N₁ | ¹³C₅₄H₉₉O₁₃N₁ | 98.8 | 0.2 | 0.2 |
| Hex2Cer 42:3;O2 B | 15.05 | C₅₄H₉₉O₁₃N₁ | ¹³C₅₄H₉₉O₁₃N₁ | 98.6 | 0.6 | 0.7 |
| Hex2Cer 42:2;O2 | 15.69 | C₅₄H₁₀₁O₁₃N₁ | ¹³C₅₄H₁₀₁O₁₃N₁ | 98.5 | 14.9 | 18.3 |
| Hex2Cer 42:1;O2 A | 16 | C₅₄H₁₀₃O₁₃N₁ | ¹³C₅₄H₁₀₃O₁₃N₁ | 98.3 | 1.5 | 0.4 |
| Hex2Cer 42:1;O2 B | 16.6 | C₅₄H₁₀₃O₁₃N₁ | ¹³C₅₄H₁₀₃O₁₃N₁ | 98.5 | 28.1 | 28.6 |
| Hex2Cer 42:0;O2 | 16.85 | C₅₄H₁₀₅O₁₃N₁ | ¹³C₅₄H₁₀₅O₁₃N₁ | 98.3 | 1.6 | 0.4 |
| Hex2Cer 44:3;O2 | 15.7 | C₅₆H₁₀₃O₁₃N₁ | ¹³C₅₆H₁₀₃O₁₃N₁ | 98.4 | 0.2 | 0.3 |
| Hex2Cer 44:2;O2 | 16.5 | C₅₆H₁₀₅O₁₃N₁ | ¹³C₅₆H₁₀₅O₁₃N₁ | 98.4 | 1.0 | 1.4 |
| Hex2Cer 44:1;O2 | 17.1 | C₅₆H₁₀₇O₁₃N₁ | ¹³C₅₆H₁₀₇O₁₃N₁ | 98.4 | 0.6 | 0.7 |
| Hex2Cer 34:0;O3 | 11.63 | C₄₆H₈₉O₁₄N₁ | ¹³C₄₆H₈₉O₁₄N₁ | 98.5 | 0.4 | 0.4 |
| Hex2Cer 42:1;O3 | 15.25 | C₅₄H₁₀₃O₁₄N₁ | ¹³C₅₄H₁₀₃O₁₄N₁ | 98.3 | 0.3 | 0.3 |
| Hex2Cer 42:0;O3 | 16.19 | C₅₄H₁₀₅O₁₄N₁ | ¹³C₅₄H₁₀₅O₁₄N₁ | 98.4 | 0.3 | 0.4 |

| **Hex4Cer** | | | | | | |
|---|---|---|---|---|---|---|
| Hex4Cer 34:1;O2 | 11.82 | C₆₀H₁₁₀O₂₃N₂ | ¹³C₆₀H₁₁₀O₂₃N₂ | 98.5 | 77.2 | 51.6 |
| Hex4Cer 42:2;O2 | 15.32 | C₆₈H₁₂₄O₂₃N₂ | ¹³C₆₈H₁₂₄O₂₃N₂ | 98.2 | 7.2 | 20.2 |
| Hex4Cer 42:1;O2 | 16.25 | C₆₈H₁₂₆O₂₃N₂ | ¹³C₆₈H₁₂₆O₂₃N₂ | 99.0 | 15.6 | 28.2 |

| **GM3** | | | | | | |
|---|---|---|---|---|---|---|
| GM3 34:1 | 10.8 | C₅₇H₁₀₄O₂₁N₂ | ¹³C₅₇H₁₀₄O₂₁N₂ | 98.7 | 39.8 | 37.3 |
| GM3 34:0 | 11.4 | C₅₇H₁₀₆O₂₁N₂ | ¹³C₅₇H₁₀₆O₂₁N₂ | 98.7 | 2.3 | 1.9 |
| GM3 36:1 | 12.4 | C₅₉H₁₀₈O₂₁N₂ | ¹³C₅₉H₁₀₈O₂₁N₂ | 99.7 | 2.4 | 2.5 |
| GM3 40:1 | 14.65 | C₆₃H₁₁₆O₂₁N₂ | ¹³C₆₃H₁₁₆O₂₁N₂ | 99.0 | 6.2 | 6.4 |
| GM3 42:2 | 14.56 | C₆₅H₁₁₈O₂₁N₂ | ¹³C₆₅H₁₁₈O₂₁N₂ | 98.7 | 18.5 | 19.3 |
| GM3 42:1 | 15.52 | C₆₅H₁₂₀O₂₁N₂ | ¹³C₆₅H₁₂₀O₂₁N₂ | 98.5 | 30.9 | 32.6 |

| **Sterols** | | | | | | |
|---|---|---|---|---|---|---|
| Cholesterol | 11.46 | C₂₇H₄₆O₁ | ¹³C₂₇H₄₆O₁ | 97.6 | 100 | 100 |

| **DG** | | | | | | |
|---|---|---|---|---|---|---|
| DG 32:1 A | 14.6 | C₃₅H₆₆O₅ | ¹³C₃₅H₆₆O₅ | 97.9 | 6.3 | 10.8 |
| DG 32:1 B | 14.8 | C₃₅H₆₆O₅ | ¹³C₃₅H₆₆O₅ | 98.7 | 5.1 | 6.4 |
| DG 34:2 A | 14.65 | C₃₇H₆₈O₅ | ¹³C₃₇H₆₈O₅ | 98.5 | 8.1 | 6.9 |
| DG 34:2 B | 14.82 | C₃₇H₆₈O₅ | ¹³C₃₇H₆₈O₅ | 98.0 | 11.0 | 9.0 |
| DG 34:1 | 15.64 | C₃₇H₇₀O₅ | ¹³C₃₇H₇₀O₅ | 98.6 | 31.2 | 30.0 |
| DG 36:2 | 15.65 | C₃₉H₇₂O₅ | ¹³C₃₉H₇₂O₅ | 98.7 | 24.5 | 21.4 |
| DG 36:1 | 16.53 | C₃₉H₇₄O₅ | ¹³C₃₉H₇₄O₅ | 98.7 | 13.8 | 15.6 |

| **TG** | | | | | | |
|---|---|---|---|---|---|---|
| TG 40:2 | 16.77 | C₄₃H₇₈O₆ | ¹³C₄₃H₇₈O₆ | 98.8 | <0.01 | 0.01 |
| TG 40:1 | 17.45 | C₄₃H₈₀O₆ | ¹³C₄₃H₈₀O₆ | 98.4 | <0.01 | 0.01 |
| TG 40:0 | 18 | C₄₃H₈₂O₆ | ¹³C₄₃H₈₂O₆ | 99.1 | <0.01 | 0.04 |
| TG 42:2 | 17.5 | C₄₅H₈₂O₆ | ¹³C₄₅H₈₂O₆ | 98.2 | <0.01 | 0.02 |
| TG 42:1 | 18.05 | C₄₃H₈₄O₆ | ¹³C₄₅H₈₄O₆ | 98.3 | <0.01 | 0.05 |
| TG 42:0 | 18.61 | C₄₅H₈₆O₆ | ¹³C₄₅H₈₆O₆ | 98.5 | 0.01 | 0.10 |
| TG 43:2 | 17.8 | C₄₆H₈₄O₆ | ¹³C₄₆H₈₄O₆ | 98.7 | <0.01 | 0.02 |
| TG 43:1 | 18.3 | C₄₆H₈₆O₆ | ¹³C₄₆H₈₆O₆ | 98.3 | <0.01 | 0.05 |
| TG 43:0 | 18.8 | C₄₆H₈₈O₆ | ¹³C₄₆H₈₈O₆ | 99.1 | <0.01 | 0.10 |
| TG 44:3 A | 17.44 | C₄₇H₈₄O₆ | ¹³C₄₇H₈₄O₆ | 98.5 | <0.01 | 0.01 |
| TG 44:3 B | 17.52 | C₄₇H₈₄O₆ | ¹³C₄₇H₈₄O₆ | 98.5 | <0.01 | 0.01 |
| TG 44:2 A | 17.95 | C₄₇H₈₆O₆ | ¹³C₄₇H₈₆O₆ | 98.4 | 0.01 | 0.04 |
| TG 44:2 B | 18.14 | C₄₇H₈₆O₆ | ¹³C₄₇H₈₆O₆ | 98.5 | 0.02 | 0.09 |
| TG 44:1 A | 18.58 | C₄₇H₈₈O₆ | ¹³C₄₇H₈₈O₆ | 98.8 | 0.04 | 0.03 |
| TG 44:1 B | 18.65 | C₄₇H₈₈O₆ | ¹³C₄₇H₈₈O₆ | 98.8 | 0.04 | 0.16 |
| TG 44:0 | 19.16 | C₄₇H₉₀O₆ | ¹³C₄₇H₉₀O₆ | 99.0 | 0.05 | 0.25 |
| TG 45:2 A | 18.22 | C₄₈H₈₈O₆ | ¹³C₄₈H₈₈O₆ | 98.4 | <0.01 | 0.03 |
| TG 45:2 B | 18.35 | C₄₈H₈₈O₆ | ¹³C₄₈H₈₈O₆ | 98.5 | <0.01 | 0.06 |
| TG 45:2 C | 18.47 | C₄₈H₈₈O₆ | ¹³C₄₈H₈₈O₆ | 98.2 | <0.01 | 0.08 |
| TG 45:1 A | 18.75 | C₄₈H₉₀O₆ | ¹³C₄₈H₉₀O₆ | 98.7 | 0.01 | 0.11 |
| TG 45:1 B | 18.84 | C₄₈H₉₀O₆ | ¹³C₄₈H₉₀O₆ | 98.5 | 0.01 | 0.21 |
| TG 45:1 C | 18.94 | C₄₈H₉₀O₆ | ¹³C₄₈H₉₀O₆ | 98.3 | <0.01 | 0.20 |
| TG 45:0 A | 19.32 | C₄₈H₉₂O₆ | ¹³C₄₈H₉₂O₆ | 98.6 | 0.01 | 0.13 |
| TG 45:0 B | 19.41 | C₄₈H₉₂O₆ | ¹³C₄₈H₉₂O₆ | 98.2 | <0.01 | 0.13 |
| TG 46:4 | 17.65 | C₄₉H₈₆O₆ | ¹³C₄₉H₈₆O₆ | 98.3 | <0.01 | <0.01 |
| TG 46:3 A | 18.02 | C₄₉H₈₈O₆ | ¹³C₄₉H₈₈O₆ | 98.4 | 0.01 | 0.03 |
| TG 46:3 B | 18.12 | C₄₉H₈₈O₆ | ¹³C₄₉H₈₈O₆ | 98.4 | 0.02 | 0.05 |
| TG 46:3 C | 18.25 | C₄₉H₈₈O₆ | ¹³C₄₉H₈₈O₆ | 98.5 | 0.01 | 0.08 |
| TG 46:2 A | 18.59 | C₄₉H₉₀O₆ | ¹³C₄₉H₉₀O₆ | 98.5 | 0.08 | 0.16 |
| TG 46:2 B | 18.65 | C₄₉H₉₀O₆ | ¹³C₄₉H₉₀O₆ | 98.5 | 0.11 | 0.21 |
| TG 46:2 C | 18.74 | C₄₉H₉₀O₆ | ¹³C₄₉H₉₀O₆ | 98.5 | 0.07 | 0.41 |
| TG 46:1 A | 19.14 | C₄₉H₉₂O₆ | ¹³C₄₉H₉₂O₆ | 98.5 | 0.34 | 0.56 |
| TG 46:1 B | 19.22 | C₄₉H₉₂O₆ | ¹³C₄₉H₉₂O₆ | 98.4 | 0.23 | 0.57 |
| TG 46:0 | 19.66 | C₄₉H₉₄O₆ | ¹³C₄₉H₉₄O₆ | 98.5 | 0.21 | 0.08 |
| TG 47:3 | 18.4 | C₅₀H₉₀O₆ | ¹³C₅₀H₉₀O₆ | 98.3 | <0.01 | 0.05 |
| TG 47:2 | 18.85 | C₅₀H₉₂O₆ | ¹³C₅₀H₉₂O₆ | 98.4 | 0.01 | 0.26 |
| TG 47:1 | 19.35 | C₅₀H₉₄O₆ | ¹³C₅₀H₉₄O₆ | 98.5 | 0.04 | 0.30 |
| TG 47:0 | 19.85 | C₅₀H₉₆O₆ | ¹³C₅₀H₉₆O₆ | 98.4 | 0.01 | 0.13 |
| TG 48:4 | 18.28 | C₅₁H₉₀O₆ | ¹³C₅₁H₉₀O₆ | 98.4 | 0.01 | 0.03 |
| TG 48:3 A | 18.64 | C₅₁H₉₂O₆ | ¹³C₅₁H₉₂O₆ | 98.5 | 0.11 | 0.14 |
| TG 48:3 B | 18.73 | C₅₁H₉₂O₆ | ¹³C₅₁H₉₂O₆ | 98.6 | 0.13 | 0.26 |
| TG 48:3 C | 18.83 | C₅₁H₉₂O₆ | ¹³C₅₁H₉₂O₆ | 98.5 | 0.06 | nd |
| TG 48:3 D | 18.92 | C₅₁H₉₂O₆ | ¹³C₅₁H₉₂O₆ | 98.5 | 0.03 | nd |
| TG 48:2 A | 19.14 | C₅₁H₉₄O₆ | ¹³C₅₁H₉₄O₆ | 98.4 | 0.70 | 0.74 |
| TG 48:2 B | 19.2 | C₅₁H₉₄O₆ | ¹³C₅₁H₉₄O₆ | 98.4 | 0.90 | 0.98 |
| TG 48:2 C | 19.27 | C₅₁H₉₄O₆ | ¹³C₅₁H₉₄O₆ | 98.4 | 0.44 | 0.73 |
| TG 48:1 A | 19.63 | C₅₁H₉₆O₆ | ¹³C₅₁H₉₆O₆ | 98.4 | 1.37 | 1.20 |
| TG 48:1 B | 19.72 | C₅₁H₉₆O₆ | ¹³C₅₁H₉₆O₆ | 98.4 | 0.74 | 0.68 |
| TG 48:0 | 20.11 | C₅₁H₉₈O₆ | ¹³C₅₁H₉₈O₆ | 98.6 | 0.07 | 0.02 |
| TG 49:3 | 18.92 | C₅₂H₉₄O₆ | ¹³C₅₂H₉₄O₆ | 98.4 | 0.02 | 0.12 |
| TG 49:2 A | 19.24 | C₅₂H₉₆O₆ | ¹³C₅₂H₉₆O₆ | 98.5 | 0.03 | 0.08 |
| TG 49:2 B | 19.35 | C₅₂H₉₆O₆ | ¹³C₅₂H₉₆O₆ | 98.5 | 0.08 | 0.21 |
| TG 49:2 C | 19.44 | C₅₂H₉₆O₆ | ¹³C₅₂H₉₆O₆ | 98.5 | 0.08 | 0.29 |
| TG 49:1 A | 19.77 | C₅₂H₉₈O₆ | ¹³C₅₂H₉₈O₆ | 98.6 | 0.10 | 0.18 |
| TG 49:1 B | 19.85 | C₅₂H₉₈O₆ | ¹³C₅₂H₉₈O₆ | 98.6 | 0.14 | 0.33 |
| TG 49:0 A | 20.24 | C₅₂H₁₀₀O₆ | ¹³C₅₂H₁₀₀O₆ | 98.5 | 0.03 | 0.03 |
| TG 49:0 B | 20.32 | C₅₂H₁₀₀O₆ | ¹³C₅₂H₁₀₀O₆ | 98.3 | 0.03 | 0.08 |
| TG 50:4 A | 18.64 | C₅₃H₉₄O₆ | ¹³C₅₃H₉₄O₆ | 98.3 | 0.04 | 0.07 |
| TG 50:4 B | 18.74 | C₅₃H₉₄O₆ | ¹³C₅₃H₉₄O₆ | 98.4 | 0.06 | 0.11 |
| TG 50:4 C | 18.88 | C₅₃H₉₄O₆ | ¹³C₅₃H₉₄O₆ | 98.5 | 0.09 | 0.12 |
| TG 50:3 A | 19.09 | C₅₃H₉₆O₆ | ¹³C₅₃H₉₆O₆ | 98.3 | 0.58 | 0.72 |
| TG 50:3 B | 19.17 | C₅₃H₉₆O₆ | ¹³C₅₃H₉₆O₆ | 98.4 | 1.07 | 0.89 |
| TG 50:3 C | 19.26 | C₅₃H₉₆O₆ | ¹³C₅₃H₉₆O₆ | 98.4 | 0.77 | 0.65 |
| TG 50:3 D | 19.43 | C₅₃H₉₆O₆ | ¹³C₅₃H₉₆O₆ | 98.5 | 0.26 | 0.30 |
| TG 50:2 A | 19.6 | C₅₃H₉₈O₆ | ¹³C₅₃H₉₈O₆ | 98.4 | 3.40 | 2.89 |
| TG 50:2 B | 19.68 | C₅₃H₉₈O₆ | ¹³C₅₃H₉₈O₆ | 98.4 | 2.96 | 2.50 |
| TG 50:1 A | 20.08 | C₅₃H₁₀₀O₆ | ¹³ C₅₃H₁₀₀O₆ | 98.4 | 2.65 | 1.64 |
| TG 50:1 B | 20.17 | C₅₃H₁₀₀O₆ | ¹³C₅₃H₁₀₀O₆ | 98.4 | 1.07 | 2.18 |
| TG 50:0 | 20.52 | C₅₃H₁₀₂O₆ | ¹³C₅₃H₁₀₂O₆ | 98.5 | 0.77 | 0.76 |
| TG 51:4 A | 18.9 | C₅₄H₉₆O₆ | ¹³C₅₄H₉₆O₆ | 98.3 | 0.01 | 0.04 |
| TG 51:4 B | 19.05 | C₅₄H₉₆O₆ | ¹³C₅₄H₉₆O₆ | 98.4 | 0.01 | 0.03 |
| TG 51:3 A | 19.34 | C₅₄H₉₈O₆ | ¹³C₅₄H₉₈O₆ | 98.3 | 0.04 | 0.07 |
| TG 51:3 B | 19.42 | C₅₄H₉₈O₆ | ¹³C₅₄H₉₈O₆ | 98.4 | 0.09 | 0.14 |
| TG 51:3 C | 19.5 | C₅₄H₉₈O₆ | ¹³C₅₄H₉₈O₆ | 98.5 | 0.05 | 0.13 |
| TG 51:2 A | 19.73 | C₅₄H₁₀₀O₆ | ¹³C₅₄H₁₀₀O₆ | 98.4 | 0.14 | 0.12 |
| TG 51:2 B | 19.84 | C₅₄H₁₀₀O₆ | ¹³C₅₄H₁₀₀O₆ | 98.5 | 0.32 | 0.34 |
| TG 51:2 C | 19.92 | C₅₄H₁₀₀O₆ | ¹³C₅₄H₁₀₀O₆ | 98.5 | 0.22 | 0.31 |
| TG 51:1 A | 20.19 | C₅₄H₁₀₂O₆ | ¹³C₅₄H₁₀₂O₆ | 98.5 | 0.20 | 0.18 |
| TG 51:1 B | 20.28 | C₅₄H₁₀₂O₆ | ¹³C₅₄H₁₀₂O₆ | 98.5 | 0.23 | 0.30 |
| TG 52:5 A | 18.76 | C₅₅H₉₆O₆ | ¹³C₅₅H₉₆O₆ | 98.3 | 0.02 | nd |
| TG 52:5 B | 18.89 | C₅₅H₉₆O₆ | ¹³C₅₅H₉₆O₆ | 98.5 | 0.08 | nd |
| TG 52:5 C | 18.96 | C₅₅H₉₆O₆ | ¹³C₅₅H₉₆O₆ | 98.5 | 0.08 | nd |
| TG 52:4 A | 19.17 | C₅₅H₉₈O₆ | ¹³C₅₅H₉₈O₆ | 98.3 | 0.45 | 0.53 |
| TG 52:4 B | 19.24 | C₅₅H₉₈O₆ | ¹³C₅₅H₉₈O₆ | 98.3 | 0.55 | 0.29 |
| TG 52:4 C | 19.4 | C₅₅H₉₈O₆ | ¹³C₅₅H₉₈O₆ | 98.4 | 0.64 | 0.50 |
| TG 52:3 A | 19.57 | C₅₅H₁₀₀O₆ | ¹³C₅₅H₁₀₀O₆ | 98.4 | 3.11 | 2.70 |
| TG 52:3 B | 19.65 | C₅₅H₁₀₀O₆ | ¹³C₅₅H₁₀₀O₆ | 98.4 | 3.43 | 3.03 |
| TG 52:3 C | 19.73 | C₅₅H₁₀₀O₆ | ¹³C₅₅H₁₀₀O₆ | 98.4 | 1.88 | 1.21 |
| TG 52:3 D | 19.8 | C₅₅H₁₀₀O₆ | ¹³C₅₅H₁₀₀O₆ | 98.4 | 0.96 | 0.41 |
| TG 52:2 A | 20.04 | C₅₅H₁₀₂O₆ | ¹³C₅₅H₁₀₂O₆ | 98.4 | 6.76 | 6.40 |
| TG 52:2 B | 20.14 | C₅₅H₁₀₂O₆ | ¹³C₅₅H₁₀₂O₆ | 98.4 | 3.36 | 2.24 |
| TG 52:1 | 20.48 | C₅₅H₁₀₄O₆ | ¹³ C₅₅H₁₀₄O₆ | 98.4 | 3.56 | 3.94 |
| TG 52:0 | 20.9 | C₅₅H₁₀₆O₆ | ¹³C₅₅H₁₀₆O₆ | 98.5 | 0.47 | 0.97 |
| TG 53:5 | 19.15 | C₅₆H₉₈O₆ | ¹³C₅₆H₉₈O₆ | 98.3 | 0.01 | nd |
| TG 53:4 A | 19.44 | C₅₆H₁₀₀O₆ | ¹³C₅₆H₁₀₀O₆ | 98.2 | 0.02 | 0.05 |
| TG 53:4 B | 19.52 | C₅₆H₁₀₀O₆ | ¹³C₅₆H₁₀₀O₆ | 98.3 | 0.04 | 0.05 |
| TG 53:4 C | 19.64 | C₅₆H₁₀₀O₆ | ¹³C₅₆H₁₀₀O₆ | 98.3 | 0.03 | nd |
| TG 53:3 A | 19.81 | C₅₆H₁₀₂O₆ | ¹³C₅₆H₁₀₂O₆ | 98.4 | 0.22 | 0.21 |
| TG 53:3 B | 19.88 | C₅₆H₁₀₂O₆ | ¹³C₅₆H₁₀₂O₆ | 98.4 | 0.22 | 0.22 |
| TG 53:2 A | 20.17 | C₅₆H₁₀₄O₆ | ¹³C₅₆H₁₀₄O₆ | 98.4 | 0.25 | 0.25 |
| TG 53:2 B | 20.24 | C₅₆H₁₀₄O₆ | ¹³C₅₆H₁₀₄O₆ | 98.4 | 0.40 | 0.38 |
| TG 53:2 C | 20.31 | C₅₆H₁₀₄O₆ | ¹³C₅₆H₁₀₄O₆ | 98.5 | 0.21 | 0.16 |
| TG 53:1 A | 20.58 | C₅₆H₁₀₆O₆ | ¹³C₅₆H₁₀₆O₆ | 98.4 | 0.10 | 0.15 |
| TG 53:1 B | 20.67 | C₅₆H₁₀₆O₆ | ¹³C₅₆H₁₀₆O₆ | 98.5 | 0.10 | 0.16 |
| TG 54:6 | 19 | C₅₇H₉₈O₆ | ¹³C₅₇H₉₈O₆ | 98.3 | 0.03 | 0.04 |
| TG 54:5 A | 19.25 | C₅₇H₁₀₀O₆ | ¹³C₅₇H₁₀₀O₆ | 98.2 | 0.15 | 0.36 |
| TG 54:5 B | 19.38 | C₅₇H₁₀₀O₆ | ¹³C₅₇H₁₀₀O₆ | 98.4 | 0.60 | 0.34 |
| TG 54:5 C | 19.44 | C₅₇H₁₀₀O₆ | ¹³C₅₇H₁₀₀O₆ | 98.4 | 0.52 | 0.17 |
| TG 54:4 A | 19.62 | C₅₇H₁₀₂O₆ | ¹³C₅₇H₁₀₂O₆ | 98.3 | 1.28 | 1.15 |
| TG 54:4 B | 19.73 | C₅₇H₁₀₂O₆ | ¹³C₅₇H₁₀₂O₆ | 98.3 | 1.25 | 0.86 |
| TG 54:4 C | 19.86 | C₅₇H₁₀₂O₆ | ¹³C₅₇H₁₀₂O₆ | 98.4 | 1.63 | 1.09 |
| TG 54:3 A | 20.01 | C₅₇H₁₀₄O₆ | ¹³C₅₇H₁₀₄O₆ | 98.4 | 5.98 | 6.09 |
| TG 54:3 B | 20.1 | C₅₇H₁₀₄O₆ | ¹³C₅₇H₁₀₄O₆ | 98.4 | 3.43 | 2.51 |
| TG 54:3 C | 20.2 | C₅₇H₁₀₄O₆ | ¹³C₅₇H₁₀₄O₆ | 98.4 | 1.39 | 0.44 |
| TG 54:2 A | 20.45 | C₅₇H₁₀₆O₆ | ¹³C₅₇H₁₀₆O₆ | 98.4 | 5.64 | 6.36 |
| TG 54:2 B | 20.57 | C₅₇H₁₀₆O₆ | ¹³C₅₇H₁₀₆O₆ | 98.4 | 1.54 | 0.94 |
| TG 54:1 | 20.86 | C₅₇H₁₀₈O₆ | ¹³C₅₇H₁₀₈O₆ | 98.4 | 1.45 | 2.04 |
| TG 54:0 | 21.24 | C₅₇H₁₁₀O₆ | ¹³C₅₇H₁₁₀O₆ | 98.6 | 0.09 | 0.32 |
| TG 55:5 A | 19.5 | C₅₈H₁₀₂O₆ | ¹³C₅₈H₁₀₂O₆ | 98.2 | 0.02 | nd |
| TG 55:5 B | 19.6 | C₅₈H₁₀₂O₆ | ¹³C₅₈H₁₀₂O₆ | 98.4 | 0.03 | nd |
| TG 55:4 A | 19.85 | C₅₈H₁₀₄O₆ | ¹³C₅₈H₁₀₄O₆ | 98.2 | 0.04 | 0.07 |
| TG 55:4 B | 19.96 | C₅₈H₁₀₄O₆ | ¹³C₅₈H₁₀₄O₆ | 98.3 | 0.09 | 0.09 |
| TG 55:3 A | 20.18 | C₅₈H₁₀₆O₆ | ¹³C₅₈H₁₀₆O₆ | 98.3 | 0.11 | 0.14 |
| TG 55:3 B | 20.25 | C₅₈H₁₀₆O₆ | ¹³C₅₈H₁₀₆O₆ | 98.3 | 0.11 | 0.17 |
| TG 55:2 A | 20.56 | C₅₈H₁₀₈O₆ | ¹³C₅₈H₁₀₈O₆ | 98.3 | 0.07 | 0.11 |
| TG 55:2 B | 20.64 | C₅₈H₁₀₈O₆ | ¹³C₅₈H₁₀₈O₆ | 98.4 | 0.07 | 0.12 |
| TG 55:1 | 20.96 | C₅₈H₁₁₀O₆ | ¹³C₅₈H₁₁₀O₆ | 98.5 | 0.02 | 0.04 |
| TG 56:6 A | 19.44 | C₅₉H₁₀₂O₆ | ¹³C₅₉H₁₀₂O₆ | 98.3 | 0.23 | 0.17 |
| TG 56:6 B | 19.54 | C₅₉H₁₀₂O₆ | ¹³C₅₉H₁₀₂O₆ | 98.4 | 0.19 | 0.11 |
| TG 56:5 A | 19.71 | C₅₉H₁₀₄O₆ | ¹³C₅₉H₁₀₄O₆ | 98.3 | 0.57 | 0.50 |
| TG 56:5 B | 19.83 | C₅₉H₁₀₄O₆ | ¹³C₅₉H₁₀₄O₆ | 98.4 | 1.73 | 1.34 |
| TG 56:4 A | 20.06 | C₅₉H₁₀₆O₆ | ¹³C₅₉H₁₀₆O₆ | 98.2 | 0.99 | 0.93 |
| TG 56:4 B | 20.17 | C₅₉H₁₀₆O₆ | ¹³C₅₉H₁₀₆O₆ | 98.4 | 1.26 | 1.03 |
| TG 56:4 C | 20.27 | C₅₉H₁₀₆O₆ | ¹³C₅₉H₁₀₆O₆ | 98.4 | 1.10 | 0.91 |
| TG 56:3 A | 20.43 | C₅₉H₁₀₈O₆ | ¹³C₅₉H₁₀₈O₆ | 98.3 | 1.82 | 1.89 |
| TG 56:3 B | 20.57 | C₅₉H₁₀₈O₆ | ¹³C₅₉H₁₀₈O₆ | 98.3 | 0.63 | 0.50 |
| TG 56:2 | 20.82 | C₅₉H₁₁₀O₆ | ¹³C₅₉H₁₁₀O₆ | 98.4 | 1.24 | 1.41 |
| TG 56:1 | 21.2 | C₅₉H₁₁₂O₆ | ¹³C₅₉H₁₁₂O₆ | 98.4 | 0.44 | 0.61 |
| TG 56:0 | 21.56 | C₅₉H₁₁₄O₆ | ¹³C₅₉H₁₁₄O₆ | 98.5 | 0.07 | 0.35 |
| TG 57:6 | 19.6 | C₆₀H₁₀₄O₆ | ¹³C₆₀H₁₀₄O₆ | 98.2 | 0.01 | 0.01 |
| TG 57:5 A | 19.94 | C₆₀H₁₀₆O₆ | ¹³C₆₀H₁₀₆O₆ | 98.3 | 0.04 | nd |
| TG 57:5 B | 19.99 | C₆₀H₁₀₆O₆ | ¹³C₆₀H₁₀₆O₆ | 98.3 | 0.03 | nd |
| TG 57:4 A | 20.26 | C₆₀H₁₀₈O₆ | ¹³ C₆₀H₁₀₈O₆ | 98.0 | 0.03 | 0.04 |
| TG 57:4 B | 20.36 | C₆₀H₁₀₈O₆ | ¹³C₆₀H₁₀₈O₆ | 98.3 | 0.04 | 0.05 |
| TG 57:3 | 20.62 | C₆oH₁₁₀O₆ | ¹³C₆₀H₁₁₀O₆ | 98.2 | 0.03 | 0.05 |
| TG 58:9 | 18.9 | C₆₁H₁₀₀O₆ | ¹³C₆₁H₁₀₀O₆ | 98.3 | 0.01 | nd |
| TG 572 A | 20.93 | C₆₀H₁₁₂O₆ | ¹³C₆₀H₁₁₂O₆ | 98.3 | 0.02 | 0.03 |
| TG 572 B | 20.99 | C₆₀H₁₁₂O₆ | ¹³C₆₀H₁₁₂O₆ | 98.5 | 0.02 | 0.05 |
| TG 57:1 | 21.32 | C₆₀H₁₁₄O₆ | ¹³C₆₀H₁₁₄O₆ | 98.5 | 0.01 | 0.01 |
| TG 58:7 A | 19.4 | C₆₁H₁₀₄O₆ | ¹³C₆₁H₁₀₄O₆ | 97.9 | 0.02 | 0.02 |
| TG 58:7 B | 19.53 | C₆₁H₁₀₄O₆ | ¹³C₆₁H₁₀₄O₆ | 98.3 | 0.05 | 0.04 |
| TG 58:7 C | 19.64 | C₆₁H₁₀₄O₆ | ¹³C₆₁H₁₀₄O₆ | 98.4 | 0.10 | 0.06 |
| TG 58:6 A | 19.8 | C₆₁H₁₀₆O₆ | ¹³C₆₁H₁₀₆O₆ | 98.2 | 0.35 | 0.25 |
| TG 58:6 B | 19.87 | C₆₁H₁₀₆O₆ | ¹³C₆₁H₁₀₆O₆ | 98.2 | 0.31 | 0.23 |
| TG 58:6 C | 19.96 | C₆₁H₁₀₆O₆ | ¹³C₆₁H₁₀₆O₆ | 98.3 | 0.28 | 0.15 |
| TG 58:5 A | 20.14 | C₆₁H₁₀₈O₆ | ¹³C₆₁H₁₀₈O₆ | 98.4 | 0.97 | 0.84 |
| TG 58:5 B | 20.24 | C₆₁H₁₀₈O₆ | ¹³C₆₁H₁₀₈O₆ | 98.3 | 0.72 | 0.63 |
| TG 58:4 A | 20.42 | C₆₁H₁₁₀O₆ | ¹³ C₆₁H₁₁₀O₆ | 98.2 | 0.46 | 0.44 |
| TG 58:4 B | 20.56 | C₆₁H₁₁₀O₆ | ¹³C₆₁H₁₁₀O₆ | 98.4 | 0.74 | 0.65 |
| TG 58:3 A | 20.79 | C₆₁H₁₁₂O₆ | ¹³C₆₁H₁₁₂O₆ | 98.4 | 1.03 | 1.00 |
| TG 58:3 B | 20.92 | C₆₁H₁₁₂O₆ | ¹³C₆₁H₁₁₂O₆ | 98.4 | 0.21 | 0.18 |
| TG 58:2 | 21.16 | C₆₁H₁₁₄O₆ | ¹³C₆₁H₁₁₄O₆ | 98.4 | 1.04 | 1.22 |
| TG 58:1 | 21.52 | C₆₁H₁₁₆O₆ | ¹³C₆₁H₁₁₆O₆ | 98.4 | 0.53 | 0.89 |
| TG 58:0 | 21.85 | C₆₁H₁₁₈O₆ | ¹³C₆₁H₁₁₈O₆ | 98.6 | 0.03 | 0.30 |
| TG 59:5 | 20.34 | C₆₂H₁₁₀O₆ | ¹³C₆₂H₁₁₀O₆ | 98.3 | 0.02 | nd |
| TG 59:4 | 20.64 | C₆₂H₁₁₂O₆ | ¹³C₆₂H₁₁₂O₆ | 98.3 | 0.01 | 0.02 |
| TG 59:3 | 20.95 | C₆₂H₁₁₄O₆ | ¹³C₆₂H₁₁₄O₆ | 98.4 | 0.02 | 0.04 |
| TG 59:2 | 21.3 | C₆₂H₁₁₆O₆ | ¹³C₆₂H₁₁₆O₆ | 98.5 | 0.02 | 0.05 |
| TG 60:8 | 19.6 | C₆₃H₁₀₆O₆ | ¹³C₆₃H₁₀₆O₆ | 98.2 | 0.01 | 0.01 |
| TG 59:1 | 21.65 | C₆₂H₁₁₈O₆ | ¹³C₆₂H₁₁₈O₆ | 98.5 | 0.01 | 0.03 |
| TG 60:7 A | 19.83 | C₆₃H₁₀₈O₆ | ¹³C₆₃H₁₀₈O₆ | 98.1 | 0.04 | 0.05 |
| TG 60:7 B | 19.96 | C₆₃H₁₀₈O₆ | ¹³C₆₃H₁₀₈O₆ | 98.3 | 0.13 | 0.09 |
| TG 60:6 A | 20.15 | C₆₃H₁₁₀O₆ | ¹³C₆₃H₁₁₀O₆ | 98.2 | 0.23 | 0.02 |
| TG 60:6 B | 20.22 | C₆₃H₁₁₀O₆ | ¹³C₆₃H₁₁₀O₆ | 98.3 | 0.42 | 0.34 |
| TG 60:5 A | 20.45 | C₆₃H₁₁₂O₆ | ¹³C₆₃H₁₁₂O₆ | 98.3 | 0.19 | 0.24 |
| TG 60:5 B | 20.55 | C₆₃H₁₁₂O₆ | ¹³C₆₃H₁₁₂O₆ | 98.4 | 0.47 | 0.39 |
| TG 60:5 C | 20.63 | C₆₃H₁₁₂O₆ | ¹³C₆₃H₁₁₂O₆ | 98.4 | 0.33 | 0.26 |
| TG 60:4 A | 20.76 | C₆₃H₁₁₄O₆ | ¹³C₆₃H₁₁₄O₆ | 98.3 | 0.45 | 0.45 |
| TG 60:4 B | 20.85 | C₆₃H₁₁₄O₆ | ¹³C₆₃H₁₁₄O₆ | 98.3 | 0.35 | 0.22 |
| TG 60:3 A | 21.12 | C₆₃H₁₁₆O₆ | ¹³C₆₃H₁₁₆O₆ | 98.4 | 1.25 | 1.42 |
| TG 60:3 B | 21.2 | C₆₃H₁₁₆O₆ | ¹³C₆₃H₁₁₆O₆ | 98.3 | 0.55 | 0.48 |
| TG 60:2 | 21.47 | C₆₃H₁₁₈O₆ | ¹³C₆₃H₁₁₈O₆ | 98.4 | 0.98 | 1.52 |
| TG 60:1 | 21.82 | C₆₃H₁₂₀O₆ | ¹³C₆₃H₁₂₀O₆ | 98.4 | 0.15 | 0.83 |
| TG 61:4 | 20.95 | C₆₄H₁₁₆O₆ | ¹³C₆₄H₁₁₆O₆ | 98.2 | 0.01 | 0.02 |
| TG 61:3 | 21.26 | C₆₄H₁₁₈O₆ | ¹³C₆₄H₁₁₈₇O₆ | 98.4 | 0.02 | 0.04 |
| TG 61:2 | 21.6 | C₆₄H₁₂₀O₆ | ¹³C₆₄H₁₂₀O₆ | 98.5 | 0.01 | 0.03 |
| TG 62:8 | 19.95 | C₆₅H₁₁₀O₆ | ¹³C₆₅H₁₁₀O₆ | 98.2 | 0.02 | 0.01 |
| TG 62:7 | 20.25 | C₆₅H₁₁₂O₆ | ¹³C₆₅H₁₁₂O₆ | 98.2 | 0.08 | 0.07 |
| TG 62:6 | 20.52 | C₆₅H₁₁₄O₆ | ¹³C₆₅H₁₁₄O₆ | 98.4 | 0.38 | 0.32 |
| TG 62:5 A | 20.78 | C₆₅H₁₁₆O₆ | ¹³C₆₅H₁₁₆O₆ | 98.2 | 0.13 | nd |
| TG 62:5 B | 20.94 | C₆₅H₁₁₆O₆ | ¹³C₆₅H₁₁₆O₆ | 98.4 | 0.24 | 0.27 |
| TG 62:4 A | 21.07 | C₆₅H₁₁₈O₆ | ¹³C₆₅H₁₁₈O₆ | 98.3 | 0.41 | 0.44 |
| TG 62:4 B | 21.14 | C₆₅H₁₁₈O₆ | ¹³C₆₅H₁₁₈O₆ | 98.2 | 0.22 | 0.12 |
| TG 62:3 | 21.42 | C₆₅H₁₂₀O₆ | ¹³C₆₅H₁₂₀O₆ | 98.4 | 0.77 | 1.11 |
| TG 62:2 | 21.76 | C₆₅H₁₂₂O₆ | ¹³C₆₅H₁₂₂O₆ | 98.4 | 0.23 | 0.75 |
| TG 63:3 | 21.55 | C₆₆H₁₂₂O₆ | ¹³C₆₆H₁₂₂O₆ | 98.3 | <0.01 | 0.01 |
| TG 64:8 A | 20.23 | C₆₇H₁₁₄O₆ | ¹³C₆₇H₁₁₄O₆ | 98.1 | 0.01 | 0.01 |
| TG 64:8 B | 20.34 | C₆₇H₁₁₄O₆ | ¹³C₆₇H₁₁₄O₆ | 98.4 | 0.05 | 0.04 |
| TG 64:7 | 20.57 | C₆₇H₁₁₆O₆ | ¹³C₆₇H₁₁₆O₆ | 98.1 | 0.03 | 0.04 |
| TG 64:6 | 20.9 | C₆₇H₁₁₈O₆ | ¹³C₆₇H₁₁₈O₆ | 98.2 | 0.08 | 0.09 |
| TG 64:5 A | 21.18 | C₆₇H₁₂₀O₆ | ¹³C₆₇H₁₂₀O₆ | 98.3 | 0.08 | 0.08 |
| TG 64:5 B | 21.27 | C₆₇H₁₂₀O₆ | ¹³C₆₇H₁₂₀O₆ | 98.4 | 0.09 | 0.14 |
| TG 64:4 A | 21.42 | C₆₇H₁₂₂O₆ | ¹³C₆₇H₁₂₂O₆ | 97.8 | 0.05 | 0.11 |
| TG 64:4 B | 21.55 | C₆₇H₁₂₂O₆ | ¹³C₆₇H₁₂₂O₆ | 98.3 | 0.04 | 0.06 |
| TG 66:8 | 20.61 | C₆₉H₁₁₈O₆ | ¹³C₆₉H₁₁₈O₆ | 98.3 | 0.02 | 0.02 |
| TG 66:7 | 20.88 | C₆₉H₁₂₀O₆ | ¹³C₆₉H₁₂₀O₆ | 98.2 | 0.01 | 0.01 |
| TG 66:6 | 21.19 | C₆₉H₁₂₂O₆ | ¹³C₆₉H₁₂₂O₆ | 98.2 | 0.02 | 0.03 |
| TG 66:5 | 21.48 | C₆₉H₁₂₄O₆ | ¹³C₆₉H₁₂₄O₆ | 98.4 | 0.03 | 0.05 |
| TG 66:4 A | 21.67 | C₆₉H₁₂₆O₆ | ¹³C₆₉H₁₂₆O₆ | 98.3 | 0.01 | 0.02 |
| TG 66:4 B | 21.85 | C₆₉H₁₂₆O₆ | ¹³C₆₉H₁₂₆O₆ | 98.5 | <0.01 | 0.02 |
| TG 68:9 | 20.68 | C₇₁H₁₂₀O₆ | ¹³C₇₁H₁₂₀O₆ | 98.4 | <0.01 | <0.01 |
| TG 68:8 | 20.92 | C₇₁H₁₂₂O₆ | ¹³C₇₁H₁₂₂O₆ | 98.7 | <0.01 | <0.01 |
| TG 68:7 | 21.18 | C₇₁H₁₂₄O₆ | ¹³C₇₁H₁₂₄O₆ | 98.0 | <0.01 | <0.01 |
| TG 68:6 | 21.5 | C₇₁H₁₂₆O₆ | ¹³C₇₁H₁₂₆O₆ | 98.3 | 0.01 | 0.01 |
| TG 68:5 | 21.75 | C₇₁H₁₂₈O₆ | ¹³C₇₁H₁₂₈O₆ | 98.3 | <0.01 | <0.01 |

**Table 5. Examples of ¹³C labeled peptides identified in cell line cultivated with ¹³C labeled substrates. A total of 3728 ¹³C labeled peptides were identified through global proteomic analysis. This table highlights a selected subset of these peptides.**

| **Peptide Sequence** | **¹³C labeling Efficiency** | **Protein accession** | **Protein Name** | **Charge** | **m/z** | **Elemental composition (neutral form)** |
|---|---|---|---|---|---|---|
| TEFLSFMNTELAA FTK (SEQ ID NO. 1) | 98.13% | P31949 | Protein S100-A11 | 2 | 968.0973 | ¹³C₈₅H₁₂₈N₁₈O₂₆S₁ |
| KQSLGELIGTLNA AK (SEQ ID NO. 2) | 98.39% | P60174 | Triosephosphate isomerase | 2 | 805.5577 | ¹³C₆₇H₁₁₉N₁₉O₂₂ |
| GAGTGGLGLAVE GPSEAK (SEQ ID NO. 3) | 98.54% | P21333 | Filamin-A | 2 | 819.0175 | ¹³C₆₆H₁₁₁N₁₉O₂₅ |

Before performing global proteomic analysis, cell cultures grown with and without ¹³C-labeled substrates were prepared and analyzed in triplicate. The samples were lysed using sodium deoxycholate, reduced with tris(2-carboxyethyl)phosphine, alkylated with methanethiosulfonate, and digested following a previously published protocol [M. Vajrychová, J. Stráník, K. Pimková, M. Barman, R. Kukla, P. Zedníková, R. Bolehovská, L. Plíšková, H. Hornychová, C. Andrys, V. Tambor, J. Lenčo, B. Jacobsson, M. Kacerovsky, Sci. Rep. 10 (2020) 17696]. Desalted peptides were analyzed using a nano-liquid chromatography system coupled to a high-resolution mass spectrometer equipped with high-field asymmetric waveform ion mobility.

The acquired data were processed using Proteome Discoverer (v3.0) with the Mascot search engine against the human reference proteome (UniProt). In the initial analysis, data from both ¹²C and ¹³C samples were processed without applying specific modifications in the search parameters. In the subsequent analysis, amino acids were grouped based on their carbon atom count, and specific modifications were assigned to reflect the incorporation of ¹³C atoms. For this, ¹³C-labeled amino acids (A, R, N, D, C, Q, E, G, H, I, L, K, M, F, P, S, T, Y, V) were defined as static modifications, while ¹³C-labeled tryptophan (W) was set as a variable modification. The total number of proteins identified using both approaches is presented in **Figure 1****.** Labeling efficiency was assessed by comparing the characteristic isotopic patterns of three selected peptides with the observed accurate masses of ions in the spectra (Table 5). The results demonstrated a high efficiency of ¹³C incorporation into complex protein structures (≥98%) and confirmed the feasibility of analyzing these extensively labeled proteins using standard proteomics tools, even though such tools were not initially designed for this purpose. Given the large number of unique peptides in each sample and the substantial number of carbon atoms in their structures, the use of highly pure ¹³C-labeled standards is critical. Impurities in the standards can result in underlabeled peptides with broad isotopic patterns, causing signal overlap, reduced MS signal intensity, and potential issues in data evaluation by proteomic software. In this study, the use of high-purity standards ensured accurate analysis and reliable results, highlighting the critical importance of such standards in advanced proteomic workflows.

### Use of the derived cell lines as stable isotope internal standard in mass spectrometry-based methods

Cell aliquots are gently defrosted and the extraction of the compounds of interest is carried out immediately. The investigated biomolecules can be extracted by various extraction procedures, such as liquid-liquid extraction, protein precipitation by organic solvent, solid phase extraction, or a selective extraction for the isolation in the case of some special applications. Then, extracted ¹³C biomolecules are mixed with the analyzed sample and sample can be prepared for the analysis. Preferably 2 million cells should be performed providing adequate number of ¹³C labeled aliquots for all study samples, but the amount of the ¹³C biomolecules as mixture of internal standards added to the sample is based on the matrix (plasma, serum, tissues, urine, cell lines, etc.) and amount of the sample used for analysis. The pooling approach is recommended to achieve a mixture of ¹²C samples and ¹³C biomolecules in an adequate ratio to obtain an optimal ratio in their intensities in the mass spectra, for example, ratio 1: 1. Cell aliquots contain a complex mixture of ¹³C labeled biomolecules with profiles comparable to those of human samples. Conventional methods using lipid species or lipid class separation can be used for the quantitation of ¹³C labeled internal standards to provide their molar concentrations. The IS mixture with known quantitative composition can be used for metabolomic, lipidomic, or proteomic quantitation of biological samples to obtain the molar concentration of endogenous biomolecules. The mixture of ¹³C labeled biomolecules can be used also for relative quantitation.

SIL-IS LC-MS data can be processed manually or using software tools, which offers significant advantages in the identification and quantification of biomolecules compared to conventional omics approaches. An algorithm can be used to search for all doublets present of endogenous compounds (¹²C) and internal labeled standards (¹³C) that lead to the highest confidential identification and absolute quantitative analysis. The retention times of both compounds should be present in the same retention time tolerance window. If no ¹³C labeled standards are matched for the ¹²C biomolecule, another ¹³C candidate with closest structural similarity within the corresponding lipid or metabolite class may be selected as alternative internal standard. This approach provides molar concentrations for all molecules detected with SIL twin peaks using stable isotope dilution MS quantitation.

### Application of ¹³C fully labeled biomolecules is fluxomics

Another key application of ¹³C fully labeled biomolecules is fluxomics and related biological experiments investigating the metabolic changes. In our experiment, the preference for the use of¹²C compounds from biosynthesis was observed; therefore, only fully ¹³C components in culture medium can be present. Cell culture without bovine serum and in culture medium with ¹³C component requires special practice leading to high resistance to stress conditions and adaptability of tumor cells, which maintain cell proliferation. In case of intentional addition of some supplement with the natural isotopic composition, the biosynthesized molecule will contain unlabeled ¹²C atoms at the positions that correspond to the metabolic biosynthetic path. This allows selective labeling at particular positions in a targeted manner for fluxomic experiments to be used for the monitoring of their biosynthetic pathways and the investigation of metabolic changes caused by serious diseases or the effect of a drug on the cellular level. This principle of labeling a targeted part of the molecule is illustrated using labeled (¹²C₃¹³C₂H₁₄NO) and unlabeled choline (¹²C₅H₁₄NO) in the culture medium. If there are two ¹³C atoms in choline, then in other products in whose biosynthesis choline participates, the masses will be shifted by two units. This assumption has been confirmed, as illustrated in **Table 6.**

**Table 6. Example of targeted labeling: Comparison of the elemental composition of lipid species found in cell lines cultivated with ¹³C substrates and the presence of either ¹²C choline or ¹³C₂-labeled choline in the supplement medium, highlighting specific systematic changes in the elemental composition of lipid species that use choline as a precursor molecule.**

| | | **Elemental Composition (neutral form)** | | **Experimental ¹³C isotopic purity [%]** | | **Relative abundance in samples** | |
|---|---|---|---|---|---|---|---|
| **Lipid species** | **tR [min]** | **¹³C cell lines cultivated with ¹²C choline** | **¹³C cell lines cultivated with ¹³C labeled choline** | **¹³C cell lines cultivated with ¹²C choline** | **¹³C cell lines cultivated with ¹³C labeled choline** | **¹³C cell lines cultivated with ¹²C choline** | **¹³C cell lines cultivated with ¹³C labeled choline** |
| **LPC** | | | | | | | |
| LPC 14:0 | 1.79 | C₅H₄₆O₇N₁P₁¹³C₁₇ | C₃H₄₆O₇N₁P₁¹³C₁₉ | 98.3 | 98.9 | 2.4 | 2.5 |
| LPC 16:0 | 2.48 | C₅H₅₀O₇N₁P₁¹³C₁₉ | C₃H₅₀O₇N₁P₁¹³C₂₁ | 98.3 | 98.7 | 18.5 | 9.4 |
| LPC 16:0 | 2.67 | C₅H₅₀O₇N₁P₁¹³C₁₉ | C₃H₅₀O₇N₁P₁¹³C₂₁ | 98.3 | 98.6 | 9.8 | 15.1 |
| LPC 16:1 | 1.90 | C₅H₄₈O₇N₁P₁¹³C₁₉ | C₃H₄₈O₇N₁P₁¹³C₂₁ | 98.7 | 98.9 | 9.3 | 8.2 |
| LPC 16:1 | 1.96 | C₅H₄₈O₇N₁P₁¹³C₁₉ | C₃H₄₈O₇N₁P₁¹³C₂₁ | 98.4 | 98.7 | 10.0 | 8.5 |
| LPC 18:2 | 2.00 | C₅H₅₀O₇N₁P₁¹³C₂₁ | C₃H₅₀O₇N₁P₁¹³C₂₃ | 98.6 | 99.4 | 1.1 | 1.3 |
| LPC 18:1 | 2.60 | C₅H₅₂O₇N₁P₁¹³C₂₁ | C₃H₅₂O₇N₁P₁¹³C₂₃ | 98.6 | 98.4 | 27.3 | 22.2 |
| LPC 18:1 | 2.80 | C₅H₅₂O₇N₁P₁¹³C₂₁ | C₃H₅₂O₇N₁P₁¹³C₂₃ | 98.4 | 98.6 | 7.3 | 12.4 |
| LPC 18:0 | 3.50 | C₅H₅₄O₇N₁P₁¹³C₂₁ | C₃H₅₄O₇N₁P₁¹³C₂₅ | 98.9 | 98.8 | 0.7 | 1.5 |
| LPC 18:0 | 3.85 | C₅H₅₄O₇N₁P₁¹³C₂₁ | C₃H₅₄O₇N₁P₁¹³C₂₅ | 98.6 | 98.5 | 4.2 | 10.9 |
| LPC 20:3 | 2.47 | C₅H₅₂O₇N₁P₁¹³C₂₃ | C₃H₅₂O₇N₁P₁¹³C₂₅ | 98.8 | 99.0 | 6.0 | 2.9 |
| LPC 20:2 | 2.80 | C₅H₅₄O₇N₁P₁¹³C₂₃ | C₃H₅₄O₇N₁P₁¹³C₂₅ | 99.2 | 99.4 | 1.0 | 0.5 |
| LPC 20:1 | 3.65 | C₅H₅₆O₇N₁P₁¹³C₂₃ | C₃H₅₆O₇N₁P₁¹³C₂₅ | 98.7 | 99.5 | 0.2 | 0.6 |
| LPC 20:1 | 3.95 | C₅H₅₆O₇N₁P₁¹³C₂₃ | C₃H₅₆O₇N₁P₁¹³C₂₅ | 99.2 | 99.7 | 0.4 | 0.8 |
| LPC 24:1 | 7.60 | C₅H₆₄O₇N₁P₁¹³C₂₇ | C₃H₆₄O₇N₁P₁¹³C₂₉ | 99.9 | 99.0 | 0.3 | 0.3 |
| LPC 24:0 | 9.82 | C₅H₆₆O₇N₁P₁¹³C₂₇ | C₃H₆₆O₇N₁P₁¹³C₂₉ | 99.1 | 99.0 | 0.9 | 1.7 |
| LPC 26:1 | 9.75 | C₅H₆₈O₇N₁P₁¹³C₂₉ | C₃H₆₈O₇N₁P₁¹³C₃₁ | 99.3 | 99.2 | 0.6 | 1.3 |

| **LPC O-** | | | | | | | |
|---|---|---|---|---|---|---|---|
| LPC 0-16:0 | 3.15 | C₅H₅₂O₆N₁P₁¹³C₁₉ | C₃H₅₂O₆N₁P₁¹³C₂₁ | 98.0 | 98.4 | 57.2 | 54.0 |
| LPC 0-18:1 | 3.28 | C₅H₅₄O₆N₁P₁¹³C₂₁ | C₃H₅₄O₆N₁P₁¹³C₂₃ | 98.9 | 98.5 | 15.2 | 10.1 |
| LPC 0-18:0 | 4.55 | C₅H₅₆O₆N₁P₁¹³C₂₁ | C₃H₅₆O₆N₁P₁¹³C₂₃ | 98.5 | 98.5 | 26.3 | 34.3 |
| LPC O-24:1 | 8.57 | C₅H₆₆O₆N₁P₁¹³C₂₇ | C₃H₆₆O₆N₁P₁¹³C₂₉ | 98.7 | 99.4 | 1.4 | 1.6 |

| **PC** | | | | | | | |
|---|---|---|---|---|---|---|---|
| PC 28:0 | 10.63 | C₅H₇₂O₈N₁P₁¹³C₃₁ | C₃H₇₂O₈N₁P₁¹³C₃₃ | 98.6 | 98.5 | 0.3 | 0.3 |
| PC 29:0 | 11.17 | C₅H₇₄O₈N₁P₁¹³C₃₂ | C₃H₇₄O₈N₁P₁¹³C₃₄ | 98.6 | 98.4 | 0.05 | 0.05 |
| PC 30:1 | 11.07 | C₅H₇₄O₈N₁P₁¹³C₃₃ | C₃H₇₄O₈N₁P₁¹³C₃₅ | 98.5 | 98.4 | 1.03 | 1.12 |
| PC 30:0 | 12.33 | C₅H₇₆O₈N₁P₁¹³C₃₃ | C₃H₇₆O₈N₁P₁¹³C₃₅ | 98.3 | 98.4 | 2.95 | 2.79 |
| PC 31:1 | 11.90 | C₅H₇₆O₈N₁P₁¹³C₃₄ | C₃H₇₆O₈N₁P₁¹³C₃₆ | 98.5 | 98.3 | 0.16 | 0.13 |
| PC 32:2 | 10.95 | C₅H₇₆O₈N₁P₁¹³C₃₅ | C₃H₇₆O₈N₁P₁¹³C₃₇ | 98.4 | 98.4 | 0.49 | 0.42 |
| PC 32:2 | 11.23 | C₅H₇₆O₈N₁P₁¹³C₃₅ | C₃H₇₆O₈N₁P₁¹³C₃₇ | 98.3 | 98.4 | 1.54 | 1.51 |
| PC 32:2 | 11.47 | C₅H₇₆O₈N₁P₁¹³C₃₅ | C₃H₇₆O₈N₁P₁¹³C₃₇ | 98.4 | 98.4 | 1.27 | 1.18 |
| PC 32:1 | 12.45 | C₅H₇₈O₈N₁P₁¹³C₃₅ | C₃H₇₈O₈N₁P₁¹³C₃₇ | 98.3 | 98.4 | 6.09 | 6.47 |
| PC 32:1 | 12.68 | C₅H₇₈O₈N₁P₁¹³C₃₅ | C₃H₇₈O₈N₁P₁¹³C₃₇ | 98.3 | 98.4 | 9.49 | 10.18 |
| PC 32:1 | 12.93 | C₅H₇₈O₈N₁P₁¹³C₃₅ | C₃H₇₈O₈N₁P₁¹³C₃₇ | 98.3 | 98.4 | 1.07 | 0.65 |
| PC 32:0 | 13.70 | C₅H₈₀O₈N₁P₁¹³C₃₅ | C₃H₈₀O₈N₁P₁¹³C₃₇ | 98.4 | 98.3 | 5.16 | 3.32 |
| PC 33:2 | 11.76 | C₅H₇₈O₈N₁P₁¹³C₃₆ | C₃H₇₈O₈N₁P₁¹³C₃₈ | 98.5 | 98.3 | 0.06 | 0.01 |
| PC 33:2 | 12.02 | C₅H₇₈O₈N₁P₁¹³C₃₆ | C₃H₇₈O₈N₁P₁¹³C₃₈ | 98.4 | 98.4 | 0.09 | 0.09 |
| PC 33:2 | 12.27 | C₅H₇₈O₈N₁P₁¹³C₃₆ | C₃H₇₈O₈N₁P₁¹³C₃₈ | 98.2 | 98.4 | 0.09 | 0.08 |
| PC 33:1 | 13.33 | C₅H₈₀O₈N₁P₁¹³C₃₆ | C₃H₈₀O₈N₁P₁¹³C₃₈ | 99.1 | nd | 0.30 | 0.28 |
| PC 34:3 | 11.20 | C₅H₇₈O₈N₁P₁¹³C₃₇ | C₃H₇₈O₈N₁P₁¹³C₃₉ | 98.5 | 98.6 | 0.14 | 0.12 |
| PC 34:3 | 11.35 | C₅H₇₈O₈N₁P₁¹³C₃₇ | C₃H₇₈O₈N₁P₁¹³C₃₉ | 98.5 | 98.5 | 0.33 | 0.29 |
| PC 34:3 | 11.60 | C₅H₇₈O₈N₁P₁¹³C₃₇ | C₃H₇₈O₈N₁P₁¹³C₃₉ | 98.4 | 98.5 | 0.60 | 0.56 |
| PC 34:3 | 11.82 | C₅H₇₈O₈N₁P₁¹³C₃₇ | C₃H₇₈O₈N₁P₁¹³C₃₉ | 98.5 | 98.5 | 0.27 | 0.25 |
| PC 34:3 | 12.05 | C₅H₇₈O₈N₁P₁¹³C₃₇ | C₃H₇₈O₈N₁P₁¹³C₃₉ | 98.5 | 98.4 | 0.36 | 0.35 |
| PC 34:2 | 12.52 | C₅H₈₀O₈N₁P₁¹³C₃₇ | C₃H₈₀O₈N₁P₁¹³C₃₉ | 98.3 | 98.4 | 4.79 | 4.88 |
| PC 34:2 | 12.75 | C₅H₈₀O₈N₁P₁¹³C₃₇ | C₃H₈₀O₈N₁P₁¹³C₃₉ | 98.3 | 98.4 | 6.73 | 7.49 |
| PC 34:2 | 12.96 | C₅H₈₀O₈N₁P₁¹³C₃₇ | C₃H₈₀O₈N₁P₁¹³C₃₉ | 98.4 | 98.4 | 1.67 | 1.67 |
| PC 34:1 | 13.75 | C₅H₈₂O₈N₁P₁¹³C₃₇ | C₃H₈₂O₈N₁P₁¹³C₃₉ | 98.3 | 99.8 | 14.72 | 15.21 |
| PC 34:1 | 13.95 | C₅H₈₂O₈N₁P₁¹³C₃₇ | C₃H₈₂O₈N₁P₁¹³C₃₉ | 98.4 | 98.4 | 2.88 | 2.81 |
| PC 34:0 | 14.81 | C₅H₈₄O₈N₁P₁¹³C₃₇ | C₃H₈₄O₈N₁P₁¹³C₃₉ | 98.4 | 98.4 | 1.69 | 1.29 |
| PC 35:2 | 13.18 | C₅H₈₂O₈N₁P₁¹³C₃₈ | C₃H₈₂O₈N₁P₁¹³C₄₀ | 98.1 | nd | 0.33 | 0.26 |
| PC 35:1 | 14.06 | C₅H₈₄O₈N₁P₁¹³C₃₈ | C₃H₈₄O₈N₁P₁¹³C₄₀ | 98.2 | 98.4 | 0.38 | 0.34 |
| PC 35:1 | 14.32 | C₅H₈₄O₈N₁P₁¹³C₃₈ | C₃H₈₄O₈N₁P₁¹³C₄₀ | 98.2 | 98.3 | 0.26 | 0.24 |
| PC 35:0 | 15.28 | C₅H₈₆O₈N₁P₁¹³C₃₈ | C₃H₈₆O₈N₁P₁¹³C₄₀ | 98.3 | 98.5 | 0.01 | 0.06 |
| PC 36:4 | 11.60 | C₅H₈₀O₈N₁P₁¹³C₃₉ | C₃H₈₀O₈N₁P₁¹³C₄₁ | 98.4 | 98.4 | 0.05 | 0.05 |
| PC 36:4 | 11.75 | C₅H₈₀O₈N₁P₁¹³C₃₉ | C₃H₈₀O₈N₁P₁¹³C₄₁ | 98.4 | 98.4 | 0.09 | 0.08 |
| PC 36:4 | 12.12 | C₅H₈₀O₈N₁P₁¹³C₃₉ | C₃H₈₀O₈N₁P₁¹³C₄₁ | 98.5 | 98.4 | 0.24 | 0.19 |
| PC 36:4 | 12.35 | C₅H₈₀O₈N₁P₁¹³C₃₉ | C₃H₈₀O₈N₁P₁¹³C₄₁ | 98.4 | 98.5 | 0.27 | 0.23 |
| PC 36:3 | 12.70 | C₅H₈₂O₈N₁P₁¹³C₃₉ | C₃H₈₂O₈N₁P₁¹³C₄₁ | 98.3 | 98.4 | 0.91 | 0.82 |
| PC 36:3 | 12.82 | C₅H₈₂O₈N₁P₁¹³C₃₉ | C₃H₈₂O₈N₁P₁¹³C₄₁ | 98.3 | 98.4 | 1.73 | 1.74 |
| PC 36:3 | 13.00 | C₅H₈₂O₈N₁P₁¹³C₃₉ | C₃H₈₂O₈N₁P₁¹³C₄₁ | 98.3 | 98.4 | 0.88 | 0.84 |
| PC 36:3 | 13.17 | C₅H₈₂O₈N₁P₁¹³C₃₉ | C₃H₈₂O₈N₁P₁¹³C₄₁ | 98.4 | 98.4 | 0.72 | 0.68 |
| PC 36:3 | 13.43 | C₅H₈₂O₈N₁P₁¹³C₃₉ | C₃H₈₂O₈N₁P₁¹³C₄₁ | 98.4 | 98.4 | 1.30 | 1.15 |
| PC 36:2 | 13.80 | C₅H₈₄O₈N₁P₁¹³C₃₉ | C₃H₈₄O₈N₁P₁¹³C₄₁ | 98.3 | 98.4 | 11.83 | 12.63 |
| PC 36:2 | 14.00 | C₅H₈₄O₈N₁P₁¹³C₃₉ | C₃H₈₄O₈N₁P₁¹³C₄₁ | 98.3 | 98.4 | 2.73 | 2.92 |
| PC 36:1 | 14.85 | C₅H₈₆O₈N₁P₁¹³C₃₉ | C₃H₈₆O₈N₁P₁¹³C₄₁ | 98.4 | 98.4 | 5.55 | 6.42 |
| PC 36:0 | 15.76 | C₅H₈₈O₈N₁P₁¹³C₃₉ | C₃H₈₈O₈N₁P₁¹³C₄₁ | 98.6 | 98.4 | 0.08 | 0.07 |
| PC 37:1 | 15.10 | C₅H₈₈O₈N₁P₁¹³C₄₀ | C₃H₈₈O₈N₁P₁¹³C₄₂ | 98.3 | 98.4 | 0.06 | 0.07 |
| PC 38:5 | 12.15 | C₅H₈₂O₈N₁P₁¹³C₄₁ | C₃H₈₂O₈N₁P₁¹³C₄₃ | 98.4 | 98.7 | 0.02 | 0.02 |
| PC 38:5 | 12.45 | C₅H₈₂O₈N₁P₁¹³C₄₁ | C₃H₈₂O₈N₁P₁¹³C₄₃ | 98.3 | 98.4 | 0.08 | 0.07 |
| PC 38:5 | 12.65 | C₅H₈₂O₈N₁P₁¹³C₄₁ | C₃H₈₂O₈N₁P₁¹³C₄₃ | 98.3 | 98.4 | 0.05 | 0.03 |
| PC 38:5 | 12.84 | C₅H₈₂O₈N₁P₁¹³C₄₁ | C₃H₈₂O₈N₁P₁¹³C₄₃ | 98.5 | 98.6 | 0.06 | 0.05 |
| PC 38:4 | 12.97 | C₅H₈₄O₈N₁P₁¹³C₄₁ | C₃H₈₄O₈N₁P₁¹³C₄₃ | 98.2 | 98.6 | 0.07 | 0.06 |
| PC 38:4 | 13.16 | C₅H₈₄O₈N₁P₁¹³C₄₁ | C₃H₈₄O₈N₁P₁¹³C₄₃ | 98.4 | 98.5 | 0.28 | 0.23 |
| PC 38:4 | 13.45 | C₅H₈₄O₈N₁P₁¹³C₄₁ | C₃H₈₄O₈N₁P₁¹³C₄₃ | 98.3 | 98.4 | 1.40 | 1.24 |
| PC 38:4 | 13.65 | C₅H₈₄O₈N₁P₁¹³C₄₁ | C₃H₈₄O₈N₁P₁¹³C₄₃ | 98.4 | 98.4 | 0.28 | 0.22 |
| PC 38:3 | 13.92 | C₅H₈₆O₈N₁P₁¹³C₄₁ | C₃H₈₆O₈N₁P₁¹³C₄₃ | 98.4 | 98.4 | 0.72 | 0.75 |
| PC 38:3 | 14.03 | C₅H₈₆O₈N₁P₁¹³C₄₁ | C₃H₈₆O₈N₁P₁¹³C₄₃ | 98.4 | 98.4 | 0.69 | 0.82 |
| PC 38:3 | 14.25 | C₅H₈₆O₈N₁P₁¹³ C₄₁ | C₃H₈₆O₈N₁P₁¹³C₄₃ | 98.5 | 98.5 | 0.50 | 0.40 |
| PC 38:3 | 14.58 | C₅H₈₆O₈N₁P₁¹³C₄₁ | C₃H₈₆O₈N₁P₁¹³C₄₃ | 98.4 | 98.4 | 0.60 | 0.61 |
| PC 38:2 | 14.80 | C₅H₈₈O₈N₁P₁¹³C₄₁ | C₃H₈₈O₈N₁P₁¹³C₄₃ | 98.4 | 98.4 | 0.79 | 0.86 |
| PC 38:2 | 14.94 | C₅H₈₈O₈N₁P₁¹³C₄₁ | C₃H₈₈O₈N₁P₁¹³C₄₃ | 98.4 | 98.4 | 0.64 | 0.63 |
| PC 38:1 | 15.77 | C₅H₉₀O₈N₁P₁¹³C₄₁ | C₃H₉₀O₈N₁P₁¹³C₄₃ | 98.2 | 98.4 | 0.13 | 0.15 |
| PC 38:0 | 16.56 | C₅H₉₂O₈N₁P₁¹³C₄₁ | C₃H₉₂O₈N₁P₁¹³C₄₃ | 98.3 | 98.5 | 0.02 | 0.02 |
| PC 40:6 | 13.10 | C₅H₈₄O₈N₁P₁¹³C₄₃ | C₃H₈₄O₈N₁P₁¹³C₄₅ | 98.5 | 98.7 | 0.08 | 0.05 |
| PC 40:5 | 13.45 | C₅H₈₆O₈N₁P₁¹³C₄₃ | C₃H₈₆O₈N₁P₁¹³C₄₅ | 98.4 | 98.6 | 0.05 | 0.04 |
| PC 40:5 | 13.65 | C₅H₈₆O₈N₁P₁¹³C₄₃ | C₃H₈₆O₈N₁P₁¹³C₄₅ | 98.3 | 98.4 | 0.05 | 0.03 |
| PC 40:5 | 13.90 | C₅H₈₆O₈N₁P₁¹³C₄₃ | C₃H₈₆O₈N₁P₁¹³C₄₅ | 98.3 | 98.4 | 0.06 | 0.03 |
| PC 40:4 | 14.30 | C₅H₈₈O₈N₁P₁¹³C₄₃ | C₃H₈₈O₈N₁P₁¹³C₄₅ | 98.5 | 98.5 | 0.45 | 0.32 |
| PC 40:4 | 14.54 | C₅H₈₈O₈N₁P₁¹³C₄₃ | C₃H₈₈O₈N₁P₁¹³C₄₅ | 98.5 | 98.6 | 0.12 | 0.11 |
| PC 40:3 | 14.98 | C₅H₉₀O₈N₁P₁¹³C₄₃ | C₃H₉₀O₈N₁P₁¹³C₄₅ | 98.7 | 98.3 | 0.11 | 0.08 |
| PC 40:3 | 15.28 | C₅H₉₀O₈N₁P₁¹³C₄₃ | C₃H₉₀O₈N₁P₁¹³C₄₅ | 98.7 | 98.5 | 0.11 | 0.10 |
| PC 40:2 | 15.75 | C₅H₉₂O₈N₁P₁¹³C₄₃ | C₃H₉₂O₈N₁P₁¹³C₄₅ | 98.5 | 98.6 | 0.13 | 0.15 |
| PC 40:1 | 16.50 | C₅H₉₄O₈N₁P₁¹³C₄₃ | C₃H₉₄O₈N₁P₁¹³C₄₅ | 98.4 | 98.4 | 0.08 | 0.06 |
| PC 42:5 | 14.55 | C₅H₉₀O₈N₁P₁¹³C₄₅ | C₃H₉₀O₈N₁P₁¹³C₄₇ | 98.4 | 98.3 | 0.05 | 0.03 |
| PC 42:4 | 15.11 | C₅H₉₂O₈N₁P₁¹³C₄₅ | C₃H₉₂O₈N₁P₁¹³C₄₇ | 98.6 | 98.3 | 0.04 | 0.03 |
| PC 42:4 | 15.25 | C₅H₉₂O₈N₁P₁¹³C₄₅ | C₃H₉₂O₈N₁P₁¹³C₄₇ | 98.7 | 98.4 | 0.07 | 0.04 |
| PC 42:3 | 15.80 | C₅H₉₄O₈N₁P₁¹³C₄₅ | C₃H₉₄O₈N₁P₁¹³C₄₇ | 98.6 | 98.6 | 0.06 | 0.05 |
| PC 42:2 | 16.48 | C₅H₉₆O₈N₁P₁¹³C₄₅ | C₃H₉₆O₈N₁P₁¹³C₄₇ | 98.4 | 98.5 | 0.17 | 0.16 |
| PC 42:1 | 17.27 | C₅H₉₈O₈N₁P₁¹³C₄₅ | C₃H₉₈O₈N₁P₁¹³C₄₇ | 98.3 | 98.4 | 0.09 | 0.10 |
| PC 42:0 | 17.90 | C₅H₁₀₀O₈N₁P₁¹³C₄₅ | C₃H₁₀₀O₈N₁P₁¹³C₄₇ | 98.2 | 98.2 | 0.02 | 0.02 |
| PC 44:2 | 17.19 | C₅H₁₀₀O₈N₁P₁¹³C₄₇ | C₃H₁₀₀O₈N₁P₁¹³C₄₉ | 98.4 | 98.3 | 0.10 | 0.07 |
| PC 44:1 | 17.90 | C₅H₁₀₂O₈N₁P₁¹³C₄₇ | C₃H₁₀₂O₈N₁P₁¹³C₄₉ | 98.4 | 98.3 | 0.04 | 0.04 |

| **PC O-** | | | | | | | |
|---|---|---|---|---|---|---|---|
| PC O-30:1 | 11.80 | C₅H₇₆O₇N₁P₁¹³C₃₃ | C₃H₇₆O₇N₁P₁¹³C₃₅ | 98.5 | 98.4 | 0.09 | 0.09 |
| PC O-30:1 | 12.07 | C₅H₇₆O₇N₁P₁¹³C₃₃ | C₃H₇₆O₇N₁P₁¹³C₃₅ | 98.3 | 98.4 | 0.25 | 0.25 |
| PC O-30:1 | 12.96 | C₅H₇₆O₇N₁P₁¹³C₃₃ | C₃H₇₆O₇N₁P₁¹³C₃₅ | 98.3 | 98.4 | 0.26 | 0.39 |
| PC O-30:0 | 13.18 | C₅H₇₈O₇N₁P₁¹³C₃₃ | C₃H₇₈O₇N₁P₁¹³C₃₅ | 98.4 | 98.4 | 3.39 | 3.71 |
| PC O-32:2 | 12.15 | C₅H₇₈O₇N₁P₁¹³C₃₅ | C₃H₇₈O₇N₁P₁¹³C₃₇ | 98.3 | 98.4 | 0.39 | 0.36 |
| PC O-32:1 | 13.27 | C₅H₈₀O₇N₁P₁¹³C₃₅ | C₃H₈₀O₇N₁P₁¹³C₃₇ | 98.3 | 98.3 | 7.47 | 8.02 |
| PC O-32:1 | 13.49 | C₅H₈₀O₇N₁P₁¹³C₃₅ | C₃H₈₀O₇N₁P₁¹³C₃₇ | 98.3 | 98.4 | 16.74 | 17.97 |
| PC O-32:0 | 14.42 | C₅H₈₂O₇N₁P₁¹³C₃₅ | C₃H₈₂O₇N₁P₁¹³C₃₇ | 98.4 | 98.4 | 13.97 | 10.50 |
| PC O-33:0 | 14.72 | C₅H₈₄O₇N₁P₁¹³C₃₆ | C₃H₈₄O₇N₁P₁¹³C₃₈ | 98.4 | 98.5 | 0.54 | 0.49 |
| PC O-33:0 | 14.95 | C₅H₈₄O₇N₁P₁¹³C₃₆ | C₃H₈₄O₇N₁P₁¹³C₃₈ | 98.3 | 98.4 | 0.30 | 0.21 |
| PC O-34:3 | 12.30 | C₅H₈₀O₇N₁P₁¹³C₃₇ | C₃H₈₀O₇N₁P₁¹³C₃₉ | 97.8 | 98.6 | 0.22 | 0.18 |
| PC O-34:2 | 13.30 | C₅H₈₂O₇N₁P₁¹³C₃₇ | C₃H₈₂O₇N₁P₁¹³C₃₉ | 98.4 | 98.5 | 2.71 | 1.74 |
| PC O-34:2 | 13.48 | C₅H₈₂O₇N₁P₁¹³C₃₇ | C₃H₈₂O₇N₁P₁¹³C₃₉ | 98.3 | 98.4 | 1.84 | 2.58 |
| PC O-34:1 | 14.45 | C₅H₈₄O₇N₁P₁¹³C₃₇ | C₃H₈₄O₇N₁P₁¹³C₃₉ | 98.3 | 98.4 | 22.35 | 23.66 |
| PC O-34:1 | 14.65 | C₅H₈₄O₇N₁P₁¹³C₃₇ | C₃H₈₄O₇N₁P₁¹³C₃₉ | 98.3 | 98.4 | 4.84 | 4.52 |
| PC O-34:0 | 15.43 | C₅H₈₆O₇N₁P₁¹³C₃₇ | C₃H₈₆O₇N₁P₁¹³C₃₉ | 98.4 | 98.4 | 7.02 | 5.80 |
| PC O-35:1 | 14.70 | C₅H₈₆O₇N₁P₁¹³C₃₈ | C₃H₈₆O₇N₁P₁¹³C₄₀ | 98.2 | 98.5 | 0.14 | 0.11 |
| PC O-35:1 | 15.00 | C₅H₈₆O₇N₁P₁¹³C₃₈ | C₃H₈₆O₇N₁P₁¹³C₄₀ | 99.5 | 98.2 | 0.28 | 0.28 |
| PC O-35:0 | 15.67 | C₅H₈₈O₇N₁P₁¹³C₃₈ | C₃H₈₈O₇N₁P₁¹³C₄₀ | 98.5 | 98.4 | 0.14 | 0.11 |
| PC O-36:2 | 14.45 | C₅H₈₆O₇N₁P₁¹³C₃₉ | C₃H₈₆O₇N₁P₁¹³C₄₁ | 98.4 | 98.4 | 3.67 | 4.08 |
| PC O-36:1 | 15.47 | C₅H₈₈C₇N₁P₁¹³C₃₉ | C₃H₈₈O₇N₁P₁¹³C₄₁ | 98.4 | 98.4 | 6.33 | 7.90 |
| PC O-36:0 | 16.30 | C₅H₉₀O₇N₁P₁¹³C₃₉ | C₃H₉₀O₇N₁P₁¹³C₄₁ | 98.7 | 98.4 | 0.18 | 0.16 |
| PC O-38:4 | 14.15 | C₅H₈₆O₇N₁P₁¹³C₄₁ | C₃H₈₆O₇N₁P₁¹³C₄₃ | 98.5 | 98.4 | 1.22 | 1.16 |
| PC O-38:4 | 14.60 | C₅H₈₆O₇N₁P₁¹³C₄₁ | C₃H₈₆O₇N₁P₁¹³C₄₃ | 98.6 | 98.5 | 0.50 | 0.38 |
| PC O-38:3 | 14.94 | C₅H₈₈O₇N₁P₁¹³C₄₁ | C₃H₈₈O₇N₁P₁¹³C₄₃ | 98.4 | 98.3 | 0.98 | 0.85 |
| PC O-38:3 | 15.20 | C₅H₈₈O₇N₁P₁¹³C₄₁ | C₃H₈₈O₇N₁P₁¹³C₄₃ | 98.3 | 98.4 | 1.95 | 2.38 |
| PC O-38:2 | 15.55 | C₅H₉₀O₇N₁P₁¹³C₄₁ | C₃H₉₀O₇N₁P₁¹³C₄₃ | 98.5 | 98.4 | 0.42 | 0.46 |
| PC O-38:1 | 16.30 | C₅H₉₂O₇N₁P₁¹³C₄₁ | C₃H₉₂O₇N₁P₁¹³C₄₃ | 98.7 | 98.3 | 0.23 | 0.23 |
| PC O-38:0 | 17.10 | C₅H₉₄O₇N₁P₁¹³C₄₁ | C₃H₉₄O₇N₁P₁¹³C₄₃ | 98.2 | 98.4 | 0.05 | 0.05 |
| PC O-40:5 | 14.68 | C₅H₈₈O₇N₁P₁¹³C₄₃ | C₃H₈₈O₇N₁P₁¹³C₄₅ | 98.5 | 98.5 | 0.25 | 0.05 |
| PC O-40:4 | 14.92 | C₅H₉₀O₇N₁P₁¹³C₄₃ | C₃H₉₀O₇N₁P₁¹³C₄₅ | 98.4 | 98.4 | 0.17 | 0.19 |
| PC O-40:4 | 15.20 | C₅H₉₀O₇N₁P₁¹³C₄₃ | C₃H₉₀O₇N₁P₁¹³C₄₅ | 98.3 | 97.9 | 0.09 | 0.07 |
| PC O-40:4 | 15.54 | C₅H₉₀O₇N₁P₁¹³C₄₃ | C₃H₉₀O₇N₁P₁¹³C₄₅ | 97.9 | 98.0 | 0.16 | 0.12 |
| PC O-40:4 | 15.70 | C₅H₉₀O₇N₁P₁¹³C₄₃ | C₃H₉₀O₇N₁P₁¹³C₄₅ | 98.7 | 98.6 | 0.11 | 0.10 |
| PC O-40:3 | 15.85 | C₅H₉₂O₇N₁P₁¹³C₄₃ | C₃H₉₂O₇N₁P₁¹³C₄₅ | 98.5 | 98.4 | 0.33 | 0.37 |
| PC O-40:2 | 16.25 | C₅H₉₄O₇N₁P₁¹³C₄₃ | C₃H₉₄O₇N₁P₁¹³C₄₅ | 98.7 | 98.2 | 0.12 | 0.15 |
| PC O-40:1 | 17.00 | C₅H₉₆O₇N₁P₁¹³C₄₃ | C₃H₉₆O₇N₁P₁¹³C₄₅ | 98.2 | 98.4 | 0.13 | 0.12 |
| PC O-42:2 | 16.95 | C₅H₉₈O₇N₁P₁¹³C₄₅ | C₃H₉₈O₇N₁P₁¹³C₄₇ | 98.5 | 98.3 | 0.17 | 0.21 |

| **SM** | | | | | | | |
|---|---|---|---|---|---|---|---|
| SM 30:1;O2 | 8.3 | C₅H₇₁O₆N₂P₁¹³C₃₀ | C₃H₇₁O₆N₂P₁¹³C₃₂ | 99.6 | 99.1 | 0.01 | 0.01 |
| SM 32:2;O2 | 9.14 | C₅H₇₃O₆N₂P₁¹³C₃₂ | C₃H₇₃O₆N₂P₁¹³C₃₄ | 98.5 | 98.2 | 0.05 | 0.04 |
| SM 32:1;O2 | 10.55 | C₅H₇₅O₆N₂P₁¹³C₃₂ | C₃H₇₅O₆N₂P₁¹³C₃₄ | 98.3 | 98.5 | 1.56 | 1.43 |
| SM 32:0;O2 | 11.27 | C₅H₇₇O₆N₂P₁¹³C₃₂ | C₃H₇₇O₆N₂P₁¹³C₃₄ | 98.3 | 98.3 | 0.17 | 0.12 |
| SM 33:1;O2 | 11.52 | C₅H₇₇O₆N₂P₁¹³C₃₃ | C₃H₇₇O₆N₂P₁¹³C₃₅ | 98.1 | 99.0 | 0.06 | 0.04 |
| SM 34:2;O2 | 10.88 | C₅H₇₇O₆N₂P₁¹³C₃₄ | C₃H₇₇O₆N₂P₁¹³C₃₆ | 98.5 | 98.7 | 0.06 | 0.05 |
| SM 34:2;O2 | 11.25 | C₅H₇₇O₆N₂P₁¹³C₃₄ | C₃H₇₇O₆N₂P₁¹³C₃₆ | 98.4 | 98.4 | 1.95 | 2.12 |
| SM 34:1;O2 | 11.8 | C₅H₇₉O₆N₂P₁¹³C₃₄ | C₃H₇₉O₆N₂P₁¹³C₃₆ | 98.2 | 98.3 | 0.84 | 0.45 |
| SM 34:1;O2 | 12.1 | C₅H₇₉O₆N₂P₁¹³C₃₄ | C₃H₇₉O₆N₂P₁¹³C₃₆ | 98.1 | 98.3 | 0.09 | 0.07 |
| SM 34:1;O2 | 12.36 | C₅H₇₉O₆N₂P₁¹³C₃₄ | C₃H₇₉O₆N₂P₁¹³C₃₆ | 98.3 | 98.3 | 43.19 | 49.29 |
| SM 34:0;O2 | 12.93 | C₅H₈₁O₆N₂P₁¹³C₃₄ | C₃H₈₁O₆N₂P₁¹³C₃₆ | 98.3 | 98.3 | 12.99 | 7.85 |
| SM 36:2;O2 | 12.9 | C₅H₈₁O₆N₂P₁¹³C₃₆ | C₃H₈₁O₆N₂P₁¹³C₃₈ | 98.0 | 98.2 | 0.56 | 0.63 |
| SM 36:1;O2 | 13.78 | C₅H₈₃O₆N₂P₁¹³C₃₆ | C₃H₈₃O₆N₂P₁¹³C₃₈ | 98.0 | 98.3 | 1.32 | 1.71 |
| SM 36:0;O2 | 14.25 | C₅H₈₅O₆N₂P₁¹³C₃₆ | C₃H₈₅O₆N₂P₁¹3C₃₈ | 98.2 | 98.3 | 0.33 | 0.25 |
| SM 40:2;O2 | 15.24 | C₅H₈₉O₆N₂P₁¹³C₄₀ | C₃H₈₉O₆N₂P₁¹³C₄₂ | 98.2 | 98.3 | 0.38 | 0.39 |
| SM 40:1;O2 | 15.92 | C₅H₉₁O₆N₂P₁¹³C₄₀ | C₃H₉₁O₆N₂P₁¹³C₄₂ | 98.4 | 98.4 | 1.89 | 2.05 |
| SM 40:0;O2 | 16.2 | C₅H₉₃O₆N₂P₁¹³C₄₀ | C₃H₉₃O₆N₂P₁¹³C₄₂ | 98.2 | nd | 0.24 | 0.16 |
| SM 41:1;O2 | 16.35 | C₅H₉₃O₆N₂P₁¹³C₄₁ | C₃H₉₃O₆N₂P₁¹³C₄₃ | 99.6 | 98.5 | 0.04 | 0.02 |
| SM 42:3;O2 | 15.17 | C₅H₉₁O₆N₂P₁¹³C₄₂ | C₃H₉₁O₆N₂P₁¹³C₄₄ | 98.5 | 98.4 | 2.29 | 2.20 |
| SM 42:2;O2 | 15.81 | C₅H₉₃O₆N₂P₁¹³C₄₂ | C₃H₉₃O₆N₂P₁¹³C₄₄ | 98.3 | 98.4 | 14.42 | 14.93 |
| SM 42:2;O2 | 16.18 | C₅H₉₃O₆N₂P₁¹³C₄₂ | C₃H₉₃O₆N₂P₁¹³C₄₄ | 98.7 | 98.4 | 4.34 | 3.22 |
| SM 42:1;O2 | 16.75 | C₅H₉₅O₆N₂P₁¹³C₄₂ | C₃H₉₅O₆N₂P₁¹³C₄₄ | 98.3 | 98.4 | 12.15 | 11.60 |
| SM42:0;O2 | 17.05 | C₅H₉₇O₆N₂P₁¹³C₄₂ | C₃H₉₇O₆N₂P₁¹³C₄₄ | 98.3 | nd | 0.40 | 0.64 |
| SM 44:2;O2 | 16.65 | C₅H₉₇O₆N₂P₁¹³C₄₄ | C₃H₉₇O₆N₂P₁¹³C₄₆ | 99.1 | 98.3 | 0.58 | 0.58 |
| SM 44:1;O2 | 17.48 | C₅H₉₉O₆N₂P₁¹³C₄₄ | C₃H₉₉O₆N₂P₁¹³C₄₆ | 98.2 | 98.1 | 0.11 | 0.12 |

## Claims

1. A method for preparation of fully ¹³C isotopically labeled biomolecules or biomolecules having a predetermined ¹³C isotopic labeling by biosynthesis in a mammalian cell line, which comprises the following steps:
a) providing a mammalian cell line capable of growing in serum-free media;
b) culturing the cell line obtained in step b) in a serum-free medium containing:
- fully ¹³C isotopically labeled amino acids or ¹³C isotopically labeled amino acids,
- fully ¹³C isotopically labeled glucose and/or fully ¹³C isotopically labeled galactose, or ¹³C isotopically labeled glucose and/or ¹³C isotopically labeled galactose,
- fully ¹³C isotopically labeled choline or ¹³C isotopically labeled choline,
- vitamins,
- distilled water,
- insulin,
- transferrin,
- sodium selenite, and
- inorganic salts wherein when the inorganic salt contains carbon, it is preferably fully ¹³C isotopically labeled;
c) obtaining ¹³C isotopically labeled biomolecules from the cells or medium after the cultivation of step b).

2. Method according to claim 1 for preparation of fully ¹³C isotopically labeled biomolecules by biosynthesis in a mammalian cell line, which comprises the following steps:
a) providing a mammalian cell line capable of growing in serum-free media;
b) culturing the cell line obtained in step b) in a serum-free medium substantially composed of:
- fully ¹³C isotopically labeled amino acids,
- fully ¹³C isotopically labeled glucose and/or fully ¹³C isotopically labeled galactose,
- fully ¹³C isotopically labeled choline or ¹³C isotopically labeled choline,
- vitamins,
- distilled water,
- insulin,
- transferrin,
- sodium selenite, and
- inorganic salts wherein when the inorganic salt contains carbon, it is fully ¹³C isotopically labeled;
c) obtaining ¹³C isotopically labeled biomolecules from the cells or medium after the cultivation of step b).

3. Method according to claim 1 for preparation of biomolecules having a predetermined ¹³C isotopic labeling by biosynthesis in a mammalian cell line, which comprises the following steps:
a) providing a mammalian cell line capable of growing in serum-free media;
b) culturing the cell line obtained in step b) in a serum-free medium containing:
- ¹³C isotopically labeled amino acids,
- ¹³C isotopically labeled glucose and/or ¹³C isotopically labeled galactose,
- ¹³C isotopically labeled choline,
- vitamins,
- distilled water,
- insulin,
- transferrin,
- sodium selenite, and
- inorganic salts wherein when the inorganic salt contains carbon, it is optionally fully ¹³C isotopically labeled;
wherein the amino acids, glucose and/or galactose, and/or choline are ¹³C isotopically labeled in positions corresponding to the predetermined ¹³C isotopic labeling of the produced biomolecule(s),
c) obtaining ¹³C isotopically labeled biomolecules from the cells or medium after the cultivation of step b).

4. The method according to any one of claims 1 to 3, wherein the mammalian cell line is a human cell line.

5. The method according to any one of claims 1 to 4, wherein the mammalian cell line is a non-adherent cell line.

6. The method according to any one of claims 1 to 5, wherein in step a), a mammalian cell line is cultured in a cell growth medium supplemented with fetal bovine serum, wherein over several passages the concentration of fetal bovine serum is reduced until zero content of fetal bovine serum in the cell growth medium is reached.

7. The method according to claim 6, wherein the cell growth medium is supplemented by glutamine, antibiotic and/or antimycotic.

8. The method according to any one of claims 1 to 7, wherein subsequently to step a) but prior to step b), the mammalian cell line capable of growing in serum-free medium is cultured in the cell growth medium wherein the content of CO₂ is gradually reduced, preferably the content of CO₂ is reduced down to 2.5 % CO₂.

9. The method according to any one of claims 1 to 8, wherein the set of amino acids contains or consists of at least the following amino acids: L-arginine, L-histidine, L-isoleucine, L-leucin, L-lysine, L-methionine, L-threonine, L-phenylalanine, L-tryptophan, L-tyrosine, L-valine, L-glutamine and L-cysteine; and wherein the set of amino acids may optionally further contain one or more of the following amino acids: glycine, L-asparagine, L-proline, L-serine, L-alanine, L-glutamic acid, L-aspartic acid.

10. The method according to any one of claims 1 to 9, wherein the set of vitamins contains or consists of at least the following vitamins: pantothenate, folic acid, niacinamide, pyridoxine and/or pyridoxal, riboflavin, thiamine, and I-inositol and/or myo-inositol; and wherein the set of vitamins may optionally further contain one or more of: biotin, vitamin B12, p-aminobenzoic acid.

11. The method according to any one of claims 1 to 10, wherein the set of inorganic salts contains or consists of at least the following salts: calcium chloride, magnesium sulfate, potassium chloride, sodium chloride and sodium phosphate monobasic; and wherein the set of inorganic salts may further optionally contain one or more of: sodium bicarbonate, magnesium chloride, iron(III) nitrate, ferric sulfate, zinc sulfate, copper sulfate, sodium phosphate dibasic.
